# EUROPEAN PATENT APPLICATION

(11) **EP 4 806 465 A2**
(43) Date of publication of application: **16.09.2026**
(21) Application number: 26195206.3
(22) Date of filing: 11.06.2021
(51) Int. Cl.: A61K 47/58

(54) **C-TYPE NATRIURETIC PEPTIDES AND METHODS THEREOF IN TREATING ACUTE LUNG INJURY**

(30) Priority: 12.06.2020 US 202063038595 P
(62) Document number(s) of the earlier application(s) in accordance with Art. 76 EPC: 21822873.2 / 4 165 067
(71) Applicant: PharmaIN Corporation, Bothell, WA 98011-8202 (US)
(72) Inventor: TACHIBANA, Hirofumi, Bothell, Washington, 98011 (US); KUMAZOE, Motofumi, Bothell, Washington, 98011 (US); TANAKA, Yasutake, Bothell, Washington, 98011 (US); NOJIRI, Takashi, Bothell, Washington, 98011 (US); CASTILLO, Gerardo, Bothell, Washington, 98011 (US); NISHIMOTO-ASHFIELD, Akiko, Bothell, Washington, 98011 (US); BOLOTIN, Elijah, Bothell, Washington, 98011 (US); YAO, Yao, Bothell, Washington, 98011 (US)
(74) Representative: J A Kemp LLP

(57) **Abstract**

The present disclosure relates to the treatment of lung, liver, and/or kidney injury, by administering to a subject in need thereof a therapeutically effective amount of a (very) long acting C-type natriuretic peptide (CNP), CNP derivative, (very) long acting CNP derivative, or (very) long acting CNP receptor (NPRB) agonist. The disclosure also relates to the treatment of non-cardiovascular causes of low blood oxygenation, elevated levels of inflammatory cells in the lung, pulmonary edema, sepsis, bacteremia, fibrosis in general, and/or interstitial lung disease using the same.

## Description

### CROSS-REFERENCE TO RELATED APPLICATION

This application claims the benefit of U.S. Patent Application No. 63/038,595, filed June 12, 2020, the disclosure of which is incorporated herein by reference in its entirety.

### STATEMENT REGARDING SEQUENCE LISTING

The sequence listing associated with this application is provided in text format in lieu of a paper copy and is hereby incorporated by reference into the specification. The name of the text file containing the sequence listing is 74043_Sequence.txt. The text file is 16 KB and was created on June 11, 2021.

### BACKGROUND

### Acute Lung Injury and Acute Respiratory Distress Syndrome

Acute lung injury (ALI) and acute respiratory distress syndrome (ARDS) are conditions having an acute onset of severe arterial hypoxemia with PaO2/FiO2 of less than or equal to 200 Torr for ARDS and less than 300 Torr for ALI, bilateral radiographic infiltrates, and no evidence of left atrial hypertension *(see, e.g.,* Bernard et al., J. Crit. Care, 1994. 9(1): p. 72-81; Rubenfeld et al., N Engl J Med, 2005. 353(16): p. 1685-93; Brun-Buisson et al., Intensive Care Med, 2004. 30(1): p. 51-61; and Phua et al., Am J Respir Crit Care Med, 2009. 179(3): p. 220-7). As used herein, PaO2 refers to the partial pressure of arterial oxygen, and FiO2 is the fraction of oxygen in the inspired air (room air has a FiO2 of about 0.21, and normal PaO2/FiO2 is about 500 Torr). ARDS is an overwhelming pulmonary inflammatory response to certain primary and secondary noxious stimuli such as pneumonia (e.g., aseptic pneumonia, viral pneumonia, bacterial pneumonia), sepsis, aspiration, inhalation injuries, near drowning, and pulmonary resection surgery (*see, e.g.,* Alam et al., Ann Thorac Surg, 2007. 84(4): p. 1085-91). ARDS is characterized by rapid-onset respiratory failure necessitating hospitalization and ventilatory support in an intensive care unit (ICU). If a patient survives ALI/ARDS, the long-term quality of life of the patient is often adversely affected due to lung scarring *(see, e.g.,* Rubenfeld et al., N Engl J Med, 2005. 353(16): p. 1685-93; Dowdy et al., Intensive Care Med, 2006. 32(8): p. 1115-24). To date, no effective agent to treat acute lung injury (ALI) and ARDS has been identified and there is a significant need for such an agent.

Supportive care for ALI includes oxygen treatment to maintain arterial partial pressure of oxygen (PaO₂) above 55 mmHg, or oxygen saturation (SaO₂) above 88%, and fluid management. However, care must be taken not provide too much oxygen *(i.e.,* oxygen should be given at less than 60%) to avoid oxygen toxicity. Moreover, this measure does not address the underlying alveolar inflammatory edema.

Agents previously tested in human clinical trials for the treatment of ALI, including glucocorticoids, surfactants, N-acetylcysteine, inhaled nitric oxide, liposomal PGE 1, ketoconazole, lisofylline, salbutamol, procysteine, activated protein C, and inhaled albuterol, have all failed *(see, e.g.,* Johnson ER and Matthay MA, J Aerosol Med Pulm Drug Deliv. 2010, 23(4):243-52). ALI treatment remains elusive to the person skilled in the art.

Lung or pulmonary fibrosis (PF) refers to a progressive scarring of the lung tissue caused by many conditions including chronic inflammatory processes (e.g., sarcoidosis, Wegener's granulomatosis), infections, environmental agents (e.g., asbestos, silica, exposure to certain gases), exposure to ionizing radiation (e.g., radiation therapy to treat tumors of the chest), chronic conditions (e.g., lupus, rheumatoid arthritis), or certain medications. Interstitial lung disease (ILD) is another umbrella term used for PF and for the purpose of this specification will be synonymous. Idiopathic pulmonary fibrosis (IPF) is a PF of unknown cause. PF or IPF are an incurable type of chronic scarring lung disease characterized by a progressive and irreversible decline in lung function with gradual onset of shortness of breath and a dry cough that affects 5 million people globally *(see, e.g.,* Raghu et al., (2011) American Journal of Respiratory and Critical Care Medicine. 183 (6): 788-824) with associated risk factors that include chemical inhalation such as cigarette smoking, viral infections, or a family history of the condition. Other symptoms may include fatigue, and abnormally large and dome-shaped fingernails and toenails (nail clubbing). *See, e.g.,* nhlbi.nih.gov/health-topics/idiopathic-pulmonary-fibrosis; en.wikipedia.org/wiki/Idiopathic_pulmonary_fibrosis. Complications may include pulmonary hypertension, heart failure, pneumonia, or pulmonary embolism.

Although C-type natriuretic peptide (CNP) can mitigate ALI, sepsis, and IPF if given continuously prior to, or during, an injury that would eventually lead to ALI or sepsis, its effectiveness when used after the injury (e.g., as a post-injury treatment) is unknown. Conventionally, CNP must be given continuously at low dosages, and cannot be given as a bolus dose because it has a very short half-life and because a bolus dose can cause an acute drop in blood pressure. If given as a high bolus dose to compensate for short half-life and to extend the duration of blood presence, a very high peak plasma concentration (Cₘₐₓ) occurs, which results in a dangerous drop in blood pressure. To mitigate these deleterious effects, CNP is usually delivered by slow infusion. *See, e.g.,* Kimura et al., J Surg Res. 2015, 194(2); 631-637.

### CNP AND NPRB RECEPTOR

CNP was first isolated in 1990 from porcine brain by Sudoh *et al.* and is a peptide that consists of 22 amino acid residues. *See, e.g.,* Sudoh et al., Biochem. Biophys. Res. Commun. 1989; 159:1427-1434. CNP has a ring structure and is similar structurally to related natriuretic peptides, atrial natriuretic peptide (ANP), and B-type natriuretic peptide (BNP), but lacks a carboxy-terminal extension. *See, e.g.,* Hunt et al., J. Clin. Endocrinol. Metab. 1994; 78:1428-1435. CNP is a highly conserved natriuretic peptide among various species. *See, e.g.,* Imura et al., Front. Neuroendocrinol. 1992; 13:217-249. For example, in humans, CNP gene (NPPC) is located on chromosome 2, whereas the mouse CNP gene is located on chromosome 1. CNP gene is composed of two exons and one intron. *See, e.g.,* Ogawa et al., The Journal of Clinical Investigation. 1994; 93:1911-192110; and Ogawa et al, Genomics. 1994; 15(24):383-387. It is produced as a preprohormone or a 126 amino acid residue parent-CNP peptide that is converted to 103 amino acid residue pro-CNP after removal of 23 amino acid residues at the carboxyl end, and is further processed to a 53 amino acid residue-containing CNP-53 and a 22 amino acid residue-containing CNP by the enzyme furin. *See, e.g.,* Lumsden et al., Curr. Pharm. Des. 2010; 16:4080-4088; Wu et al., J. Biol. Chem. 2003; 278:25847-25852; and Chopra et al., Indian J. Endocrinol. Metab. 2013; 17:83-90. The higher molecular weight CNP-53 (CNP 51-103) predominates in tissues, whereas CNP-22 (CNP 82-103) is found mainly in plasma and cerebrospinal fluid but both contain 17-amino acid residue ring structure common to all natriuretic peptide. In comparison to ANP and BNP, the plasma half-life of CNP is relatively short and is about 2 to 3 min in humans. *See, e.g.,* Potter LR. FEBS J. 2011; 278:1808-1817. Normal plasma CNP concentrations (both forms) are in low femtomole per milliliter range. *See, e.g.,* Das B.B. and Solinger R., Cardiovasc Hematol Agents Med Chem. 2009, 7, 29-42. CNP is mainly produced and secreted from the endothelium of vasculature and male genital glands and acts as a relaxing peptide. *See, e.g.,* Suga et al., Endocrinology. 1998; 139:1920-1926.

CNP peptides have two known membrane receptors, namely natriuretic peptide receptor B (NPRB) and natriuretic peptide receptor C (NPRC). The NPRB receives messages from CNP and activates downstream signaling pathways, whereas NPRC is mainly a clearance receptor that is primarily involved in clearance or degradation of CNP. *See, e.g.,* Itoh H and Nakao K, Nihon Rinsho. 1997; 55:1923-1936; Koller et al., Science. 1991; 252:120-123; Suga et al., Endocrinology. 1992; 130:229-239; and Potter LR and Hunter T. J. Biol. Chem. 2001; 276:6057-6060. NPRB is also known by other names such as guanylate cyclase B (GC-B) or B-type natriuretic peptide receptor 2 (NPR2).

The remaining natriuretic peptide receptor, NPRA, is activated by atrial natriuretic peptide (ANP) and B-type natriuretic peptide (BNP), but is not activated by CNP. While ANP and BNP activate both NPRA and NPRB, CNP selectively activates NPRB, and all three natriuretic peptides bind to NPRC (which lacks guanylyl cyclase activity) and undergo clearance and degradation. *See, e.g.,* Koller et al., Science. 1991; 252:120-123; Suga et al., Endocrinology. 1992; 130:229-239; and Potter LR and Hunter T. J. Biol. Chem. 2001; 276:6057-6060. The differences in the physiological consequences of activation of one receptor versus both NPRA and NPRB receptors remain unclear. In addition, testing of the *in vivo* effects of CNP is confounded by the difficulty of simple bolus administration of CNP because of its short half-life (2-13 minutes), and the fact that bolus administration is associated with an acute drop in blood pressure. *See, e.g.*, Kimura et al., J Surg Res. 2015, 194(2); 631-637. Indeed, it is unknown prior to the present disclosure whether any NPRB agonist, CNP, or a CNP derivative can be given as a bolus to treat ALI or ARDS without a significant drop in blood pressure (e.g., more than 20%, more than 15 %, more than 10%, or more than 5% drop in blood pressure), while increasing cyclic-GMP by a significant amount (e.g., above 1.5x, above 2x, above 3x, above 4x, or above 5x of a baseline plasma cyclic-GMP level), over a sustained period of time *(i.e.,* for 6 hours, 8 hours, 12 hours, 16 hours, 24 hours, 48 hours, 72 hours, 84 hours, or 168 hours).

The expression and secretion of CNP are also regulated by various cytokines and growth factors such as tumor necrosis factor (TNF), lipopolysaccharide (LPS), basic fibroblast growth factor (bFGF), interleukin-1 (IL-1), transforming growth factor beta (TGFβ), and thrombin which are involved in vascular remodeling and inflammation. *See*, *e.g.,* Suga et al., Endocrinology. 1993; 133:3038-3041; Suga et al., J. Clin. Invest. 1992; 90:1145-1149; Woodard et al., Am. J. Physiol. Regul. Integr. Comp. Physiol. 2002; 282:R156-R165; Hama et al., Biochem. Biophys. Res. Commun. 1994; 198:1177-1182; and Okahara et al.. FEBS Lett. 1995; 373:108-110. During endothelial damage, sepsis, hypoxia, and chronic renal failure, the levels of CNP are elevated in blood. *See, e.g.,* Hama et al., Biochem. Biophys. Res. Commun. 1994; 198:1177-1182. Shear stress also induces the expression of CNP gene in human endothelial cells. *See, e.g.,* Okahara et al., FEBS Lett. 1995; 373:108-110. The promoter region of CNP gene has binding sites for the transcription factor TSC-22 *(see, e.g.,* Sellitti et al., Peptides. 2011; 32:1964-1971), which is believed to be involved in the regulation of hematopoietic precursor cells function and is a putative tumor suppressor gene that is hypermethylated and silenced in T or NK LGL leukemia. *See, e.g.,* Yu et al., Blood. 2009; 113(22): 5558-67. CNP gene promoter also has binding sites for transcription factors such as NF-κB, STAT1, ATF6 and E2F1. *See, e.g.,* Santhekadur et al., Biomed Pharmacother. 2017; 92: 826-835. However, it is unknown whether an NPRB agonist such as CNP or its derivatives can be used to treat ALI or ARDS. In fact, some inflammation is associated with an increase in CNP expression and secretion. Indeed, the consequences of bolus administration of more CNP, its derivatives, or other NPRB agonists to treat ALI or ARDS are not known. This uncertainty is further confused by the complexity and unpredictability of biological systems.

A previous study in healthy human volunteers demonstrated that CNP bolus injection caused a transient but significant decrease in both systolic and diastolic blood pressure with a significant increase in heart rate with only a limited and transient increase in plasma cyclic-GMP of less than 90 minutes. Igaki et al., Hypertens Res 1998; 21: 7-13. In general, all CNPs produce hemodynamic effects or similar blood pressure-reducing activity in mice, nonhuman primates, rats, dogs, and humans. *See, e.g.,* Wendt et al., J Pharmacol Exp Ther 353:132-149, April 2015. Another CNP variant (BMN-111; sequence PGQEHPNARK YKGANKKGLS KGCFGLKLDR IGSMSGLGC(SEQ ID NO. 1)) with increased neutral-endopeptidase (NEP) resistance is currently in development. Studies of BMN-111 in animals and man have demonstrated that as the dose increases to the desired therapeutic level, arterial blood pressure (BP) drops and heart rate (HR) increases. In addition to investigating various variants of CNP, different CNP conjugates were obtained by conjugating the CNP moiety to either PEG or proteinaceous compounds. These PEGylated and chimeric CNP exhibited a similar hemodynamic response as observed for the non-PEGylated CNP variants. All variants previously studied showed similar BP-reducing activity. *See*, *e.g.,* Wendt, J., Pharmacol Exp Ther 353:132-149, April 2015. Therefore, without wishing to be bound by theory, it is believed that increasing the bolus dose of a drug having CNP activity to increase drug exposure may be associated with unacceptable cardiovascular side-effects, such as hypotension.

Thus, there is a need for more efficacious and safer treatments of ALI and/or ARDS, which avoid the cardiovascular side-effects, such as hypotension, while maintaining or enhancing plasma levels of a CNP therapeutic agent. There is also a need for CNP derivative or CNP receptor (NPRB) agonists with long half-life that can have an extended presence in blood, which can be used for the treatment of ALI and/or ARDS. The present disclosure seeks to fulfill these needs and provides further related advantages.

### SUMMARY

This summary is provided to introduce a selection of concepts in a simplified form that are further described below in the Detailed Description. This summary is not intended to identify key features of the claimed subject matter, nor is it intended to be used as an aid in determining the scope of the claimed subject matter.

In one aspect, the present disclosure features a method of treating a subject having a lung, liver, and/or kidney injury; or a symptom associated with a lung, liver, and/or kidney injury, comprising: administering to the subject a therapeutically effective bolus dose of a composition comprising a long acting CNP, a long acting CNP derivative, a long acting NPRB agonist, a very long acting CNP, a very long acting CNP derivative, a very long acting NPRB agonist, a long acting CNP agonist, a very long acting CNP agonist, or any combination thereof, wherein the composition does not decrease blood pressure by more than 20% *(e.g.,* by more than 15%, by more than 10%, or by more than 5%) of a baseline blood pressure measurement, where the baseline blood pressure measurement is an average blood pressure prior to administration of the composition, wherein the composition increases plasma cyclic-GMP level at from 1 hour to 12 hours (e.g., 2 to 12 hours, 4 to 12 hours, 1 hour to-24 hours, 2 to 24 hours, 4 to 24 hours, 1 hour to 84 hours, 2 to 84 hours, 4 to 84 hours, 12 to 84 hours, 1 hour to 168 hours, 2 to 168 hours, 4 to 168 hours, or 12 to 168 hours) after administration to above 1.5x (e.g., above 2x, above 3x, above 4x, or above 5x) of a baseline plasma cyclic-GMP level, the baseline plasma cyclic-GMP level is an average plasma cyclic-GMP level prior to administration of the composition or the average plasma cyclic-GMP level of a healthy subject (preferably the average plasma cyclic-GMP level prior to administration of the composition for the subject), and wherein the lung, liver, and/or kidney injury, or the symptom associated with lung liver, and/or kidney injury is selected from acute lung injury (ALI), acute respiratory distress syndrome (ARDS), pulmonary edema, elevated level of inflammatory cells in the lung, increased level or expression of inflammatory cytokines in the lung as compared to healthy lung, increased protein level in lung alveolar space as compared to healthy lung, low arterial blood oxygenation, wherein low arterial blood oxygenation is a blood PaO2 of below 60 mm Hg and/or a blood hemoglobin oxygen saturation (SpO2) of below 90%, pneumonia, fibrosis *(e.g.,* lung fibrosis, liver fibrosis, kidney fibrosis), kidney injury, and any combination thereof.

In another aspect, the present disclosure features a long acting CNP derivative or a very long acting CNP derivative including U-GLSKGCFGLKLDRIGSMSGLGC [SEQ ID NO. 2], U-GLSKGCFGLK(U)LDRIGSMSGLGC [SEQ ID NO. 3], GLSKGCFGLK(U)LDRIGSMSGLGC [SEQ ID NO. 4], U-CFGLKLDRIGSxSGLGC, where x is a natural or unnatural amino acid residue [SEQ ID NO. 11], or any combination thereof; where each individual capital letter, with the exception of U, is an amino acid residue as represented by one-letter amino acid nomenclature, and where U is a moiety of Formula (I) or (II), where Formula (I) is

(aliphatic)ₐ-(X)-; (I)

wherein a is 0 or 1 (preferably a is 1); aliphatic is an optionally substituted C₄₋₂₄ chain (e.g., optionally substituted C₁₀₋₂₄ chain, optionally substituted C₁₂₋₁₈ chain), covalently bound to X via a chemical linkage, such as a carbonyl *(e.g.,* as part of an amide or an ester linkage), a thioether, an ether, a thioether, a carbamate moiety, a bond, or the like with X; preferably via a carbonyl as part of an amide or an ester linkage; or more preferably via a carbonyl as part of an amide linkage with X; X is a 1-10 amino acid residue or peptide sequence, wherein each amino acid residue is independently selected from lysine (K), arginine (R), glycine (G), alanine (A), glutamic acid (E), and aspartic acid (D); or X is a linker (γE)ₘ-(B)ₙ, wherein B is a 1-8 amino acid residue or peptide sequence wherein each amino acid residue is independently selected from 2-[2-(2-aminoethoxy)ethoxy] acetic acid residue, Gly, Ala, Leu, Ser, Arg, and Lys; m is 0, 1, 2, or 3; n is 0, 1, 2, or 3; and the sum of m and n is at least 1, and
Formula (II) is

   (polymer)ₐ-(Y)-; (II)
wherein a is 0 or 1 (preferably a is 1); polymer is cellulose, poly(ethylene glycol) (PEG), methoxy poly(ethylene glycol) (MPEG), poly(lactic-co-glycolic acid), poly(N-vinyl pyrrolidone), or a derivative thereof; Y is: a 1-10 amino acid residue or peptide sequence, wherein each amino acid residue is independently selected from lysine (K), arginine (R), glycine (G), alanine (A), glutamic acid (E), and aspartic acid (D); a non-amino acid linker comprising an ester, an amide, a thioether, an ether, a thioether, a carbamate moiety, or a combination thereof; an amino acid residue-containing linker, wherein the amino acid residue is covalently attached to (polymer)ₐ; or a peptide linker different from the 1-10 amino acid residue or peptide sequence. In some embodiments, Y is a linker (γE)ₘ-(B)ₙ, wherein B is a 1-8 amino acid residue or peptide sequence wherein each amino acid residue is independently selected from 2-[2-(2-aminoethoxy)ethoxy]acetic acid residue, Gly, Ala, Leu, Ser, Arg, and Lys; m is 0, 1, 2, or 3; n is 0, 1, 2, or 3; and the sum of m and n is at least 1.

In yet another aspect, the present disclosure features a method of treating a subject having ALI and/or ARDS, or at risk of developing ALI and/or ARDS, including: administering to the subject a therapeutically effective bolus dose of a composition comprising a long acting CNP derivative or a very long acting CNP derivative comprising U-GLSKGCFGLKLDRIGSMSGLGC [SEQ ID NO. 2], U-GLSKGCFGLK(U)LDRIGSMSGLGC [SEQ ID NO. 3], or GLSKGCFGLK(U)LDRIGSMSGLGC [SEQ ID NO. 4], U-CFGLKLDRIGSxSGLGC, where x is a natural or unnatural amino acid residue [SEQ ID NO. 11], or any combination thereof. U is defined as in Formula (1) or (II), described above. U can be covalently bound to an N-terminal G or C residue and/or to an epsilon amino group of K residue. The composition does not decrease blood pressure by more than 15% *(e.g.,* by more than 10%, or by more than 5%) of a baseline blood pressure measurement, where the baseline blood pressure measurement is an average blood pressure prior to administration of the composition; and the composition increases plasma cyclic-GMP level at from 1 hour to 12 hours (e.g., 2 to 12 hours, 4 to 12 hours, 1 hour to-24 hours, 2 to 24 hours, 4 to 24 hours, 1 hour to 84 hours, 2 to 84 hours, 4 to 84 hours, 12 to 84 hours, 1 hour to 168 hours, 2 to 168 hours, 4 to 168 hours, or 12 to 168 hours) after administration to above 1.5x (e.g., above 2x, above 3x, above 4x, or above 5x) of a baseline plasma cyclic-GMP level, where the baseline plasma cyclic-GMP level is an average plasma cyclic-GMP level prior to administration of the composition or the average plasma cyclic-GMP level of a healthy subject (preferably the average plasma cyclic-GMP level prior to administration of the composition for the subject).

In one aspect, the present disclosure provides a composition including a long acting CNP derivative comprising a peptide of formula U-CFGLKLDRIGSxSGLGC [SEQ ID NO. 30], wherein x is a natural or unnatural amino acid residue, provided that x is not a methionine residue; and U has is a moiety of Formula (I):

(aliphatic)ₐ-(X)-; (I)

wherein a is 0 or 1 (preferably a is 1);
aliphatic is an optionally substituted C₄₋₂₄ chain (e.g., optionally substituted C₁₀₋₂₄ chain, optionally substituted C₁₂₋₁₈ chain), covalently bound to X via a chemical linkage, such as a carbonyl (e.g., as part of an amide or an ester linkage), a thioether, an ether, a thioether, a carbamate moiety, a bond, or the like with X; preferably via a carbonyl as part of an amide or an ester linkage; or more preferably via a carbonyl as part of an amide linkage with X; and
X is a linker (γE)ₘ-(B)ₙ wherein B is 1-8 amino acid residue or peptide sequence wherein each amino acid residue is independently selected from 2-[2-(2-aminoethoxy)ethoxy]acetic acid residue, Gly, Ala, Leu, Ser, Arg, and Lys; m is 0, 1, 2, or 3; n is 0, 1, 2, or 3; and the sum of m and n is at least 1.

### DESCRIPTION OF THE DRAWINGS

The foregoing aspects and many of the attendant advantages of this disclosure will become more readily appreciated as the same become better understood by reference to the following detailed description, when taken in conjunction with the accompanying drawings, wherein:

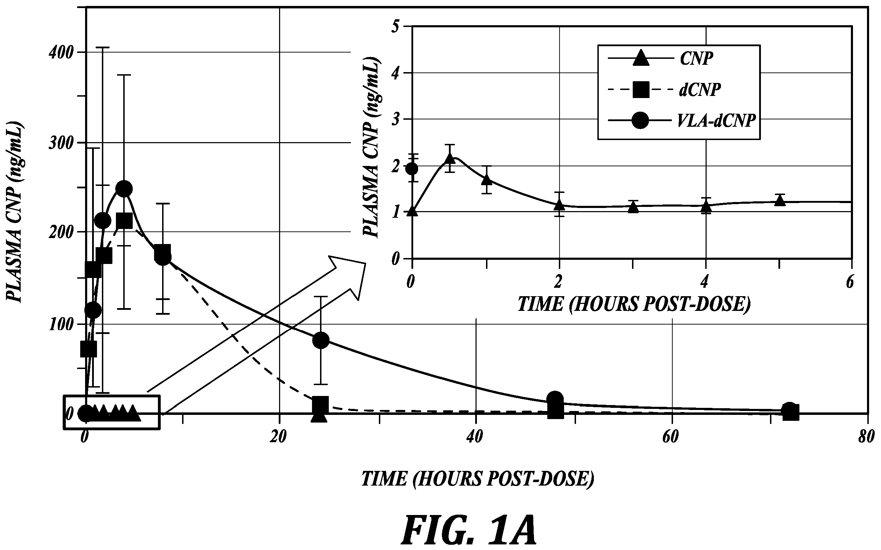

FIGURE 1A is a plot showing plasma CNP [mean (SD); n= 5] in CD-1 mice after subcutaneous administration 2.0 mg/Kg of a native CNP, a CNP derivative (dCNP), and a very long acting CNP derivative (VLA-dCNP). The inset is an enlarged scale of the left bottom corner to show the low plasma level of CNP (diamond) when native CNP is administered. Error bars represent standard deviation of n=5 plasma samples. Baseline CNP level prior to administration is 1.74 (0.6) ng/mL [mean (SD); n=15]. FIGURE 1A shows the sustained plasma presence of dCNP and VLA-dCNP after bolus administrations in mice.

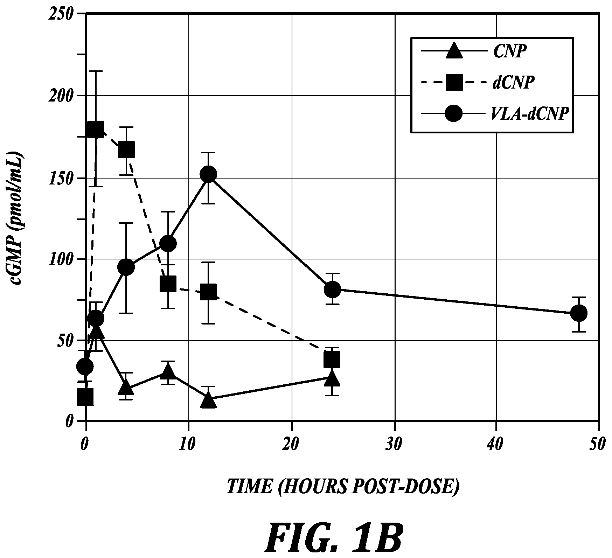

FIGURE 1B is a plot showing plasma cyclic-GMP in male C57BL/6J mice measured by cyclic-GMP kit from CisBio (Codolet, France) after subcutaneous administration 1.0mg/Kg of native CNP, CNP derivative (dCNP), and very long acting CNP derivative (VLA-dCNP). Baseline plasma cyclic-GMP level was 20 ((3.7) mean (SEM); n=8) pmol/mL or 7 ((1.3) mean (SEM); n=8) ng/mL; [n=8]. At 2 hours and beyond, subcutaneous administration of native CNP did not show significant elevation of plasma cyclic-GMP compared to the baseline, while similar administration of long acting CNPs (dCNP and VLA-dCNP) showed significant elevation of cyclic-GMP for at least 24 hours. FIGURE 1B shows the sustain presence of cyclic-GMP after bolus administrations of dCNP and VLA-dCNP compared to native CNP in mice.

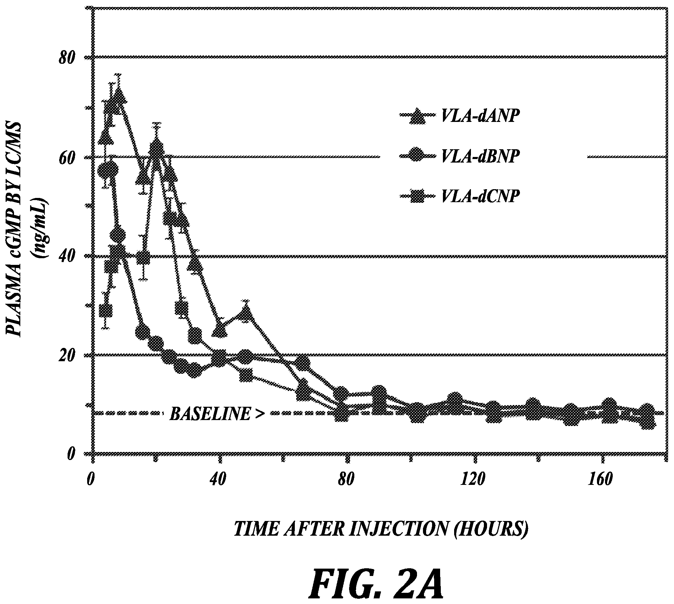

FIGURE 2A is a plot showing the corresponding increase in plasma cyclic-GMP [mean (SEM); n=12] as monitored after a bolus administration of 25 µg/Kg of very long acting CNP derivative (VLA-dCNP), very long acting BNP derivative (VLA-dBNP), and very long acting ANP derivative (VLA-dANP). Baseline plasma cyclic-GMP level was 8 (2) ng/mL [mean (SD); n=12], a level which is similar to a healthy human. *See, e.g.,* Igaki, et al., Hypertens Res 1998; 21: 7-13. All very long acting formulations of natriuretic peptide increased cyclic-GMP above the baseline of 8ng/ml. The cyclic-GMP AUC values were VLA-dANP 3,483 ng*h/mL, VLA-dBNP 2,585 ng*h/mL, VLA-dCNP 2,627 ng*h/mL. The very long acting CNP derivative (VLA-dCNP) increased plasma cyclic-GMP for 3 days without an associated drop in blood pressure. FIGURE 2A shows the sustained presence of cyclic-GMP after bolus administration of VLA-dCNP compared to two other very long acting natriuretic peptides from the same family.

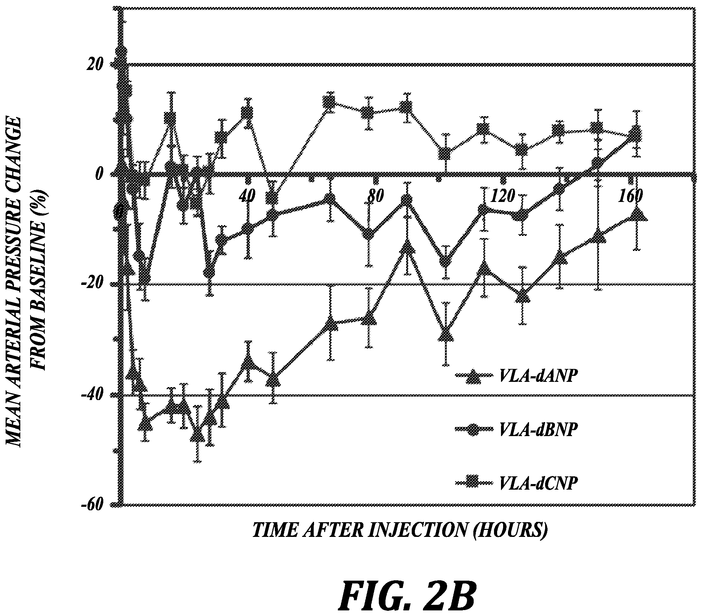

FIGURE 2B is a plot showing mean arterial pressure in dogs [mean (SEM); n=12] as monitored after a bolus administration of 25 µg/Kg of very long acting CNP derivative (VLA-dCNP), very long acting BNP derivative (VLA-dBNP), and very long acting ANP derivative (VLA-dANP). VLA-dCNP did not cause significant drop in blood pressure from baseline (0 hr) after administration at a very high dose. In comparison, other very long acting natriuretic peptides such as VLA-dBNP and VLA-dANP derivatives caused more than a 15% drop in blood pressure. This was especially true for VLA-dANP where a drop in blood pressure could be as much as 50% for similar increase in cyclic-GMP. In stark contrast, the very long acting CNP derivative (VLA-dCNP) increased plasma cyclic-GMP for 3 days without an associated drop in blood pressure. FIGURE 2B shows that absence of a drop in blood pressure after bolus administrations in dogs of a high dose of VLA-dCNP while the two other very long acting natriuretic peptides from the same family showed dramatic drop in blood pressure despite elevation of plasma cyclic-GMP (FIGURE 2A). This indicates that plasma cyclic-GMP is not the cause of the drop in blood pressure.

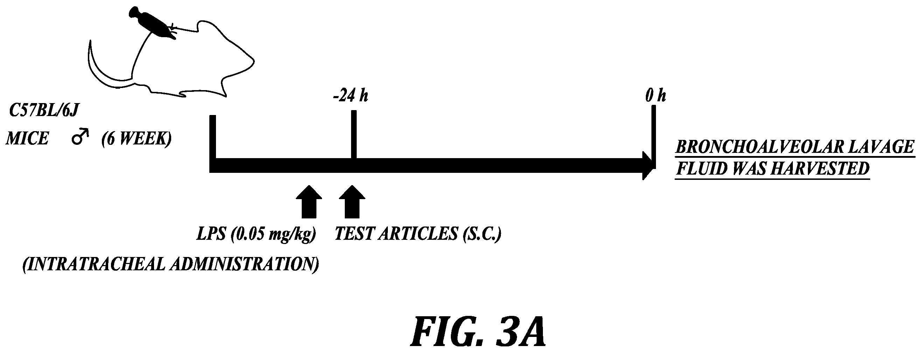

FIGURE 3A is a timeline of a protocol for evaluating dCNP-suppressed LPS-induced acute lung injury. The protocol included treating mice with LPS (0.05 mg/kg intratracheal administration) and with VLA-dCNP (L: 0.1 mg/kg s.c.; M: 0.3 mg/kg s.c.; H: 1.0 mg/kg s.c.), dCNP (H 1.0 mg/kg s.c.), CNP (1.0 mg/kg s.c.), atrial natriuretic peptide (ANP) (H 1.0 mg/kg s.c.), brain natriuretic peptide (BNP) (H 1.0 mg/kg s.c.), anti-mouse TNFα (TNFa ab) (clone XT3.11; BioXcell West Lebanon, NH) at 1.0 mg/Kg s.c., or Vardenafil (VDN) (Cayman Chemicals Ann Arbor, MI) at 1.0 mg/Kg s.c. Test articles were administered right after LPS administration. After 24 hours from treatment, mice were sacrificed under isoflurane anesthesia and then bronchoalveolar lavage fluid (BALF) was harvested.

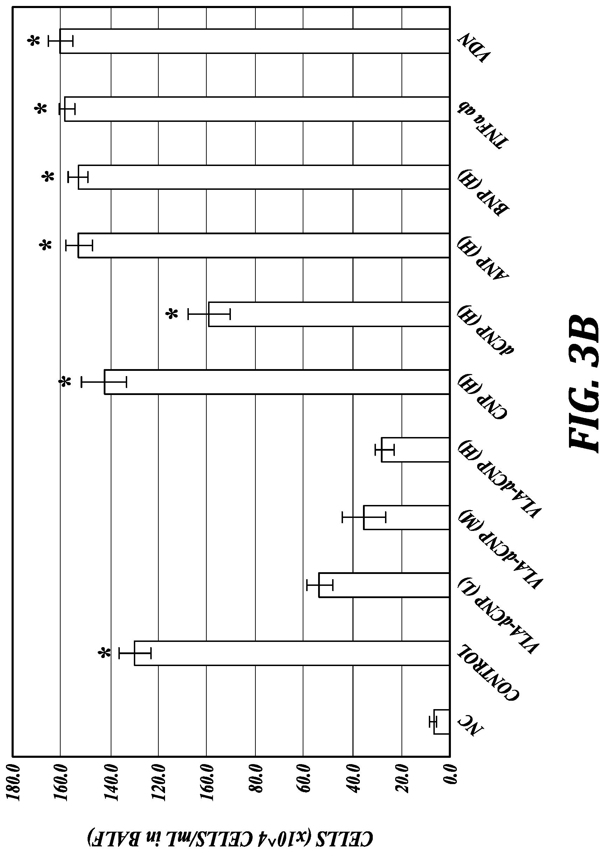

FIGURE 3B is a bar graph showing an increase in cells in BALF, especially neutrophils, in ALI and ARDS, following the protocol shown in FIGURE 3A. The decrease in cells indicated resolution of ALI/ARDS. Statistical analysis was based on Dunnett's test performed by using GraphPad InStat 3 (n = 15, 23, 7, 7, 7, 7, 7, 7 and 9; NC, Control, CNP (H), dCNP (H), ANP (H), BNP (H), TNFα ab, VDN, VLA-dCNP (H). * P < 0.01 vs VLA-dCNP (H)). FIGURE 3B is a bar graph showing that bolus administration of VLA-dCNP ameliorated LPS-induced cell infiltration of alveolar space.

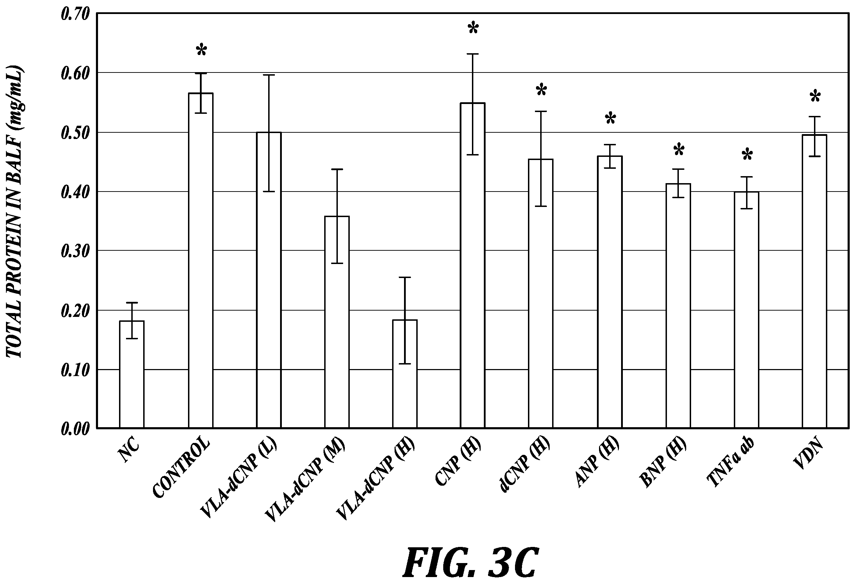

FIGURE 3C is a bar graph showing the total proteins in BALF, in ALI and ARDS, following the protocol shown in FIGURE 3A. The decrease in total proteins indicated resolution of ALI/ARDS. Statistical analysis was based on Dunnett's test performed by using GraphPad InStat 3 (n = 15, 23, 7, 7, 7, 7, 7, 7 and 9; NC, Control, CNP (H), dCNP (H), ANP (H), BNP (H), TNFα ab, VDN, VLA-dCNP (H). * P < 0.01 vs VLA-dCNP (H)).

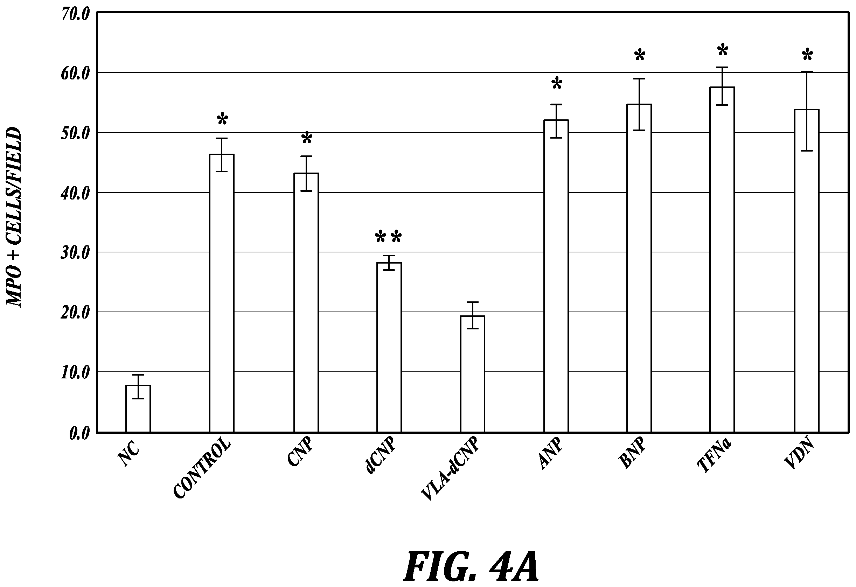

FIGURE 4A is a bar graph showing that VLA-dCNP treatment ameliorated LPS-induced MPO+ cell increase *(i.e.,* The MPO+ cells are decreased relative to control), a marker neutrophil granulocyte pro-inflammatory cell. Statistical analysis was based on Dunnett's test performed by using GraphPad InStat 3 Control (n = 18, 6, 6, 6, 6, 6, 6 and 6; Control, CNP, dCNP, ANP, BNP, TNFα ab, VDN, VLA-dCNP. * P < 0.01 vs VLA-dCNP and ** P< 0.05 vs VLA-dCNP).

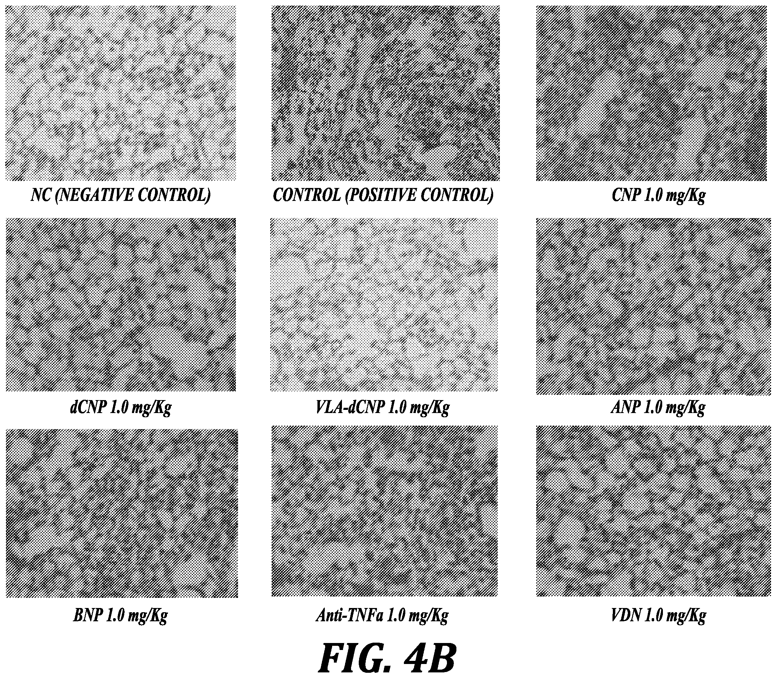

FIGURE 4B is a series of photographs h showing that bolus administration of VLA-dCNP or treatment ameliorated LPS-induced inflammatory lung damage. Shown is a series of micrographs of hematoxylin-eosin (HE) staining of paraffin-sections of lung tissue showing an intensity indicative of an increase in nucleated-cell number, extracellular matrix and protein in general, scarring, and/or protein permeation in the alveolar space. Inflammatory cell infiltration as seen by HE stains indicated inflammation in the lung (panels showing darker staining as cell numbers indicating presence of inflammatory pathology and protein increase indicating protein leakage into alveoli and/or extracellular matrix or scar deposition). For these studies, mice were treated with LPS (Sigma-Aldrich; 0.05 mg/kg intratracheal administration) and then treated with very long acting CNP derivative or VLA-dCNP (1.0 mg/kg s.c.), native C-type natriuretic peptide or CNP (1.0 mg/kg s.c.), CNP derivative or dCNP (1.0 mg/kg s.c.), atrial natriuretic peptide (ANP) (1.0 mg/kg s.c.), B-Type natriuretic peptide or BNP (1.0 mg/kg s.c.), anti-tumor necrosis factor alpha antibody or TNFα ab (1.0 mg/kg s.c.), and cyclic-GMP degradation inhibitor or PDE5 inhibitor called Vardenafil (VDN) (1.0 mg/kg s.c.). Test articles were administered right after LPS administration. At 24 hours after treatment, mice were sacrificed under isoflurane anesthesia and lung tissue was harvested and fixed by 4% paraformaldehyde. Paraffin section of fixed lung tissue was stained by anti-MPO antibody and hematoxylin-eosin stain.

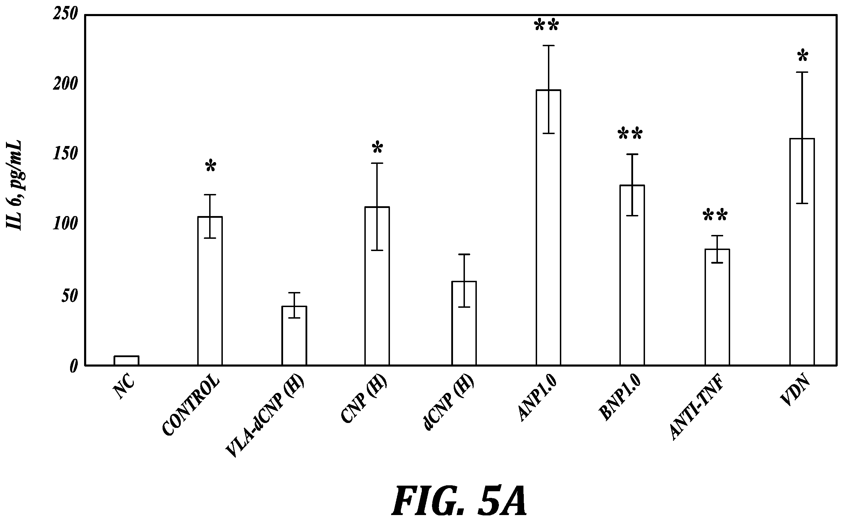

FIGURE 5A is a bar graph showing that bolus administration of VLA-dCNP and dCNP or treatment attenuated LPS-induced upregulation of inflammatory cytokines (IL6) in BALF to facilitate resolution of ARDS/ALI. Male C57BL/6J mice (6 week) were treated with LPS (0.05 mg/kg intratracheal administration) and treated with very long acting CNP derivative or VLA-dCNP (1.0 mg/kg s.c.), native C-type natriuretic peptide or CNP (1.0 mg/kg s.c.), CNP derivative or dCNP (1.0 mg/kg s.c.), atrial natriuretic peptide (ANP) (1.0 mg/kg s.c.), B-Type natriuretic peptide or BNP (1.0 mg/kg s.c.), anti-Tumor necrosis factor alpha antibody or TNFα ab (1.0 mg/kg s.c.), and cyclic-GMP degradation inhibitor or PDE5 inhibitor called Vardenafil (VDN) (1.0 mg/kg s.c.). After 24 hours from treatment, bronchoalveolar lavage fluid (BALF) was harvested and IL-6 cytokines were measured. Statistical analysis was based on Student's t-test. (n = 15, 23, 7, 7, 7, 7, 7, 7 and 9; NC, Control, CNP, dCNP, ANP, BNP, TNFα ab, VDN, and VLA-dCNP. * P < 0.01 vs. VLA-dCNP and ** P< 0.05 vs. VLA-dCNP).

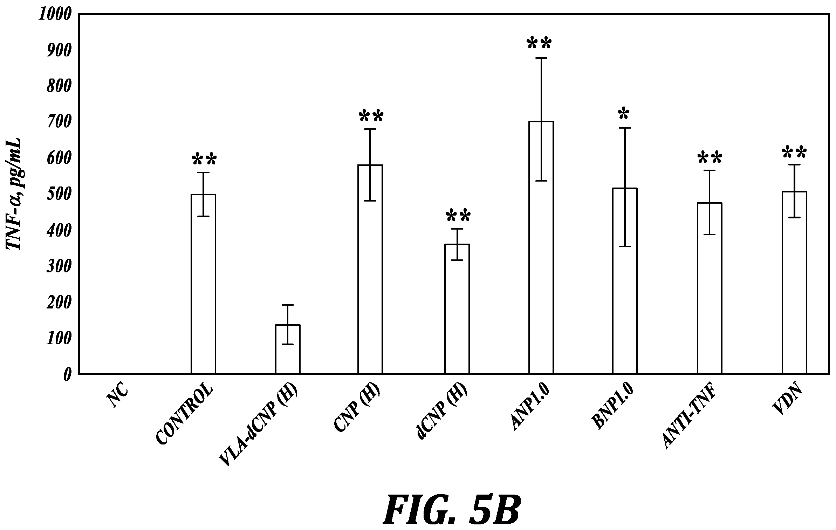

FIGURE 5B is a bar graph showing that bolus administration of VLA-dCNP and dCNP or treatment attenuated LPS-induced up-regulation of inflammatory cytokines (TNFα) in BALF to facilitate resolution of ARDS/ALI. The protocol was the same as that described in FIGURE 5A, except that bronchoalveolar lavage fluid (BALF) was harvested and measured for TNFα cytokines.

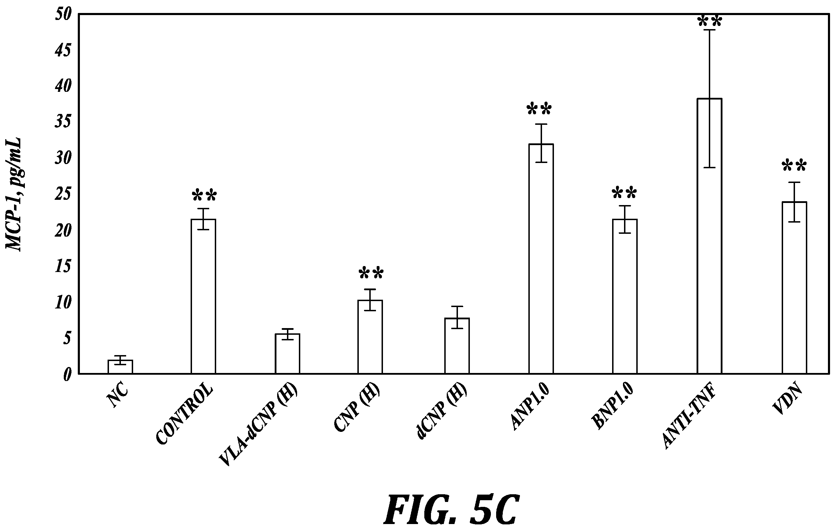

FIGURE 5C is a bar graph showing that bolus administration of VLA-dCNP and dCNP or treatment attenuated LPS-induced upregulation of inflammatory cytokines (MCP-1) in BALF to facilitate resolution of ARDS/ALI. The protocol was the same as that described in FIGURE 5A, with the exception that bronchoalveolar lavage fluid (BALF) was harvested and measured for MCP-1 cytokines.

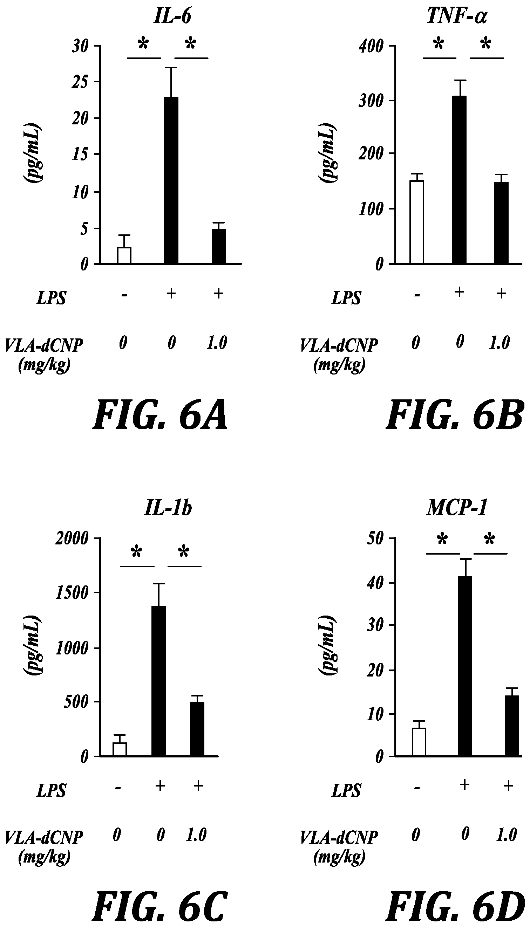

FIGURES 6A-6D are bar graphs showing that bolus administration of VLA-dCNP or treatment attenuated LPS-induced upregulation of inflammatory cytokines in lung tissue to facilitate resolution of ARDS/ALI. Male C57BL/6J mice (6 week) were treated with LPS (0.05 mg/kg intratracheal administration) and then treated with VLA-dCNP (1.0 mg/kg s.c.). 24 hours after treatment, lung tissue was harvested. Each cytokine concentration in extracted lung protein was measured by using ELISA kits. These cytokines were interleukin-6 (IL-6), tumor necrosis factor a (TNF-α), interleukin-1β (IL-1β) and macrophage chemoattractant protein-1 (MCP-1). Statistical analysis was based on Student's t-test (n = 10, 10, 9; NC, Control, VLA-dCNP. * P < 0.05 vs Control).
FIGURE 6A is a bar graph showing bolus administration of VLA-dCNP or treatment attenuated LPS-induced upregulation of IL-6 in lung tissue to facilitate resolution of ARDS/ALI.
FIGURE 6B is a bar graph showing bolus administration of VLA-dCNP or treatment attenuated LPS-induced upregulation of TNF-α in lung tissue to facilitate resolution of ARDS/ALI.
FIGURE 6C is a bar graph showing bolus administration of VLA-dCNP or treatment attenuated LPS-induced upregulation of MCP-1 in lung tissue to facilitate resolution of ARDS/ALI.
FIGURE 6D is a bar graph showing bolus administration of VLA-dCNP or treatment attenuated LPS-induced upregulation of IL-1b in lung tissue to facilitate resolution of ARDS/ALI.

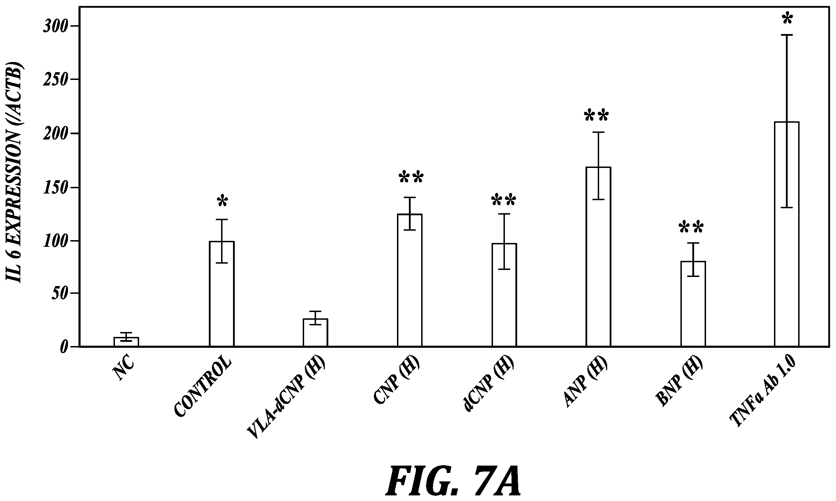

FIGURE 7A is a bar graph showing that bolus administration of VLA-dCNP attenuated LPS-elicited inflammatory cytokine expression including IL-6 that is commonly regulated by NFkb systems, the master regulator of inflammation systems suggesting that VLA-dCNP broadly suppressed inflammation response in the subject's body to facilitate resolution of ARDS/ALI. Measurement of inflammatory related gene expression in ALI lung tissue. Male C57BL/6J mice (6 week) were treated with LPS (0.05 mg/kg intratracheal administration) and then treated with very long acting CNP derivative or VLA-dCNP (1.0 mg/kg s.c.), native C-type natriuretic peptide or CNP (1.0 mg/kg s.c.), CNP derivative or dCNP (1.0 mg/kg s.c.), atrial natriuretic peptide or ANP (1.0 mg/kg s.c.), B-Type natriuretic peptide or BNP (1.0 mg/kg s.c.), tumor necrosis factor alpha antibody or TNFα ab (1.0 mg/kg s.c.), and cyclic-GMP degradation inhibitor or PDE5 inhibitor called Vardenafil (VDN) (1.0 mg/kg s.c.). After 24 hours from the treatment, lung tissue was harvested. Total RNA was extracted from harvested lung tissue. Statistical analysis was based on Student's t-test. (n = 15, 22, 6, 6, 6, 6, 6, 5 and 9; NC, Control, CNP, dCNP, ANP, BNP, TNFα ab, VDN, and VLA-dCNP. * P < 0.01 vs. VLA-dCNP and ** P< 0.05 vs. VLA-dCNP).
FIGURE 7B is a bar graph showing that bolus administration of VLA-dCNP attenuated LPS-elicited inflammatory cytokine expression including iNOS, suggesting that VLA-dCNP broadly suppressed inflammation response in a subject to facilitate resolution of ARDS/ALI. The protocol was as described for FIGURE 7A.
FIGURE 7C is a bar graph showing that bolus administration of VLA-dCNP attenuated LPS-elicited inflammatory cytokine expression including MCP-1, suggesting that VLA-dCNP broadly suppresses inflammation response in the subject's body to facilitate resolution of ARDS/ALI. The protocol was as described for FIGURE 7A.
FIGURE 7D is a bar graph showing that bolus administration of VLA-dCNP attenuated LPS-elicited inflammatory cytokine expression including IL-1b, suggesting that VLA-dCNP broadly suppressed inflammation response in the subject's body to facilitate resolution of ARDS/ALI. The protocol was as described for FIGURE 7A.
FIGURE 7E is a bar graph showing that bolus administration of VLA-dCNP attenuated LPS-elicited inflammatory cytokine expression including IFNg, suggesting that VLA-dCNP broadly suppressed inflammation response in the subject's body to facilitate resolution of ARDS/ALI. The protocol was as described for FIGURE 7A.
FIGURE 8 is a series of bar graphs showing that bolus administration of VLA-dCNP suppressed inflammation levels in lung tissue to facilitate resolution of ARDS/ALI. Male C57BL/6J mice (6 week) were treated with LPS (0.05 mg/kg intratracheal administration) and treated with VLA-dCNP (1.0 mg/kg s.c.). At 24 hours after treatment, lung tissue was harvested. Western blot analysis was performed by using antibody Elf-1, Tollip, IRAK-1, P-P38, P-P65 and β-actin (internal standard). Statistical analysis was based on Student's t-test (n = 5, * P < 0.05 vs Cont.).
FIGURE 9 is a series of bar graphs showing that bolus administration of VLA-dCNP suppressed STAT levels in lung tissue to facilitate resolution of ARDS/ALI. Male C57BL/6J mice (6 week) were treated with LPS (0.05 mg/kg intratracheal administration) and treated with VLA-dCNP (1.0 mg/kg s.c.). At 24 h after treatment, lung tissue was harvested. Western blot analysis was performed by using antibody anti-STAT-1, P-STAT-1, STAT-2, STAT-3, STAT-6 and β-actin (internal standard). Statistical analysis was based on Student's t-test (n = 5, * P < 0.05 vs Control).

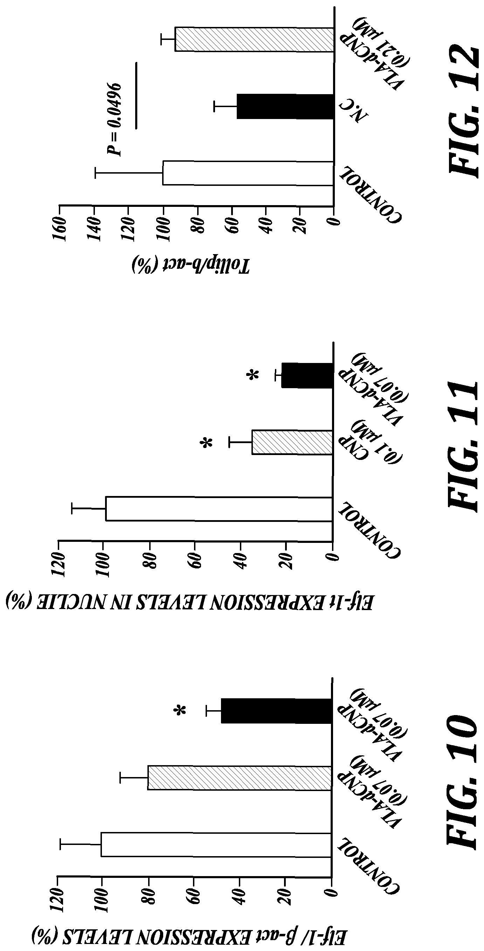

FIGURE 10 is a bar graph showing that bolus administration of VLA-dCNP suppressed Elf-1 expression in human umbilical vein endothelial cells. Human umbilical vein endothelial cells (HUVECs) were as maintained in HuMedia-EG2 and inoculated into 12 well plates (1 X 105 cells/well in 2 mL in HuMedia-EG2). After 24 hours, cells were treated with each concentration of VLA-dCNP (0.07uM (0.21 µg/mL) or 0.7uM (2.1 µg/mL)) (in M199 1%BSA) for 6 hours. Protein levels were assessed by western blot analysis by using anti-Elf-1 and β-actin (internal standard). Statistical analysis was based on Student's t-test (n = 4, * P < 0.05 vs Control).
FIGURE 11 is a bar graph showing that bolus administration of VLA-dCNP suppressed Elf-1 levels in nuclei of human umbilical vein endothelial cells. Human umbilical vein endothelial cells (HUVECs) were maintained in HuMedia-EG2. Cells were plated into grass bottom dish at the density of 1 X 10⁵ cells/well in 2 mL in HuMedia-EG2. After 24 hours, cells were treated with each concentration of VLA-dCNP (0.07uM (0.21 µg/mL)) or CNP 0.1µM (0.21µg/mL)) in M199 (Thermo Fisher Scientific, Waltham MA) supplemented with 1%BSA (Sigma-Aldrich, St. Louis MO) for 6 hours. Cells were fixed by 4% paraformaldehyde and treated with anti-Elf-1 Ab (Santa Cruz Biotechnology, Dallas TX) followed by incubation with Alexa Fluor 488 labeled-secondary antibody (Thermo Fisher Scientific, Waltham MA) and Hoechst 33342.
FIGURE 12 is a bar graph showing that bolus administration of VLA-dCNP elicits Tollip expression in human lung fibroblast cell line HFL1. Human lung fibroblast HFL1 (1.0 x 105 cells /well) was cultured with DMEM medium for 16 hours and then incubated with 1% BSA-M199 medium with 0.21 µM (0.66 ug/mL) VLA-dCNP and without VLA-dCNP (N.C.). After a 12-hour incubation, cells were stimulated with LPS (final concentration of 1.0 µg/mL). After another 2-hour incubation, cells were harvested and lysed. The amount of protein expression in the cells were evaluated by western blotting with anti Tollip and β-actin (internal standard). Statistical analysis was based on Student's t-test (n = 4, * P < 0.05 vs Cont.).
FIGURE 13A is a graph showing that bolus administration of VLA-dCNP had protective effect on LPS-induced sepsis lethality. Balb/c (11 week-old male) mice were treated with LPS (10 mg/kg i.p.) and treated with each dose of VLA-dCNP (Low 0.1 mg/kg s.c.; Medium 0.3 mg/kg s.c.; High 1.0 mg/kg s.c.). Survival was observed every 2 hours. Statistical analysis was performed by Log rank test based on Graphpad Prism 6.0 (n = 10, 10, 10, 11).
FIGURE 13B is a graph showing C57BL/6J (6 week-old male) mouse treated with LPS (15 mg/kg i.p.) and treated with a given bolus dose of VLA-dCNP (Low 0.1 mg/Kg s.c.; Medium 0.3 mg/kg s.c.; High 1.0 mg/Kg s.c.). Survival was observed every 2 hours. Statistical analysis was performed by Log rank test. (n = 11, 10, 11, 11). VLA-dCNP had protective effect on LPS-induced sepsis.

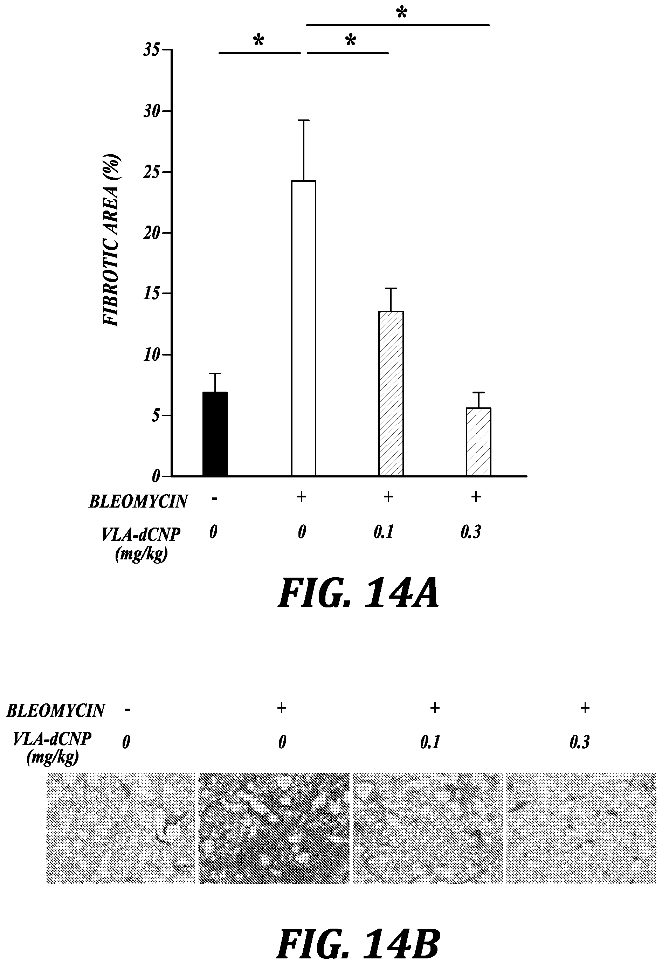

FIGURE 14A is a bar graph showing that bolus administration of VLA-dCNP decreased fibrotic area in the lung in this animal model of interstitial lung disease (ILD) or idiopathic pulmonary fibrosis (IPF). Male C57BL/6J mice (6 week) were treated with bleomycin (1.0 mg/kg intratracheal administration) and treated with each dose of VLA-dCNP (0.1 mg/kg s.c. and 0.3 mg/kg s.c). VLA-dCNP was administered at 7^{th} day after bleomycin administration (5 times/week). At 21^{st} day, mice were sacrificed, and lung tissue was harvested and performed Masson's Trichrome staining. Fibrosis area was measured by using Image J (NIH, Bethesda, Maryland, USA). Statistical analysis was based on Dunnett's test performed by using GraphPad Prism 6. (n = 5, 8, 9, 7; Negative Control, Control, VLA-dCNP 0.1, and VLA-dCNP 0.3. * P < 0.05 vs Control.).
FIGURE 14B is a series of micrographs showing the Masson's trichrome stained lung tissue samples of FIGURE 14A. Blue and light blue in the lung tissue indicate advanced collagen/fibrosis.

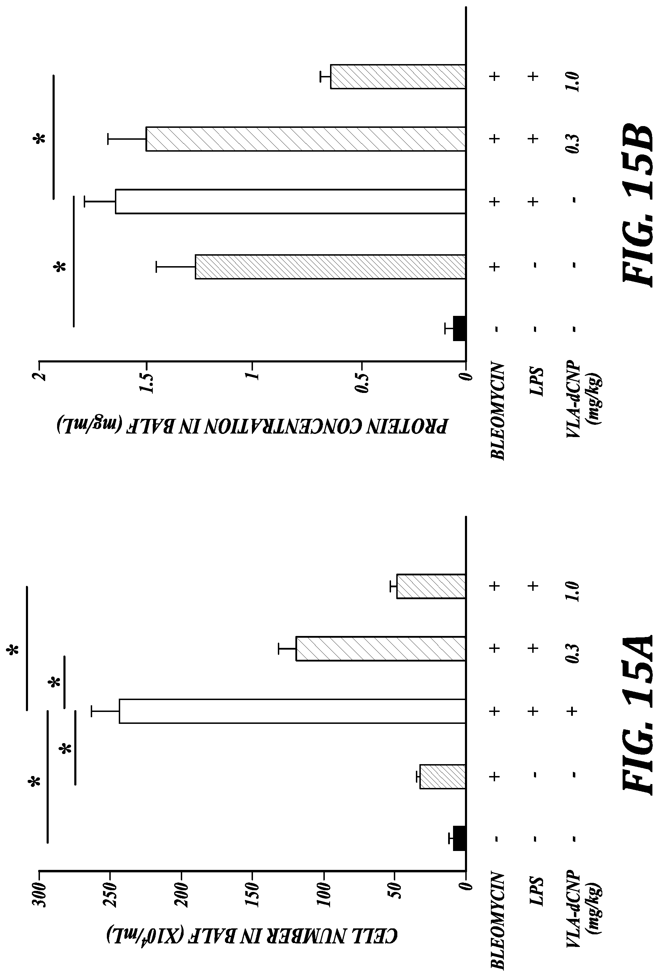

FIGURE 15A is a bar graph showing that bolus administration of VLA-dCNP decreased cell numbers in BALF from acute exacerbations of idiopathic pulmonary fibrosis (IPF-AE) model. Male C57BL/6J mice (6 week) were treated with Bleomycin (1.0 mg/kg intratracheal administration) and after 3 week, mice were treated with LPS (0.05 mg/kg intratracheal administration) and treated with each dose of VLA-dCNP (0.3 mg/kg s.c. and 1.0 mg/kg s.c.). VLA-dCNP was administered right after LPS administration. After 24 h treatment, mice were sacrificed. Statistical analysis was based on Student's t test performed by using GraphPad Prism 6 (n = 6, 6, 9, 9, 9; Negative Control, Bleomycin, Control, VLA-dCNP 0.3, and VLA-dCNP 1.0. * P < 0.05 vs Control.)
FIGURE 15B is a bar graph showing that bolus administration of VLA-dCNP decreased protein levels in BALF from Acute exacerbations of idiopathic pulmonary fibrosis (IPF-AE) model. The protocol is as described for FIGURE 15A.
FIGURE 15C is a bar graph showing that VLA-dCNP attenuated IL-6 in BALF from acute exacerbations of idiopathic pulmonary fibrosis (IPF-AE) model. The protocol is as described for FIGURE 15A.
FIGURE 15D is a bar graph showing that bolus administration of VLA-dCNP decreased cell numbers and protein levels and attenuated TNFα in BALF from acute exacerbations of idiopathic pulmonary fibrosis (IPF-AE) model. The protocol is as described for FIGURE 15A.
FIGURE 16A is a series of micrographs of kidney tissue.
FIGURE 16B is a graph showing tubular injury as a function of bolus administration of VLA-dCNP in a model of acute kidney injury.
FIGURE 17A is a bar graph showing significant decrease in liver enzyme aspartate aminotransferase (AST) in a diet-induced model of liver fibrosis, when VLA-dCNP or long acting CNP is administered to subjects.
FIGURE 17B is a bar graph showing significant decrease in liver enzyme alanine aminotransferase (ALT) in a diet-induced model of liver fibrosis, when VLA-dCNP or long acting CNP is administered to subjects.
FIGURE 17C is a bar graph showing significant decrease in alpha smooth muscle actin (a-SMA) in a diet-induced model of liver fibrosis, when VLA-dCNP or long acting CNP is administered to subjects.
FIGURE 17D is a bar graph showing significant decrease in tumor necrosis growth factor alpha (TNF-a), a marker of inflammation inducing fibrosis, in a diet-induced model of liver fibrosis, when VLA-dCNP or long acting CNP is administered to subjects.
FIGURE 17E is a bar graph showing significant decrease in monocytes chemoattractant protein 1 (MCP-1), a mediator of macrophage-induced inflammation in liver tissue, in a diet-induced model of liver fibrosis, when VLA-dCNP or long acting CNP is administered to subjects.
FIGURE 18A is a bar graph showing significant improvement in kidney function based on decrease in serum creatinine, when VLA-dCNP or long acting CNP is administered to subjects.
FIGURE 18B is a bar graph showing significant improvement in kidney function based on decreased albumin level in urine by calculating albumin-to-creatinine ratio, when VLA-dCNP or long acting CNP is administered to subjects.
FIGURE 18C is a bar graph showing significant decrease in % fibrosis area in kidney, when VLA-dCNP or long acting CNP is administered to subjects. Fibrosis area was measured by using Image J (NIH, Bethesda, Maryland, USA);
FIGURE 18D is a series of representative images of Masson's Trichrome (MT) stain of kidneys. Magnification is X20. In this Masson's Trichrome stain, the nucleus is stained with iron hematoxylin (brown/black color in the image), cytoplasm is stained with acid fuchsin (pink/red color in the image), and collagen fibrotic area is stained with aniline blue (blue color in the image).
FIGURE 19A is a bar graph showing significant decrease in fibrosis based on a decrease in hydroxyproline, a maj or component of the collagen, in lung tissue, when VLA-dCNP or long acting CNP is administered to subjects.
FIGURE 19B is a bar graph showing a significant decrease in the % fibrosis area in lungs, when VLA-dCNP or long acting CNP is administered to subjects, based on quantification of evaluation of histological Masson's Trichrome staining of lung tissue sections. Fibrosis area was measured by using Image J (NIH, Bethesda, Maryland, USA).
FIGURE 19C is series of representative images of Masson's Trichrome (MT) stained kidneys at magnification is X20.
FIGURE 20 is a graph of plasma CNP [mean (SEM); n= 5] in CD-1 mice after subcutaneous administration of 2.0mg/Kg of CNP derivative s1 (dCNP-s1), and CNP derivative s2 (dCNP-s2). The inset shows the low plasma level of CNP (diamond) when native CNP was administered. Error bars represent standard error of the mean of n=5 plasma samples. Baseline CNP level prior to administration was 0.391 (0.02) ng/mL [mean (SEM); n=10]. Long acting dCNP-s1 and dCNP-s2 provides 10-fold higher blood level of CNP in a sustain manner (at least 8 hours) than native CNP when given at similar dose weight/Kg dose.

### DETAILED DESCRIPTION

The present disclosure relates to the treatment of lung, liver, and/or kidney injury, or a symptom associated with a lung, liver, and/or kidney injury, such as acute lung injury (ALI) and the prevention of its deterioration to a more severe form, namely Acute Respiratory Distress Syndrome (ARDS) and death or lung/liver/kidney fibrosis, by administering to a subject in need thereof a therapeutically effective amount of a long acting C-type natriuretic peptide (CNP), CNP derivative, long acting CNP derivative, or long acting CNP receptor (NPRB) agonist. The disclosure also relates to the treatment of non-cardiovascular causes of low blood oxygenation, elevated levels of inflammatory cells in the lung, pulmonary edema, sepsis, bacteremia, and/or fibrosis *(e.g.,* non-cardiovascular causes of low blood oxygenation, elevated levels of inflammatory cells in the lung, pulmonary edema, and/or fibrosis) using the same.

The present disclosure is also related to treatment of fibrosis in general, including lung fibrosis, liver fibrosis, cirrhosis, and kidney glomerular sclerosis, and treatment of/protection from kidney injury, including administering a therapeutically effective amount of the compositions of the present disclosure as a bolus, without decreasing blood pressure by more than 20% *(e.g.,* by more than 15%, by more than 10%, or by more than 5%) of a baseline blood pressure measurement, where the baseline blood pressure measurement is an average blood pressure prior to administration of the composition, and providing an increase in plasma cyclic-GMP level at from 1 hour to 12 hours (e.g., 2 to 12 hours, 4 to 12 hours, 1 hour to-24 hours, 2 to 24 hours, 4 to 24 hours, 1 hour to 84 hours, 2 to 84 hours, 4 to 84 hours, 12 to 84 hours, 1 hour to 168 hours, 2 to 168 hours, 4 to 168 hours, or 12 to 168 hours) after administration to above 1.5x (e.g., above 2x, above 3x, above 4x, or above 5x) of a baseline plasma cyclic-GMP level, wherein the baseline plasma cyclic-GMP level is an average plasma cyclic-GMP level prior to administration of the composition or the average plasma cyclic-GMP level of a healthy subject (preferably the average plasma cyclic-GMP level prior to administration of the composition for the subject).

Unlike conventional methods for treating the ALI and sepsis, a therapeutically effective amount of the compositions of the present disclosure can be administered as a bolus, either before, during, and/or after an injury that would lead to acute lung injury (ALI), acute respiratory distress syndrome (ARDS), pulmonary edema, elevated level of inflammatory cells in the lung, increased level or expression of inflammatory cytokines in the lung (compared to healthy lung), increased protein level in lung alveolar space (compared to healthy lung), low arterial blood oxygenation (wherein low arterial blood oxygenation is a blood PaO2 of below 60 mm Hg and/or a blood hemoglobin oxygen saturation (SpO2) of below 90%), sepsis, bacteremia, pneumonia, lung/pulmonary fibrosis, idiopathic pulmonary fibrosis (IPF), or interstitial lung disease (ILD), without decreasing blood pressure by more than 20% (e.g., by more than 15%, by more than 10%, or by more than 5%) of a baseline blood pressure measurement, where the baseline blood pressure measurement is an average blood pressure prior to administration of the composition. The therapeutically effective amount of the compositions of the present disclosure can also increase plasma cyclic-GMP level at from 1 hour to 12 hours (e.g., 2 to 12 hours, 4 to 12 hours, 1 hour to-24 hours, 2 to 24 hours, 4 to 24 hours, 1 hour to 84 hours, 2 to 84 hours, 4 to 84 hours, 12 to 84 hours, 1 hour to 168 hours, 2 to 168 hours, 4 to 168 hours, or 12 to 168 hours) after administration to above 1.5x (e.g., above 2x, above 3x, above 4x, or above 5x) of a baseline plasma cyclic-GMP level, wherein the baseline plasma cyclic-GMP level is an average plasma cyclic-GMP level prior to administration of the composition or the average plasma cyclic-GMP level of a healthy subject (preferably the average plasma cyclic-GMP level prior to administration of the composition for the subject). In some embodiments, the therapeutically effective amount of the compositions of the present disclosure can be administered as a bolus after an injury that would lead to the aforementioned conditions. In some embodiments, the therapeutically effective amount of the compositions of the present disclosure can be administered as a bolus before an injury that would lead to the aforementioned conditions. In some embodiments, the therapeutically effective amount of the compositions of the present disclosure can be administered as a bolus during an injury that would lead to the aforementioned conditions. Unlike conventional methods of continuous administration, bolus administration of the compositions herein provides advantages such as ease of administration, with an unexpected decrease in undesirable side effects (such as hypotension).

### Definitions

At various places in the present specification, substituents of compounds of the disclosure are disclosed in groups or in ranges. It is specifically intended that the disclosure include each and every individual subcombination of the members of such groups and ranges. For example, the term "C₁₋₆ alkyl" is specifically intended to individually disclose methyl, ethyl, C₃ alkyl, C₄ alkyl, C₅ alkyl, and C₆ alkyl.

One letter codes for amino acids are used herein. For example, alanine is A, arginine is R, asparagine is N, aspartic acid is D, cysteine is C, glutamic acid is E, glutamine is Q, glycine is G, histidine is H, isoleucine is I, leucine is L, lysine is K, methionine is M, phenylalanine is F, proline is P, serine is S, threonine is T, tryptophan is W, tyrosine is Y, valine is V, and γE is glutamic acid where the R-group *(i.e.,* side chain) carboxyl (gamma, γ) is the moiety used to link to any of the primary amino group of a peptide or to the N-terminal portion of a peptide rather than the alpha-carboxyl. For the purpose of the present application, the one letter codes for amino acids includes L and/or D amino acid stereoisomers. It is understood that when the amino acids combine to form a peptide, the amino acids are referred to as amino acid residues where the elements of water are removed. Furthermore, where the present disclosure refers to an amino acid in a peptide sequence, it is understood to be an amino acid residue.

As used herein, the term "aliphatic" refers to a compound or group containing carbon and hydrogen joined together in straight chains, branched chains, or non-aromatic rings. Aliphatic compounds or groups may be saturated (e.g., an alkane such as hexane and other alkanes, an alkyl such as hexyl and other alkyls) or unsaturated (e.g., hexene and other alkenes, as well as alkynes, hexenyl and other alkenyl, as well as alkynyl). The aliphatic compound or group (e.g., an alkyl, alkenyl, or alkynyl) can be substituted, for example, with 1, 2, 3, 4, 5, 6, 7, or 8 substituents such as (=O), hydroxyl, carboxyl, carbonyl, and/or an ester group. For example, the aliphatic group can have a carboxyl group as a substituent as a pendant group and/or at a terminus. When the aliphatic group is part of a compound, it is understood that the aliphatic group can be covalently bound to the compound via a chemical linkage, such as a carbonyl (C=O, also represented by C(O) or C(=O)) *(e.g.,* as part of an amide or an ester linkage), a thioether, an ether, a thioether, a carbamate moiety, a bond, or the like. It is understood that the number of carbons in the aliphatic chain includes the backbone carbons in the chemical linkage. For example, a saturated C8 aliphatic chain that includes a C(=O) linkage, when linear, can be represented by CH₃(CH₂)₆C(=O). As another example, a saturated C8 aliphatic chain that has a carboxyl group at a first terminus and that includes a C(=O) linkage at a second terminus, when linear, can be represented by HOC(=O)(CH₂)₆C(=O). For example, a saturated C18 aliphatic chain that includes a C(=O) linkage, when linear, can be represented by CH₃(CH₂)₁₆C(=O). As another example, a saturated C18 aliphatic chain that has a carboxyl group at a first terminus and that includes a C(=O) linkage at a second terminus, when linear, can be represented by HOC(=O)(CH₂)₁₆C(=O). The aliphatic group can be derived from a fatty acid and/or the aliphatic group can be derived from a diacid.

As used herein, the term "alkyl" refers to a saturated hydrocarbon group which is straight-chained (e.g., linear) or branched. Example alkyl groups include methyl (Me), ethyl (Et), propyl *(e.g.,* n-propyl and isopropyl), butyl *(e.g.,* n-butyl, isobutyl, t-butyl), pentyl (e.g., n-pentyl, isopentyl, neopentyl), and the like. An alkyl group can contain from 1 to about 30, from 1 to about 24, from 2 to about 24, from 1 to about 20, from 2 to about 20, from 1 to about 10, from 1 to about 8, from 1 to about 6, from 1 to about 4, or from 1 to about 3 carbon atoms.

As used herein, the term "fatty acid" refers to an aliphatic chain that is substituted with a carboxyl group, which is either saturated or unsaturated. Examples of fatty acids includes caprylic acid, capric acid, lauric acid, myristic acid, palmitic acid, stearic acid, arachidic acid, behemic acid, and/or lignoceric acid.

As used herein, the term "fatty acid ester" refers to a long aliphatic chain (saturated or unsaturated) having a -C(=O)O- moiety at an end of the chain.

As used herein, the term "fatty acid amide" refers to a long aliphatic chain (saturated or unsaturated) having a -C(=O)NR- moiety at an end of the chain.

As used herein, the term "individual," "subject," or "patient," used interchangeably, refers to any animal, including mammals, preferably mice, rats, other rodents, rabbits, dogs, cats, swine, cattle, sheep, horses, or primates, and most preferably humans.

As used herein, the phrase "therapeutically effective amount" refers to an amount of a therapeutic agent (i.e., drug, or therapeutic agent composition) that elicits the biological or medicinal response that is being sought in a tissue, system, animal, individual or human by a researcher, veterinarian, medical doctor or other clinician, which includes one or more of the following:
(1) preventing the disease; for example, preventing a disease, condition or disorder in an individual who may be predisposed to the disease, condition or disorder but does not yet experience or display the pathology or symptomatology of the disease;
(2) inhibiting the disease; for example, inhibiting a disease, condition or disorder in an individual who is experiencing or displaying the pathology or symptomatology of the disease, condition or disorder; and
(3) ameliorating the disease; for example, ameliorating a disease, condition or disorder in an individual who is experiencing or displaying the pathology or symptomatology of the disease, condition or disorder *(i.e.,* reversing the pathology and/or symptomatology) such as decreasing the severity of disease, prolonging survival time, and/or preventing death.

As used herein, the term "bolus dose" refers to a single dose of a drug or other substance given or administered over a short period of time, for example, less than 10 minutes (e.g., less than 8 minutes, less than 5 minutes, less than 3 minutes, or less than 1 minute). In some embodiments, a bolus dose is administered in less than 5 minutes. In some embodiments, a bolus dose is administered in less than 3 minutes. In some embodiments, a bolus dose is administered in less than 1 minute. Administration includes one of: injection in any part of the body (including but not limited to blood vessels, subcutaneous, intrathecal, or intradermal), orally (as a dosage form), inhalation (e.g., by intratracheal inhalation administration, where a subject is exposed to high aerosol concentrations such that the active pharmaceutical ingredient is deposited directly in the lower respiratory tract), or nasally *(e.g.,* as an aerosol, liquid, or powder).

As used herein, the term "a blood pressure drop," "a drop in blood pressure," or "hypotension" are used interchangeably, and refer to a statistically significant decrease in blood pressure in a subject below a baseline blood pressure. The baseline blood pressure is the mean blood pressure measured prior to treatment or administration of any drug to a subject, or the mean blood pressure of a normal healthy subject. The standard deviation of most blood pressure measuring device can be between 5-15% depending on the method of measurement and position, state of mind, or movement of the subject during measurement. For the clarity of the present specification the change in blood pressure will be expressed as statistically significant percent increase, decrease, or drop in blood pressure from the mean/average baseline blood pressure prior to drug or test article administration. Statistically significant means that P<0.05 as known to those skilled in the art of statistics.

As used herein, the term "C-type natriuretic peptide" or "CNP" is a peptide including 22 amino acid residues, having a 17 amino acid residue ring structure formed by a disulfide bond, and an additional 5-amino acid residue extension at the N-terminal (GLSKGCFGLKLDRIGSMSGLGC [SEQ ID NO. 10]; where the letters are in accordance with conventional amino acid nomenclature, and the amino acid residues C-6 (at position 6) and C-22 (at position 22) are linked by a disulfide bond). *See, e.g.,* Sudoh et al., Biochem. Biophys. Res. Commun. 1989; 159:1427-1434.

As used herein, the terms "NPRB receptor," "natriuretic peptide receptor B (NPRB)," or "NPR2," "guanylate cyclase B (GC-B)," or "B-type natriuretic peptide receptor 2" (NPR2) are used interchangeably. In humans, a NPRB receptor is encoded by NPR2 gene, which is located on chromosome 9 and in mouse on chromosome 4. *See, e.g.,* Nuglozeh et al., Genome. 1997; 8:624-625. The expression of NPRB is reported in various organs such as heart, brain, uterus, ovary, kidney, lungs, liver and adipocytes and in some cancers. Schulz et al., Cell. 1989; 58:1155- 1162; Nagase et al., J. Hypertens. 1997; 15:1235-1243; Chrisman et al., J. Biol. Chem. 1993; 268:3698-3703. NPRB is selectively activated by CNP and not by ANP or BNP (the other known natriuretic peptides). The ubiquitous expression of NPRB signifies its role in many physiological functions. While the other natriuretic peptide receptor, NPRA, is activated by physiological concentrations of ANP and BNP, NPRA is not activated by CNP. The differences in the physiological consequences of activation of one versus both NPRA and NPRB receptors remains unclear prior to the present disclosure, making the methods of the present disclosure non-obvious and inventive.

As used herein, the term "long-acting C-type natriuretic peptide" or "long acting CNP" refers to a CNP formulation that when administered as a single bolus dose to a mammalian subject (human, non-human, primate, dogs, rats, mice, etc.), the resulting elevation of CNP level in the plasma or elevation of plasma cyclic-GMP level above the baseline will be sustained for a duration of greater than 4 hours or greater than 6 hours depending on the species. A long-acting C-type natriuretic peptide or a long acting CNP encompasses a very long-acting C-type natriuretic peptide or a very long acting CNP. The elevation of plasma cyclic-GMP is a result of CNP structure activity itself, or from the combination of the CNP with one or more components of a formulation containing the CNP. The presence in the plasma (or elevation) means a detectable presence over and above the analytical baseline level, wherein the baseline level is the level measured in the absence of long-acting CNP formulation administration. The length of sustained plasma cyclic-GMP elevation is the duration of biological activity of the CNP formulation. A CNP formulation refers to a composition containing a CNP peptide with one or more excipient or carrier such as a polymer, protein, sugar, detergent, and/or buffer. The CNP in the CNP formulation may or may not be covalently linked to excipient or carrier. The sustained presence in the blood can be evaluated by pharmacokinetic/pharmacodynamic analysis after administration.

As used herein, a formulation containing a "very long-acting C-type natriuretic peptide" or "very long acting CNP" refers to a long-acting CNP formulation containing the 22 amino acid residue CNP formulated in such a way that when administered as a single bolus dose to subject, will have sustained presence in the plasma or sustained plasma cyclic-GMP elevation over the baseline of 24 hours or greater (e.g., up to 2-3 days or up to 1-4 weeks). Thus, a very long-acting C-type natriuretic peptide or very long acting CNP is a subset of a long-acting C-type natriuretic peptide or long acting CNP. The presence in the plasma means a detectable presence over and above the endogenous native agonist that are normally made by the subject or an analytical baseline level in the absence of administration of a therapeutic CNP formulation. The duration *(i.e.,* length of time) of plasma cyclic-GMP elevation or the presence of detectable CNP over the baseline can be from 24 to 192 hours, or 24 to 48 hours, or 48 to 72 hours, or 72 to 96 hours, or 96-120 hours, or 120 to 144 hours, 144 to 168 hours, or 168 to 192 hours. As described above, a CNP formulation is a composition containing CNP peptide with one or more excipient or carrier such as polymer, protein, sugar, detergent, and/or buffer. The CNP in CNP formulation may or may not be covalently linked to excipient or carrier. The sustained presence in the blood can be evaluated by pharmacokinetic/pharmacodynamic analysis after administration.

As used herein, the term "long acting CNP derivative" is a CNP derivative that when administered as a single bolus dose to a mammalian subject or patient has sustained presence in the plasma or sustained plasma cyclic-GMP elevation over the baseline of greater than 4 hours, or greater than 6 hours, depending on the species. A long acting CNP derivative encompasses a very long-acting CNP derivative. The long-acting nature can result from the CNP derivative structure itself, or from the combination of the CNP derivative with one or more components of a formulation containing the CNP derivative. The presence in the plasma or blood refers to a detectable presence over the endogenous native agonist that are normally made by the mammals or above an analytical baseline level in the absence of administration of a therapeutic compound, peptide, protein or formulation. The sustained presence in the blood can be evaluated by pharmacokinetic/ pharmacodynamic analysis after administration. In some embodiments, the CNP derivative is a modified CNP with at least 72% (e.g., at least 80%, at least 85%, at least 90%, at least 95%, or at least 98%) sequence homology or identity to native CNP. In some embodiments, the CNP derivative is an addition derivative where a native CNP is modified by covalent addition of a chemical moiety, such as one or more additional amino acids and/or fatty acids and/or any chemical moiety and/or moieties at the N-terminal, C-terminal, or in the R-group of any amino acid residue in the CNP peptide. In some embodiments, the CNP derivative includes a substitution derivative where 1 to 6 amino acid residues (or 5 to 28% of the amino acid residues) in native CNP is replaced by different or unnatural amino acid residues. In certain embodiments, the CNP derivative includes a subtraction derivative where 1 to 6 amino acid residues (or 5 to 28% of the amino acid residues) in a native CNP are deleted. In certain embodiments, the CNP derivative includes a subtraction derivative where 1 to 6 amino acid residues (or 5 to 28% of the amino acid residues) in a native CNP are deleted and/or substituted. A CNP derivative formulation refers to a composition containing a CNP derivative with one or more excipient or carrier such as polymer, protein, sugar, detergent, or buffer.

As used herein, the term "very long acting CNP derivative" refers to a long acting CNP derivative or CNP derivative, that when administered as a single bolus dose to mammalian subject or patient, has sustained presence in the plasma or sustained plasma cyclic-GMP elevation over the baseline that has a duration of 24 hours or greater. Thus, a very long acting CNP derivative is a subset of long acting CNP derivative. The very long acting CNP derivative can result from the CNP derivative structure itself, or from the combination of the CNP derivative with one or more components of a formulation containing the CNP derivative. The presence in the plasma refers to a detectable presence over an analytical baseline plasma level in the absence of administration of the very long acting CNP derivative. The duration of plasma cyclic-GMP elevation or the presence of detectable CNP derivative over the baseline can be from 24 to 192 hours, or 24 to 48 hours, or 48 to 72 hours, or 72 to 96 hours, or 96-120 hours, or 120 to 144 hours, 144 to 168 hours, or 168 to 192 hours. The sustained presence in the blood can be evaluated by pharmacokinetic/pharmacodynamic analysis after administration. In some embodiments, the CNP derivative includes a modified CNP with 72% (e.g., at least 80%, at least 85%, at least 90%, at least 95%, or at least 98%) sequence identity to native CNP. In some embodiments, the CNP derivative is an addition derivative where native CNP is modified by covalent addition of chemical moiety such as additional amino acids and/or fatty acid and/or any chemical moiety and/or moieties at N-terminal, C-terminal, or in the R-group of any amino acid residue in the CNP peptide. In some embodiments, the CNP derivative is a substitution derivative where 1-6 amino acid residues (or 5-28% of the amino acid residues) in native CNP is replaced by different or unnatural amino acid residues. In certain embodiments, the CNP derivative is a subtraction derivative where 1-6 amino acid residues (or 5-28% of the amino acid residues) in native CNP were deleted. In certain embodiments, the CNP derivative includes a subtraction derivative where 1 to 6 amino acid residues (or 5 to 28% of the amino acid residues) in a native CNP are deleted and/or substituted. CNP derivative formulation is a composition containing CNP derivative with one or more excipient or carrier such as polymer, protein, sugar, detergent, or buffer.

As used herein, the term "formulation of a CNP" or a "formulation of a CNP derivative" refers to a composition containing CNP peptide or its derivative that may or may not be covalently linked to an excipient or carrier such as polymer, protein, and/or lipid.

As used herein, the term "NPRB agonist" or "NPR2 agonist" refers to any compound, peptide or protein that does not contain the 22 amino acid residue CNP sequence in its structure and that can bind to NPRB, a cell catalytic receptor, and stimulate its intracellular guanylyl cyclase activity to increase intracellular or blood cyclic-GMP level, but with limited or no capability to bind and stimulate NPRA receptor. Since not all cells express similar levels of NPRB, the NPRB agonist is tailored to primarily affect those cells expressing NPRB. This selectivity can be readily measured by those skilled in the art by measuring the activity in cells that expresses NPRB, compared to activity in cells that expresses NPRA.

As used herein, the term "long acting NPRB agonist" refers to an NPRB agonist defined above, that, when administered as a single bolus dose to a mammalian subject or patient has sustained presence in the plasma or sustained plasma cyclic-GMP elevation over the baseline of greater than 4 hours or greater than 6 hours depending on the species. A long acting NPRB agonist encompasses a very long acting NPRB agonist. The long acting nature of the NPRB agonist can result from the NPRB agonist structure itself, or from the combination of the NPRB agonist with one or more components of a formulation containing the NPRB agonist. The presence in the plasma means a detectable presence over an analytical baseline level in the absence of administration of a long acting NPRB agonist. A formulation of a long acting NPRB agonist or a long acting NPRB agonist formulation is a composition containing a long acting NPRB agonist, or a long acting NPRB agonist with one or more an excipient or carrier such as a polymer, protein, sugar, lipid, or buffer. The long acting NPRB agonist may or may not be covalently linked to excipient or carrier. The sustained presence in the blood can be evaluated by pharmacokinetic/pharmacodynamic analysis after administration. The sustained plasma elevation of cyclic-GMP above the baseline can be evaluated by pharmacodynamic analysis after administration.

As used here, the term "very long acting NPRB agonist" refers to a long NPRB agonist that, when administered as a single bolus dose to a mammalian subject or patient, will have sustained presence in the plasma or sustained plasma cyclic-GMP elevation over the baseline of 24 hours or greater. A very long acting NPRB agonist is a subset of a long acting NPRB agonist. The very long acting nature of the NPRB agonist can result from the NPRB agonist structure itself, or from the combination of the NPRB agonist with one or more components of a formulation containing the NPRB agonist. The presence in the plasma means its detectable presence over an analytical baseline level in the absence of administration of a very long acting NPRB agonist. The duration of plasma cyclic-GMP elevation or the presence of detectable NPRB agonist over the baseline can be from 24 to 192 hours, 24 to 48 hours, or 48 to 72 hours, or 72 to 96 hours, or 96-120 hours, or 120 to 144 hours, 144 to 168 hours, or 168 to 192 hours. A formulation of a very long acting NPRB agonist or a very long acting NPRB agonist formulation refers to a composition containing a very long acting NPRB agonist, or a very long acting NPRB agonist with one or more an excipient or carrier such as a polymer, protein, sugar, lipid, or buffer. The very long acting NPRB agonist may or may not be covalently linked to excipient or carrier. The sustained presence in the blood can be evaluated by pharmacokinetic/ pharmacodynamic analysis after administration. The sustained presence in the blood can be evaluated by pharmacokinetic/pharmacodynamic analysis after administration. The sustained plasma elevation of cyclic-GMP above the baseline can be evaluated by pharmacodynamic analysis after administration.

As used herein, the phrase "NPRB agonist with limited or no agonistic activity against NPRA" refers to an NPRB agonist that has greater than 5-fold binding affinity (or lower EC50) for NPRB than NPRA.

As used herein, the term "polymer" refers to a macromolecule formed chiefly or entirely of many similar repeating units covalently bonded together. The term polymer includes cellulose derivatives, poly(ethylene glycol) (PEG), methoxy poly(ethylene glycol) (MPEG), poly(lactic-co-glycolic acid), and poly(N-vinyl pyrrolidone) and derivatives thereof. These polymers can be branched or linear. As used herein, a polymer can be attached to peptides, protein or a linker group by amide, ester, ether, thioether, thioester, or carbamate bond or by linkers containing one of those bonds. Polymer can also be grafted with each other for make a protected graft co-polymer excipient that, when mixed with an active pharmaceutical ingredient, can enhance pharmacokinetic and pharmacodynamics performance of active pharmaceutical ingredient by extending its presence in the blood or plasma after administration *in vivo.*

The term "amino acids" as used herein are organic compounds with molecular weight of less than 500Da that contain amino (-NH₂) and carboxyl (-COOH) functional groups, along with a side chain (R group) specific to each amino acid. The key elements of an amino acid are carbon (C), hydrogen (H), oxygen (O), and nitrogen (N), although other elements are found in the side chains of certain amino acids. About 500 naturally occurring amino acids are known as of 1983 (though only 20 appear in the mammalian genetic code, these 20 amino acids are also referred to herein as "natural amino acids)). Amino acids can be alpha amino acids, where the amino group is bonded directly to the alpha carbon. Amino acids can be non-alpha amino acid, where the primary amino group is linked to a carbon other than the alpha position. The alpha carbon is the carbon directly adjacent to the carboxyl group.

The term "derivative" or "analog" as used herein includes compounds whose core structures are the same as, or closely resemble that of, a parent compound, but which have a chemical or physical modification, such as different or additional groups; the term includes co-polymers of parent compounds that can be linked to other atoms or molecules. The term also includes a peptide or protein with at least 72% (e.g., at least 80%, at least 85%, at least 90%, at least 95%, or at least 98%) sequence identity with the parent peptide or protein. The term also includes a peptide with additional groups attached to it, such as additional label or tag, compared to the parent peptide. The term also includes a polymer with additional group attached to it, such as alkoxy or methoxy group, compared to the parent polymer.

As used herein, an "addition derivative" or "expansion derivative" refers to a peptide derivative where the main backbone amino acid sequence for a peptide remains the same, but the addition of extra functional groups and/or amino acid residue to the main amino acid sequence using one or more reactive moieties in the main amino acid sequence provides the addition derivative or the expansion derivative. The addition derivative or expansion derivative is different from a truncation and/or substitution peptide derivative where one or more amino acid residues in the main backbone amino acid sequence of the peptide have been removed and/or replaced by different functional groups and/or amino acids, respectively.

As used herein, the term "linker group" or "linking group" or "linker" refers to atoms or chemical moieties that covalently link or bond two entities (e.g., portions of two molecules) together. For example, a linker precursor such as an amino acid, a peptide, or non-amino acid molecule derived from commercially available crosslinkers can be reacted with two entities, linking the two entities together via the linker group. Once the two moieties are linked together, the linker group is the portion that remains from the linker precursor in the final linked entities. For example, if molecule A is to be linked to molecule B, a linker group can have two chemical functional groups where one functional group will react with A and the other functional group will react with B resulting in "A-linker group-B". In this case, the linker group is the portion of the linker precursor that remains after the covalent linking of A and B.

As used herein, the term "polypeptide" refers to a polymer of amino acids.

As used herein, the term "peptide" refers to a polypeptide with three or more amino acids covalently linked together by amide bonds through alpha amino and alpha carboxyl. The number of amino acid residues in a peptide can be 3 to about 100 units.

As used herein, the term "protein" refers to a polypeptide large enough to have a 3-dimensional structure, such as a β-barrel, or an α-helix.

As used herein, the term "antibody" refers to a protein produced by the immune cells that recognize a specific antigen. It is a protein produced in response to and counteracting a specific antigen in the blood. Antibodies combine chemically with substances which the body recognizes as alien, such as bacteria, viruses, and foreign substances in the blood

As used herein, the term "humanized antibody" refers an antibody from non-human species whose protein sequences have been modified to increase their similarity to antibody variants produced naturally in humans.

As used herein, the term "subcutaneous administration," "s.c.", "s.c. administration," "SC," or "SC administration" refers to a delivery of drug, usually in liquid form, directly into the fatty tissues just beneath the skin. The delivery is usually carried out by direct injection. These injections are shallower than those injected into muscle tissues. Providers often use subcutaneous injections for medications that are suitable for absorption into the bloodstream slowly and steadily,

As used herein, the term "intravenous administration," "IV administration," or "IV injection" refers to a delivery of drug, typically in liquid form, directly into a vein of an animal or human. The delivery methods are usually by direct injection. The intravenous route of administration can be used both for injections, using a syringe at higher pressures; as well as for infusions, for example, using the pressure supplied by gravity.

As used herein, the term "intramuscular administration," "IM administration," or "IM injection" refers to an intramuscular delivery of drug, usually in liquid form, directly into the muscles of an animal or human. The delivery is usually by direct injection. This allows the medication to be absorbed into the bloodstream quickly. In some instances, a person may also self-administer an IM injection. In some embodiments, IM injections can be used instead of intravenous injections, for example, when certain therapeutic agents are irritating to veins, or when a suitable vein cannot be located.

As used herein, the term "nasal administration" refers to a delivery of a therapeutic agent (e.g., in form of gel, liquid, aerosol, gas, or powder) by topical application, dropping as a liquid, insufflation (or blown or sprayed), into the nose of an animal or a human. This form of administration can be used, depending on the formulation, for example, to deliver a therapeutic agent to the nasal cavity or the lungs (depending on the device used), and/or may not be absorbed systemically (purely local administration), and/or may be totally absorbed systemically (purely systemic), and/or more frequently partially absorbed (both local and systemic). Nasal sprays can include locally acting drugs such as decongestants for cold and allergy treatment, whose systemic effects are typically minimal. Examples of systemically active drugs available as nasal sprays include, for example, migraine drugs, nicotine replacement, and hormone treatments.

As used herein, the term "parenteral" or "non-gastrointestinal" administration refers to a route of administration that is not through enteral or gastrointestinal routes. Examples of parenteral administration include subcutaneous (under the skin), intravenous (into a vein), intra-arterial (into an artery), intramuscular (into a muscle), intraperitoneal (infusion or injection into the peritoneum), inhalation *(e.g.,* by intratracheal inhalation administration, where a subject is exposed to high aerosol concentrations of the active pharmaceutical ingredient such that the active pharmaceutical ingredient is deposited directly in the lower respiratory tract), nasal administration (through the nose), sublingual and buccal medication, intrathecal (into the spinal canal), intracerebral (into the cerebrum), intracerebroventricular (into the cerebral ventricles), intradermal (into the skin itself), or any other administration routes not involving the gastrointestinal tract. As used herein, the term "enteral" means administration to any region of the alimentary tract and including mouth (oral), pharynx (throat), esophagus, stomach, small intestine, large intestine, rectum, and anus or through an artificial opening in any of these regions.

As used herein, the term "therapeutic agent," "drug," or "active pharmaceutical ingredient" refers to a substance or a molecule capable of producing a curative effect in a disease state.

As used herein, the term "excipient" refers to a substance that is formulated together with or mixed with an active pharmaceutical ingredient for the purpose of long-term stabilization, to bulk up formulations that contain potent active ingredients in small amounts (thus often referred to as "bulking agents", "fillers", or "diluents"), and/or to confer a therapeutic enhancement on the active pharmaceutical ingredient in the final dosage form, such as to facilitate drug absorption and/or potency/dose, reduce viscosity, enhance solubility, and/or prolong the action or presence of the active pharmaceutical ingredient in the blood. The selection of appropriate excipients depends upon the route of administration and the dosage form, the active pharmaceutical ingredient, and other factors. The excipient can include, for example, sugar, amino acid, buffer, antioxidant, chelating agent, solvent or vehicle, and/or a complex polymer that binds and stabilizes an active pharmaceutical ingredient *in vitro* and/or *in vivo.* Though excipients were at one time assumed to be "inactive" ingredients, it is now understood that they can sometimes be "a key determinant of dosage form performance." In other words, the effects of an excipient on pharmacodynamics and pharmacokinetics can be important and can require extensive research and study. How an excipient influences delivery of an active pharmaceutical ingredient is often unpredictable.

As used herein, the term "treatment" refers to a procedure performed after diagnosis of the condition.

As used herein, the term "mitigation" refers a procedure that is performed to prevent, or decrease the likelihood, of an anticipated injury or disease.

As used herein, the term "healthy subject" refers to individual (human and/or mammalian animals) who are participant in a research study with no significant health related issues. For the purpose of the present disclosure these are individuals without lung, liver and/or kidney disease who are of the same age range as the individual having lung, liver and/or kidney disease as evaluated by those skilled in the art (physician and/or clinician). As an example, healthy human adult subject with healthy lung will have average lung capacity of about 4.8-7.2L as measured by spirometry, arterial blood hemoglobin saturation of 95-100% and/or blood oxygen level of 80-100mmHg, and arterial blood carbon dioxide of about 35-45 mm Hg. Healthy human subject with healthy liver will have total plasma protein of about 60 to 83 g/L, albumin of about 34 to 54 g/L, total bilirubin of about 0-12mg/L for adults (0-10mg/L for those under 18), direct bilirubin (conjugated) of about 0-3mg/L, serum alkaline phosphatase (ALP) of adults of about 44-147 international units per liter (IU/L) or 0.73-2.45 microkatal per liter (µkat/L) but about twice in children under 18 years of age, aspartate aminotransferase (AST) of about 5-40U/L, and alanine aminotransferase (ALT) of about 7-56U/L of serum. Healthy human subjects with healthy kidney will have kidney panel results that do not deviate from the following parameters: glomerular filtration rate greater than 60 mL/min/1.73 sqm, blood creatinine of about 5.0 to 15 mg/L and varies by about 20% depending on the assay used, blood urea nitrogen (BUN) 70 to 240 mg/L, BUN to creatinine of about 6 to 25, serum sodium of about 135-145 mM, serum potassium of about 3.6-5.2 mM. chloride of about 98-112 mM, bicarbonate of about 17-29mM anion gap of 7-15, and phosphorous 43-45mg/L. In addition, a generally normal healthy human subject will have resting pulse rate ranges between 50 and 90 beats/min for-human while wider ranges seem acceptable provided there are no indications of thyroid dysfunction or other known significant health problems.

As used herein, the term "elevated total protein in the lungs" or "elevated total protein in BALF" refers to an increase concentration of protein in bronchoalveolar lavage fluid (BALF) by at least 1.5-fold compared to BALF from a normal healthy control subject measured in the same manner. The level can be up to 4-fold *(e.g.,* up to 2-fold, up to 3-fold, or up to 4-fold) compared to the normal healthy control subjects.

As used herein, the term "elevated level of inflammatory cytokines in BALF" refers to an increase concentration of inflammatory cytokines (e.g., IL-6, TNFα (TNF-a), MCP-1, IL-1b) in BALF by at least 4-fold compared to BALF from a normal healthy control subject measured in the same manner. The level can be up to 10-fold to 100-fold *(e.g.,* up to 20-fold, up to 30-fold, up to 40-fold, up to 50-fold, up to 60-fold, up to 70-fold, up to 80-fold, up to 90-fold, or up to 100-fold) compared to BALF of the normal healthy control subjects.

As used herein, a "liquid" is a substance which flows freely at room temperature, such that its shape changes but its volume retains constant, *e.g.*, as would water or an oil.

As used herein, "room temperature" denotes a typical ambient indoor temperature of about 25°C.

Unless defined otherwise, any feature within any aspect or embodiment of the disclosure may be combined with any feature within any other aspect or embodiment of the invention, and such combination are encompassed in the present disclosure. This also applies, but not exclusively, to endpoints of ranges disclosed herein. For instance, if a given substance is disclosed as existing in a composition in a concentration range of X-Y% or A-B%, the present disclosure is to be understood as explicitly disclosing not only the ranges X-Y% and A-B%, but also the ranges X-B%, A-Y% and, in as far as numerically possible, Y-A% and B-X%. Each of these ranges, and range combinations, are contemplated, and are to be understood as being directly and unambiguously disclosed in the present application.

Unless stated otherwise, the designation of a range in the present application using a hyphen ("-") separating two bracketing values X and Y, or two bracketing ratios, is to be understood as meaning and disclosing the specified range in which both endpoint values X and Y are included. The same applies to a range expressed as "from X to Y". Accordingly, the expressions of ranges as "X-Y", "of X to Y", "from X to Y", "of X-Y" and "from X-Y" are to be understood equivalently as meaning and disclosing a range encompassing the end value X, all values (including decimals) between X and Y, as well as the end value Y.

As used herein the term "about" when referring to a particular value, *e.g.*, an endpoint or endpoints of a range, encompasses and discloses, in addition to the specifically recited value itself, a certain variation around that specifically recited value. Such a variation may for example arise from normal measurement variability, *e.g.*, in the weighing or apportioning of various substances by methods known to the skilled person. The term "about" shall be understood as encompassing and disclosing a range of variability above and below an indicated specific value, said percentage values being relative to the specific recited value itself, as follows: The term "about" may encompass and disclose variability of ± 5.0%. The term "about" may encompass and disclose variability of ± 4.5%. The term "about" may encompass and disclose variability of ± 4.0%. The term "about" may encompass and disclose variability of ± 3.5%. The term "about" may encompass and disclose variability of ± 3.0%. The term "about" may encompass and disclose variability of ± 2.5%. The term "about" may encompass and disclose variability of ± 2.0%. The term "about" may encompass and disclose variability of ± 1.5%. The term "about" may encompass and disclose variability of ± 1.0%. The term "about" may encompass and disclose variability of ± 0.5%. The term "about", in reference to the particular recited value, may encompass and disclose that exact particular value itself, irrespective of any explicit mention that this exact particular value is included; even in the absence of an explicit indication that the term "about" includes the particular exact recited value, this exact particular value is still included in the range of variation created by the term "about", and is therefore disclosed in the present application. Unless stated otherwise, where the term "about" is recited before the first endpoint of a numerical range, but not before the second endpoint of that range, this term, and the variability it implies in scope and disclosure, refers to both the first endpoint of the range and the second endpoint of the range. For instance, a recited range of "about X to Y" should be read as "about X to about Y". The same applies for a recited range of ratios. For instance, a recited range of weight ratios of "about X:Y - A:B" should be read as a weight ratio of "(about X):(about Y) - (about A):(about B)".

Unless otherwise defined, all technical and scientific terms used herein have the same meaning as commonly understood by one of ordinary skill in the art. Although methods and materials similar or equivalent to those described herein can be used in the practice or testing of the present disclosure, suitable methods and materials are described below. All publications, patent applications, patents, and other references mentioned herein are incorporated by reference in their entirety. In case of conflict, the present specification, including definitions, will control. In addition, the materials, methods, and examples are illustrative only and not intended to be limiting.

It will be readily understood that the aspects of the present disclosure, as generally described herein, and illustrated in the figures, can be arranged, substituted, combined, separated, and designed in a wide variety of different configurations, all of which are explicitly contemplated herein.

Furthermore, the particular arrangements shown in the FIGURES should not be viewed as limiting. It should be understood that other embodiments may include more or less of each element shown in a given FIGURE. Further, some of the illustrated elements may be combined or omitted. Yet further, an example embodiment may include elements that are not illustrated in the FIGURES.

### Treatment methods

The present disclosure features a treatment methods, including method of treating a subject (e.g., a mammalian subject, a patient in need thereof) having a lung, liver, and/or kidney injury, or a condition or a symptom associated with a lung, liver, and/or kidney injury. The lung, liver, and/or kidney injury, or the condition or symptom associated with the lung, liver, and/or kidney injury can include, for example, acute lung injury (ALI), acute respiratory distress syndrome (ARDS), pulmonary edema, elevated level of inflammatory cells in the lung, increased level or expression of inflammatory cytokines in the lung (compared to healthy lung), increased protein level in lung alveolar space (compared to healthy lung), low arterial blood oxygenation (wherein low arterial blood oxygenation is a blood PaO2 of below 60 mm Hg and/or a blood hemoglobin oxygen saturation (SpO2) of below 90%), sepsis, bacteremia, pneumonia, fibrosis *(e.g.,* lung, liver, or kidney fibrosis), and/or kidney injury. In some embodiments, the lung, liver, and/or kidney injury, or the symptom associated with the lung, liver, and/or kidney injury can include, for example, acute lung injury (ALI), acute respiratory distress syndrome (ARDS), pulmonary edema, elevated level of inflammatory cells in the lung, increased level or expression of inflammatory cytokines in the lung (compared to healthy lung), increased protein level in lung alveolar space (compared to healthy lung), low arterial blood oxygenation (wherein low arterial blood oxygenation is a blood PaO2 of below 60 mm Hg and/or a blood hemoglobin oxygen saturation (SpO2) of below 90%), pneumonia, fibrosis, and/or kidney injury. The subject can have low arterial blood oxygenation, defined as a blood PaO2 below 60 mm Hg and/or a blood hemoglobin oxygen saturation (SpO2) below 90%.

The present disclosure also features a method of treatment of fibrosis in general, including, for example, lung fibrosis, liver fibrosis, cirrhosis, and kidney glomerular sclerosis, and treatment of or providing protection from kidney injury. The methods include administering to the subject a therapeutically effective bolus dose of a composition including a long acting CNP, a very long acting CNP, a long acting CNP derivative, a very long acting CNP derivative, a long acting NPRB agonist, and/or a very long acting NPRB agonist. The therapeutically effective bolus dose is a dose that does not decrease or cause a decrease in blood pressure (or mean arterial pressure) by more than 20% *(e.g.,* by more than 15%, by more than 10%, or by more than 5%) of a baseline blood pressure measurement, where the baseline blood pressure measurement is an average blood pressure prior to administration of the composition, but the dose can increase plasma cyclic-GMP level at from 1 hour to 12 hours (e.g., 2 to 12 hours, 4 to 12 hours, 1 hour to-24 hours, 2 to 24 hours, 4 to 24 hours, 1 hour to 84 hours, 2 to 84 hours, 4 to 84 hours, 12 to 84 hours, 1 hour to 168 hours, 2 to 168 hours, 4 to 168 hours, or 12 to 168 hours) after administration to above 1.5x (e.g., above 2x, above 3x, above 4x, or above 5x) of a baseline plasma cyclic-GMP level, where the baseline plasma cyclic-GMP level is defined as the plasma level prior to administration of the bolus dose, the plasma level of the healthy subject which is 4 +/- 1 pmol/ml, or about 1.4 mg/ml in humans (but which can vary between species). *See, e.g.,* Shotan et al., Plasma cyclic guanosine monophosphate in chronic heart failure: hemodynamic and neurohormonal correlations and response to nitrate therapy. Clin Pharmacol Ther, 1993. 54(6): p. 638-44, incorporated herein in its entirety. In a preferred embodiment, the baseline level is the measured level prior to drug administration for the same subject to which treatment is provided, and that level can vary from one subject to the next. In practicing the present disclosure, any baseline parameter that used as a reference parameter to evaluate the effect of the treatment is established by measurement prior to treatment. Typically, but not exclusively, the baseline plasma cyclic-GMP level varies depending on the time of the day with lower level during day-time wakefulness, higher soon after bedtime, and can vary from 2-8 pmol/ml throughout the day in human. Thus, the measured baseline plasma cyclic GMP level prior to administration of the composition and the measured plasma cyclic GMP level after administration of the compositions of the present disclosure can occur at the same predetermined time every day. Where an average baseline is described, the average baseline can be the average baseline measurement taken at least 3 times at an interval of at least 4 hours for a given parameter within 24 hour period for a given subject. This controls for inter-subject or inter-individual variability. In patients with congestive heart failure the baseline plasma cyclic-GMP level may be 2 to 3-fold higher and the baseline is established prior to treatment for each individual subject or group of subjects. Similarly for blood pressure, the baseline will be measured level prior to drug administration and is used as reference to evaluate the effect of the treatment. The baseline cGMP level in healthy mouse with no known symptoms of any health condition is 20 (3.7) pmol/mL [mean (SEM); n=8] or 7 (1.3) ng/mL [mean (SEM); n=8]. The baseline cGMP level in dogs with no known symptoms of any health condition is 5-12 ng/ml.

In some embodiments, administering to the subject a therapeutically effective bolus dose of the composition further decreases a total number of cells and total proteins in a BALF sample from the subject. In certain embodiments, administering to the subject a therapeutically effective bolus dose of the composition further decreases MPO (an activated neutrophil marker) in a lung tissue from the subject, compared the to the MPO prior to administration of the composition. In certain embodiments, administering to the subject a therapeutically effective bolus dose of the composition further attenuates inflammatory cytokine expression (e.g., IL-6, IL-1b, TNFα, MCP-1, and IFNg; which can be present, for example, in ARDS) in the subject, compared to the inflammatory cytokine expression prior to administration of the composition. In certain embodiments, administering to the subject a therapeutically effective bolus dose of the composition decreases a fibrotic area *(e.g.,* a fibrotic area in lung fibrosis, liver fibrosis, cirrhosis, and/or kidney glomerular sclerosis) compared to the fibrotic area prior to administration of the composition, or provides treatment of/protection from kidney injury. In certain embodiments, administering to the subject a therapeutically effective bolus dose of the composition further decreases a fibrotic area in a lung in a subject having idiopathic pulmonary fibrosis, compared to prior to administration of the composition. In some embodiments, administering to the subject a therapeutically effective bolus dose of the composition further decreases cell numbers and protein levels, and decreases the expression of any one of IL-6, IL-1b, TNFα, MCP-1, and IFNg or any combinati on thereof in a subject having idiopathic pulmonary fibrosis, compared to prior to administration of composition. In some embodiments, administering to the subject the therapeutically effective bolus dose of the composition decreases the expression of any one of AST, ALT, α-SMA, IL-6, IL-1b, TNFα, MCP-1, IFNg, iNOS, Elf-1, Tollip, IRAK-1, P-P38, P-P65, β-act, STAT1, P-STAT1, STAT2, STAT3, STAT6, a fibrotic area, serum creatinine, an albumin/creatinine ratio in urine, hydroxyproline in a lung, or any combination thereof, of the subject.

In some embodiments, the therapeutically effective bolus dose does not decrease or cause a decrease in blood pressure (or mean arterial pressure) by more than 20% *(e.g.,* by more than 15%, by more than 10%, or by more than 5%) of a baseline blood pressure measurement, where the baseline blood pressure measurement is an average blood pressure prior to administration of the composition, but increases plasma cyclic-GMP level at from 1 hour to 12 hours (e.g., 2 to 12 hours, 4 to 12 hours, 1 hour to-24 hours, 2 to 24 hours, 4 to 24 hours, 1 hour to 84 hours, 2 to 84 hours, 4 to 84 hours, 12 to 84 hours, 1 hour to 168 hours, 2 to 168 hours, 4 to 168 hours, or 12 to 168 hours) after administration to above 1.5x (e.g., above 2x, above 3x, above 4x, or above 5x) of a baseline plasma cyclic-GMP level, where the baseline plasma cyclic-GMP level is defined as the average plasma level prior to administration of the bolus dose or the average plasma level of the healthy subject.

In some embodiments, the therapeutically effective bolus dose does not decrease or cause a decrease in blood pressure (or mean arterial pressure) by more than 15% of a baseline blood pressure measurement, where the baseline blood pressure measurement is an average blood pressure prior to administration of the composition, but increases plasma cyclic-GMP level at from 1 hour to 12 hours (e.g., 2 to 12 hours, 4 to 12 hours, 1 hour to-24 hours, 2 to 24 hours, 4 to 24 hours, 1 hour to 84 hours, 2 to 84 hours, 4 to 84 hours, 12 to 84 hours, 1 hour to 168 hours, 2 to 168 hours, 4 to 168 hours, or 12 to 168 hours) after administration to above 1.5x (e.g., above 2x, above 3x, above 4x, or above 5x) of a baseline plasma cyclic-GMP level, where the baseline plasma cyclic-GMP level is defined as the average plasma level prior to administration of the bolus dose or the average plasma level of the healthy subject.

In some embodiments, the therapeutically effective bolus dose does not decrease blood pressure by more than 10% of a baseline blood pressure measurement, where the baseline blood pressure measurement is an average blood pressure prior to administration of the composition, but the dose increases plasma cyclic-GMP level at from 1 hour to 12 hours (e.g., 2 to 12 hours, 4 to 12 hours, 1 hour to-24 hours, 2 to 24 hours, 4 to 24 hours, 1 hour to 84 hours, 2 to 84 hours, 4 to 84 hours, 12 to 84 hours, 1 hour to 168 hours, 2 to 168 hours, 4 to 168 hours, or 12 to 168 hours) after administration to above 1.5x (e.g., above 2x, above 3x, above 4x, or above 5x) of a baseline plasma cyclic-GMP level, where the baseline plasma cyclic-GMP level is defined as the average plasma level prior to administration of the bolus dose or the average plasma level of the healthy subject.

In some embodiments, the therapeutically effective bolus dose does not decrease blood pressure by more than 5% but the dose increases plasma cyclic-GMP level at from 1 hour to 12 hours (e.g., 2 to 12 hours, 4 to 12 hours, 1 hour to-24 hours, 2 to 24 hours, 4 to 24 hours, 1 hour to 84 hours, 2 to 84 hours, 4 to 84 hours, 12 to 84 hours, 1 hour to 168 hours, 2 to 168 hours, 4 to 168 hours, or 12 to 168 hours) after administration to above 1.5x *(e.g.,* above 2x, above 3x, above 4x, or above 5x) of a baseline plasma cyclic-GMP level, where the baseline plasma cyclic-GMP level is defined as the average plasma level prior to administration of the bolus dose or the average plasma level of the healthy subject.

The methods of the present disclosure are made possible by the surprising discovery that CNP can be modified, derivatized, and/or formulated in such a way that it can induce/cause an increase and/or maximize cyclic-GMP production without the associated detrimental drop in blood pressure. In particular, the blood pressure effect of CNP can be minimized or eliminated at a therapeutic bolus dose that increases plasma cyclic-GMP by 1.5-fold or greater above the baseline in a sustained manner for greater than 4 hours or 6 hours, depending on the administered peptide.

In some embodiments, the present disclosure features methods of treating a subject *(e.g.,* a mammalian subject, a patient in need thereof) having a lung, liver, and/or kidney injury, or a condition or symptom associated with a lung, liver, and/or kidney injury, such as: acute lung injury (ALI), acute respiratory distress syndrome (ARDS), pulmonary edema, elevated level of inflammatory cells in the lung, increased level or expression of inflammatory cytokines in the lung (compared to healthy lung), increased protein level in lung alveolar space (compared to healthy lung), low arterial blood oxygenation (wherein low arterial blood oxygenation is a blood PaO2 of below 60 mm Hg and/or a blood hemoglobin oxygen saturation (SpO2) of below 90%), sepsis, bacteremia, pneumonia, fibrosis in general (e.g., lung/pulmonary fibrosis, liver fibrosis, cirrhosis, and/or kidney glomerular sclerosis), and/or kidney injury. In some embodiments, the lung, liver, and/or kidney injury, or the symptom associated with a lung, liver, and/or kidney injury include: acute lung injury (ALI), acute respiratory distress syndrome (ARDS), pulmonary edema, elevated level of inflammatory cells in the lung, increased level or expression of inflammatory cytokines in the lung (compared to healthy lung), increased protein level in lung alveolar space (compared to healthy lung), low arterial blood oxygenation (wherein low arterial blood oxygenation is a blood PaO2 of below 60 mm Hg and/or a blood hemoglobin oxygen saturation (SpO2) of below 90%), pneumonia, fibrosis in general *(e.g.,* lung/pulmonary fibrosis, liver fibrosis, cirrhosis, and/or kidney glomerular sclerosis), and/or kidney injury. In some embodiments, the subject can have a low arterial blood oxygenation, defined as a blood PaO2 below 60 mm Hg and/or a blood hemoglobin oxygen saturation (SpO2) below 90%. The methods are described below.

In some embodiments, the methods include administering to the subject a therapeutically effective bolus dose of a composition including a long acting CNP; wherein therapeutically effective bolus dose does not decrease or a drop in blood pressure (or mean arterial pressure) by more than 20% *(e.g.,* by more than 15%, by more than 10%, or by more than 5%) of a baseline blood pressure measurement, where the baseline blood pressure measurement is an average blood pressure prior to administration of the composition, but enough to increase plasma cyclic-GMP level at from 1 hour to 12 hours *(e.g.,* 2 to 12 hours, 4 to 12 hours, 1 hour to-24 hours, 2 to 24 hours, 4 to 24 hours, 1 hour to 84 hours, 2 to 84 hours, 4 to 84 hours, 12 to 84 hours, 1 hour to 168 hours, 2 to 168 hours, 4 to 168 hours, or 12 to 168 hours) after administration to above 1.5x *(e.g.,* above 2x, above 3x, above 4x, or above 5x) of a baseline plasma cyclic-GMP level, wherein baseline is the average plasma level prior to administration of the composition or the average plasma level of a healthy subject (preferably the average plasma cyclic-GMP level prior to administration of the composition for the subject). In some embodiments, the therapeutically effective bolus dose does not decrease or cause a decrease in blood pressure (or mean arterial pressure) by more than 20% *(e.g.,* by more than 15%, or by more than 10%, or by more than 5%) of a baseline blood pressure measurement, where the baseline blood pressure measurement is an average blood pressure prior to administration of the composition, but can increase plasma cyclic-GMP level at from 1 hour to 12 hours (e.g., 2 to 12 hours, 4 to 12 hours, 1 hour to-24 hours, 2 to 24 hours, 4 to 24 hours, 1 hour to 84 hours, 2 to 84 hours, 4 to 84 hours, 12 to 84 hours, 1 hour to 168 hours, 2 to 168 hours, 4 to 168 hours, or 12 to 168 hours) after administration to above 1.5x *(e.g.,* above 2x, above 3x, above 4x, or above 5x) of a baseline plasma cyclic-GMP level, where the baseline plasma cyclic-GMP level is defined as the average plasma level prior to administration of the bolus dose or the average plasma level of the healthy subject (preferably the average plasma cyclic-GMP level prior to administration of the composition for the subject).

In some embodiments, the methods include administering to the subject a therapeutically effective bolus dose of a composition including a very long acting CNP; wherein therapeutically effective bolus dose does not decrease or a drop in blood pressure (or mean arterial pressure) by more than 20% *(e.g.,* by more than 15%, by more than 10%, or by more than 5%) of a baseline blood pressure measurement, where the baseline blood pressure measurement is an average blood pressure prior to administration of the composition, but enough to increase plasma cyclic-GMP level at from 1 hour to 12 hours *(e.g.,* 2 to 12 hours, 4 to 12 hours, 1 hour to-24 hours, 2 to 24 hours, 4 to 24 hours, 1 hour to 84 hours, 2 to 84 hours, 4 to 84 hours, 12 to 84 hours, 1 hour to 168 hours, 2 to 168 hours, 4 to 168 hours, or 12 to 168 hours) after administration to above 1.5x *(e.g.,* above 2x, above 3x, above 4x, or above 5x) of a baseline plasma cyclic-GMP level, wherein cyclic-GMP baseline is the average plasma level prior to administration of the composition or the average plasma level of healthy mammalian subject. In some embodiments, the therapeutically effective bolus dose does not decrease or cause a decrease in blood pressure (or mean arterial pressure) by more than 20% *(e.g.,* by more than 15%, by more than 10%, or by more than 5%) of a baseline blood pressure measurement, where the baseline blood pressure measurement is an average blood pressure prior to administration of the composition, but can increase plasma cyclic-GMP level at from 1 hour to 12 hours (e.g., 2 to 12 hours, 4 to 12 hours, 1 hour to-24 hours, 2 to 24 hours, 4 to 24 hours, 1 hour to 84 hours, 2 to 84 hours, 4 to 84 hours, 12 to 84 hours, 1 hour to 168 hours, 2 to 168 hours, 4 to 168 hours, or 12 to 168 hours) after administration to above 1.5x (e.g., above 2x, above 3x, above 4x, or above 5x) of a baseline plasma cyclic-GMP level, where the baseline plasma cyclic-GMP level is defined as the average plasma level prior to administration of the bolus dose or the average plasma level of the healthy subject (preferably the average plasma cyclic-GMP level prior to administration of the composition for the subject).

In some embodiments, the methods include administering to the subject a therapeutically effective bolus dose of a composition including a long acting CNP derivative; wherein therapeutically effective bolus dose does not decrease or cause a drop in blood pressure (or mean arterial pressure) by more than 20% (e.g., by more than 15%, by more than 10%, or by more than 5%) of a baseline blood pressure measurement, where the baseline blood pressure measurement is an average blood pressure prior to administration of the composition, but enough to increase plasma cyclic-GMP level at from 1 hour to 12 hours (e.g., 2 to 12 hours, 4 to 12 hours, 1 hour to-24 hours, 2 to 24 hours, 4 to 24 hours, 1 hour to 84 hours, 2 to 84 hours, 4 to 84 hours, 12 to 84 hours, 1 hour to 168 hours, 2 to 168 hours, 4 to 168 hours, or 12 to 168 hours) after administration to above 1.5x (e.g., above 2x, above 3x, above 4x, or above 5x) of a baseline plasma cyclic-GMP level, wherein baseline is the average plasma cyclic-GMP level prior to administration of the composition or the average plasma level of healthy mammalian subject. The therapeutically effective bolus dose is a dose that does not decrease or cause a decrease in blood pressure (or mean arterial pressure) by more than 20% *(e.g.,* by more than 15%, by more than 10%, or by more than 5%) of a baseline blood pressure measurement, where the baseline blood pressure measurement is an average blood pressure prior to administration of the composition; but the dose can increase plasma cyclic-GMP level at from 1 hour to 12 hours (e.g., 2 to 12 hours, 4 to 12 hours, 1 hour to-24 hours, 2 to 24 hours, 4 to 24 hours, 1 hour to 84 hours, 2 to 84 hours, 4 to 84 hours, 12 to 84 hours, 1 hour to 168 hours, 2 to 168 hours, 4 to 168 hours, or 12 to 168 hours) after administration to above 1.5x *(e.g.,* above 2x, above 3x, above 4x, or above 5x) of a baseline plasma cyclic-GMP level, where the baseline plasma cyclic-GMP level is defined as the average plasma level prior to administration of the bolus dose or the average plasma level of the healthy subject (preferably the average plasma cyclic-GMP level prior to administration of the composition for the subject).

In some embodiments, the methods include administering to the subject a therapeutically effective bolus dose of a composition including a very long acting CNP derivative; wherein therapeutically effective bolus dose does not decrease or cause a drop in blood pressure (or mean arterial pressure) by more than 20% (e.g., by more than 15%, by more than 10%, or by more than 5%) of a baseline blood pressure measurement, where the baseline blood pressure measurement is an average blood pressure prior to administration of the composition, but enough to increase plasma cyclic-GMP level at from 1 hour to 12 hours *(e.g.,* 2 to 12 hours, 4 to 12 hours, 1 hour to-24 hours, 2 to 24 hours, 4 to 24 hours, 1 hour to 84 hours, 2 to 84 hours, 4 to 84 hours, 12 to 84 hours, 1 hour to 168 hours, 2 to 168 hours, 4 to 168 hours, or 12 to 168 hours) after administration to above 1.5x (e.g., above 2x, above 3x, above 4x, or above 5x) of a baseline plasma cyclic-GMP level, wherein baseline is the average plasma cyclic-GMP level prior to administration of the composition or the average plasma level of healthy mammalian subject. The therapeutically effective bolus dose is a dose that does not decrease or cause a decrease in blood pressure (or mean arterial pressure) by more than 20% *(e.g.,* by more than 15%, by more than 10%, or by more than 5%) a baseline blood pressure measurement, where the baseline blood pressure measurement is an average blood pressure prior to administration of the composition, but the dose can increase plasma cyclic-GMP level at from 1 hour to 12 hours (e.g., 2 to 12 hours, 4 to 12 hours, 1 hour to-24 hours, 2 to 24 hours, 4 to 24 hours, 1 hour to 84 hours, 2 to 84 hours, 4 to 84 hours, 12 to 84 hours, 1 hour to 168 hours, 2 to 168 hours, 4 to 168 hours, or 12 to 168 hours) after administration to above 1.5x *(e.g.,* above 2x, above 3x, above 4x, or above 5x) of a baseline plasma cyclic-GMP level, where the baseline plasma cyclic-GMP level is defined as the average plasma level prior to administration of the bolus dose or the average plasma level of the healthy subject (preferably the average plasma cyclic-GMP level prior to administration of the composition for the subject).

In some embodiments, the methods include administering to the subject a therapeutically effective bolus dose of a composition including a long acting NPRB agonist; wherein therapeutically effective bolus dose does not decrease or a drop in blood pressure (or mean arterial pressure) by more than 20% (e.g., by more than 15%, by more than 10%, or by more than 5%) of a baseline blood pressure measurement, where the baseline blood pressure measurement is an average blood pressure prior to administration of the composition, but enough to increase plasma cyclic-GMP level at from 1 hour to 12 hours (e.g., 2 to 12 hours, 4 to 12 hours, 1 hour to-24 hours, 2 to 24 hours, 4 to 24 hours, 1 hour to 84 hours, 2 to 84 hours, 4 to 84 hours, 12 to 84 hours, 1 hour to 168 hours, 2 to 168 hours, 4 to 168 hours, or 12 to 168 hours) after administration to above 1.5x (e.g., above 2x, above 3x, above 4x, or above 5x) of a baseline plasma cyclic-GMP level, wherein baseline is the average plasma level prior to administration of the composition or the average plasma level of healthy mammalian subject. The therapeutically effective bolus dose is a dose that does not decrease or cause a decrease in blood pressure (or mean arterial pressure) by more than 20% *(e.g.,* by more than 15%, by more than 10%, or by more than 5%) of a baseline blood pressure measurement, where the baseline blood pressure measurement is an average blood pressure prior to administration of the composition; but the dose can increase plasma cyclic-GMP level at from 1 hour to 12 hours (e.g., 2 to 12 hours, 4 to 12 hours, 1 hour to-24 hours, 2 to 24 hours, 4 to 24 hours, 1 hour to 84 hours, 2 to 84 hours, 4 to 84 hours, 12 to 84 hours, 1 hour to 168 hours, 2 to 168 hours, 4 to 168 hours, or 12 to 168 hours) after administration to above 1.5x (e.g., above 2x, above 3x, above 4x, or above 5x) of a baseline plasma cyclic-GMP level, where the baseline plasma cyclic-GMP level is defined as the average plasma level prior to administration of the bolus dose or the average plasma level of the healthy subject (preferably the average plasma cyclic-GMP level prior to administration of the composition for the subject).

In some embodiments, the methods include administering to the subject a therapeutically effective bolus dose of a composition including a very long acting NPRB agonist; wherein therapeutically effective bolus dose does not decrease or a drop in blood pressure (or mean arterial pressure) by more than 20% *(e.g.,* by more than 15%, by more than 10%, or by more than 5%) of a baseline blood pressure measurement, where the baseline blood pressure measurement is an average blood pressure prior to administration of the composition, but enough to increase plasma cyclic-GMP level at from 1 hour to 12 hours (e.g., 2 to 12 hours, 4 to 12 hours, 1 hour to-24 hours, 2 to 24 hours, 4 to 24 hours, 1 hour to 84 hours, 2 to 84 hours, 4 to 84 hours, 12 to 84 hours, 1 hour to 168 hours, 2 to 168 hours, 4 to 168 hours, or 12 to 168 hours) after administration to above 1.5x (e.g., above 2x, above 3x, above 4x, or above 5x) of a baseline plasma cyclic-GMP level, wherein baseline is the average plasma level prior to administration of the composition or the average plasma level of healthy mammalian subject. The therapeutically effective bolus dose is a dose that does not decrease or cause a decrease in blood pressure (or mean arterial pressure) by more than 20% *(e.g.,* by more than 15%, by more than 10%, or by more than 5%) of a baseline blood pressure measurement, where the baseline blood pressure measurement is an average blood pressure prior to administration of the composition; but the dose can increase plasma cyclic-GMP level at from 1 hour to 12 hours *(e.g.,* 2 to 12 hours, 4 to 12 hours, 1 hour to-24 hours, 2 to 24 hours, 4 to 24 hours, 1 hour to 84 hours, 2 to 84 hours, 4 to 84 hours, 12 to 84 hours, 1 hour to 168 hours, 2 to 168 hours, 4 to 168 hours, or 12 to 168 hours) after administration to above 1.5x (e.g., above 2x, above 3x, above 4x, or above 5x) of a baseline plasma cyclic-GMP level, where the baseline plasma cyclic-GMP level is defined as the average plasma level prior to administration of the bolus dose or the average plasma level of the healthy subject (preferably the average plasma cyclic-GMP level prior to administration of the composition for the subject).

The subject can have a low arterial blood oxygenation, defined as a blood PaO2 below 60 mm Hg and/or a blood hemoglobin oxygen saturation (SpO2) below 90%. The method includes administering to the subject a therapeutically effective bolus dose of a composition including very long acting NPRB agonist; wherein therapeutically effective bolus dose does not decrease or a drop in blood pressure (or mean arterial pressure) by more than 20% (e.g., by more than 15%, by more than 10%, or by more than 5%) of a baseline blood pressure measurement, where the baseline blood pressure measurement is an average blood pressure prior to administration of the composition, but enough to increase plasma cyclic-GMP level at from 1 hour to 12 hours *(e.g.,* 2 to 12 hours, 4 to 12 hours, 1 hour to-24 hours, 2 to 24 hours, 4 to 24 hours, 1 hour to 84 hours, 2 to 84 hours, 4 to 84 hours, 12 to 84 hours, 1 hour to 168 hours, 2 to 168 hours, 4 to 168 hours, or 12 to 168 hours) after administration to above 1.5x (e.g., above 2x, above 3x, above 4x, or above 5x) of a baseline plasma cyclic-GMP level, wherein baseline is the average plasma cyclic-GMP level prior to administration of the composition or the average plasma level of healthy mammalian subject. The therapeutically effective bolus dose is a dose that does not decrease or cause a decrease in blood pressure (or mean arterial pressure) by more than *20% (e.g.,* by more than 15%, by more than 10%, or by more than 5%) of a baseline blood pressure measurement, where the baseline blood pressure measurement is an average blood pressure prior to administration of the composition; but the dose can increase plasma cyclic-GMP level at from 1 hour to 12 hours (e.g., 2 to 12 hours, 4 to 12 hours, 1 hour to-24 hours, 2 to 24 hours, 4 to 24 hours, 1 hour to 84 hours, 2 to 84 hours, 4 to 84 hours, 12 to 84 hours, 1 hour to 168 hours, 2 to 168 hours, 4 to 168 hours, or 12 to 168 hours) after administration to above 1.5x *(e.g.,* above 2x, above 3x, above 4x, or above 5x) of a baseline plasma cyclic-GMP level, where the baseline plasma cyclic-GMP level is defined as the average plasma level prior to administration of the bolus dose or the average plasma level of the healthy subject (preferably the average plasma cyclic-GMP level prior to administration of the composition for the subject).

The long acting NPRB agonist or the very long acting NPRB agonist can include a polypeptide, such as an antibody. In some embodiments, the long acting NPRB agonist or the very long acting NPRB agonist includes a molecule having a molecular weight of less than 2kDa.

In some embodiments, in any one of the above methods, the composition has limited or no agonistic activity against NPRA and/or has greater than 5-fold greater binding affinity (or 5-fold lower EC50) for NPRB receptor than NPRA receptor.

In some embodiments, for any one of the above methods, administering to a subject includes an administration method such as oral administration or parenteral administration. Examples of parenteral administration are subcutaneous, intravenous, intramuscular, inhalation, nasal, or any combination thereof. In some embodiments, the methods above can include oral administration and/or subcutaneous administration. In certain embodiments, the methods above include intravenous administration. In some embodiments, the methods above include intramuscular administration. In some embodiments, the methods above include administration by inhalation *(e.g.,* by intratracheal inhalation administration, where a subject is exposed to high aerosol concentrations so that the active pharmaceutical ingredient is deposited directly in the lower respiratory tract). In certain embodiments, the methods above include nasal administration. In some embodiments, the methods above include oral administration.

In some embodiments, for any one of the above methods, administering to a subject consists essentially of, or consists of, administering the compositions of the present disclosure as a bolus dose. In some embodiments, for any one of the above methods, administering to a subject does not include administration of the compositions of the present disclosure by infusion over a sustained period of time *(e.g.,* by continuous infusion). In some embodiments, for any of the above methods, administering to a subject does not include administering the compositions of the present disclosure as a bolus dose followed by an infusion over a sustained period of time. In some embodiments, for any one of the above methods, administering to a subject does not include oral administration of the compositions of the present disclosure.

### Active pharmaceutical ingredients

For any of the above methods described above, the long acting CNP derivative or very long acting CNP derivative can include U-GLSKGCFGLKLDRIGSMSGLGC [SEQ ID NO. 2]; U-GLSKGCFGLK(U)LDRIGSMSGLGC [SEQ ID NO. 3]; GLSKGCFGLK(U)LDRIGSMSGLGC [SEQ ID NO. 4]; and/or U-CFGLKLDRIGSxSGLGC, where x is a natural or unnatural amino acid residue [SEQ ID NO. 11], where U is attached to the N-terminal G, C and/or to the epsilon amino of K residue.

In some embodiments, U in the sequences above is a moiety of Formula (I) or (11), where Formula (I) is

(aliphatic)ₐ-(X)-; (I)

wherein:
a is 0 or 1 (preferably a is 1);
aliphatic is an optionally substituted C₄₋₂₄ chain (e.g., an optionally substituted C₁₀₋₂₄ chain, an optionally substituted C₁₂₋₁₈ chain), covalently bound to X via a chemical linkage, such as a carbonyl *(e.g.,* as part of an amide or an ester linkage), a thioether, an ether, a thioether, a carbamate moiety, a bond, or the like with X; preferably via a carbonyl as part of an amide or an ester linkage; or more preferably via a carbonyl as part of an amide linkage with X;
X is a 1-10 amino acid residue or peptide sequence, wherein each amino acid residue is independently selected from lysine (K), arginine (R), glycine (G), alanine (A), glutamic acid (E), and aspartic acid (D); or
X is a linker (γE)ₘ-(B)ₙ,
   wherein B is a 1-8 amino acid residue or peptide sequence wherein each amino acid residue is independently selected from 2-[2-(2-aminoethoxy)ethoxy]acetic acid residue, Gly, Ala, Leu, Ser, Arg, and Lys;
   m is 0, 1, 2, or 3;
   n is 0, 1, 2, or 3; and
   the sum of m and n is at least 1,
and Formula (II) is

   (polymer)ₐ-(Y)-; (II)
wherein a is 0 or 1 (preferably a is 1);
polymer is cellulose, poly(ethylene glycol) (PEG), methoxy poly(ethylene glycol) (MPEG), poly(lactic-co-glycolic acid), poly(N-vinyl pyrrolidone), or a derivative thereof;
Y is:
   a 1-10 amino acid residue or peptide sequence, wherein each amino acid residue is independently selected from lysine (K), arginine (R), glycine (G), alanine (A), glutamic acid (E), and aspartic acid (D);
   a non-amino acid linker comprising an ester, an amide, a thioether, an ether, a thioether, a carbamate moiety, or a combination thereof;
   an amino acid residue-containing linker, wherein the amino acid residue is covalently attached to (polymer)ₐ; or
   a peptide linker different from the 1-10 amino acid residue or peptide sequence.

In some embodiments, in the above Formula (II), Y is a linker (γE)ₘ-(B)ₙ, wherein B is a 1-8 amino acid residue or peptide sequence wherein each amino acid is independently selected from 2-[2-(2-aminoethoxy)ethoxy]acetic acid, Gly, Ala, Leu, Ser, Arg, and Lys; m is 0, 1, 2, or 3; n is 0, 1, 2, or 3; and the sum of m and n is at least 1.

In the present disclosure, lower case "x" refers to a natural or unnatural amino acid residue in the peptide sequence where it appears. Upper case X refers to a linker in Formula (I) and (II). In some embodiments, x is not a methionine residue (M), is not an asparagine residue (N), or is neither a methionine (M) nor an asparagine residue (N). In some embodiments, x is not any one of the 20 natural amino acid residues encoded by the mammalian genome, such as amino acids A, C, D, E, F, G, H, I, K, L, M, N, P, Q, R, S, T, V, W, and Y. In some embodiments, x is an unnatural amino acid residue *(i.e.,* an amino acid residue not encoded by the mammalian genome). In some embodiments, x is homoglutamine (also referred to herein as homoQ).

In some embodiments, the long acting CNP derivative or very long acting CNP derivative includes U-GLSKGCFGLKLDRIGSMSGLGC [SEQ ID NO. 2]; where U is attached to the N-terminal G of GLSKGCFGLKLDRIGSMSGLGC, and U is (aliphatic)ₐ-(X)-; wherein a is 1; aliphatic is an optionally substituted C₄₋₂₄ chain (e.g., an optionally substituted C₁₀₋₂₄ chain, an optionally substituted C₁₀₋₁₈ chain, or an optionally substituted C₁₂₋₁₈ chain), covalently bound to X via a chemical linkage, such as a carbonyl (e.g., as part of an amide or an ester linkage), a thioether, an ether, a thioether, a carbamate moiety, a bond, or the like with X; preferably via a carbonyl as part of an amide or an ester linkage; or more preferably via a carbonyl as part of an amide linkage with X; X is a 1-10 amino acid residue or peptide sequence, wherein each amino acid residue is independently selected from lysine (K), arginine (R), glycine (G), alanine (A), glutamic acid (E), and aspartic acid (D).

In some embodiments, the long acting CNP derivative or very long acting CNP derivative includes U-CFGLKLDRIGSxSGLGC [SEQ ID NO. 12] where x is a natural or unnatural amino acid residue and U has formula (aliphatic)ₐ-(X)- (Formula I); wherein 0 or 1 (preferably a is 1); aliphatic is an optionally substituted C₄₋₂₄ chain (e.g., an optionally substituted C₁₀₋₂₄ chain, an optionally substituted C₁₀₋₁₈ chain, or an optionally substituted C₁₂₋₁₈ chain) covalently bound to X via a chemical linkage, such as a carbonyl (e.g., as part of an amide or an ester linkage), a thioether, an ether, a thioether, a carbamate moiety, a bond, or the like with X; preferably via a carbonyl as part of an amide or an ester linkage; or more preferably via a carbonyl as part of an amide linkage with X; and X is a linker (γE)ₘ-(B)ₙ, wherein B is a 1-8 amino acid residue or peptide sequence wherein each amino acid residue is independently selected from 2-[2-(2-aminoethoxy)ethoxy]acetic acid residue, Gly, Ala, Leu, Ser, Arg, and Lys; m is 0, 1, 2, or 3; n is 0, 1, 2, or 3; and the sum of m and n is at least 1.

In some embodiments, the long acting CNP derivative or very long acting CNP derivative includes U-CFGLKLDRIGSxSGLGC [SEQ ID NO. 30] where x is a natural or unnatural amino acid residue, and provided that x is not M (methionine); U has formula (aliphatic)ₐ-(X)- (Formula I); wherein 0 or 1 (preferably a is 1); aliphatic is an optionally substituted C₄₋₂₄ chain (e.g., an optionally substituted C₁₀₋₂₄ chain, an optionally substituted C₁₀₋₁₈ chain, or an optionally substituted C₁₂₋₁₈ chain) covalently bound to X via a chemical linkage, such as a carbonyl (*e.g*., as part of an amide or an ester linkage), a thioether, an ether, a thioether, a carbamate moiety, a bond, or the like with X; preferably via a carbonyl as part of an amide or an ester linkage; or more preferably via a carbonyl as part of an amide linkage with X; and X is a linker (γE)ₘ-(B)ₙ, wherein B is a 1-8 amino acid residue or peptide sequence wherein each amino acid residue is independently selected from 2-[2-(2-aminoethoxy)ethoxy]acetic acid residue, Gly, Ala, Leu, Ser, Arg, and Lys; m is 0, 1, 2, or 3; n is 0, 1, 2, or 3; and the sum of m and n is at least 1.

In some embodiments, the long acting CNP derivative or very long acting CNP derivative can include U-GLSKGCFGLKLDRIGSMSGLGC [SEQ ID NO. 2]; U-GLSKGCFGLK(U)LDRIGSMSGLGC [SEQ ID NO. 3]; GLSKGCFGLK(U)LDRIGSMSGLGC [SEQ ID NO. 4]; U-CFGLKLDRIGSxSGLGC, where x is a natural or unnatural amino acid residue [SEQ ID NO. 12], or any combination thereof,
wherein:
U is a moiety of Formula (I), where Formula (I) is

   (aliphatic)ₐ-(X)-; (I)
wherein
   a is 0 or 1 (preferably a is 1);
   aliphatic is an optionally substituted C₁₀₋₂₄ chain (*e.g*., an optionally substituted C₁₂₋₁₈ chain), covalently bound to X via a chemical linkage, such as a carbonyl (*e.g*., as part of an amide or an ester linkage), a thioether, an ether, a thioether, a carbamate moiety, a bond, or the like with X; preferably via a carbonyl as part of an amide or an ester linkage; or more preferably via a carbonyl as part of an amide linkage with X;
   X is a 1-10 amino acid residue or peptide sequence, wherein each amino acid residue is independently selected from lysine (K), arginine (R), glycine (G), alanine (A), glutamic acid (E), and aspartic acid (D); or
   X is a linker (γE)ₘ-(B)ₙ,
   wherein B is a 1-8 amino acid residue or peptide sequence wherein each amino acid residue is independently selected from 2-[2-(2-aminoethoxy)ethoxy]acetic acid residue, Gly, Ala, Leu, Ser, Arg, and Lys;
   m is 0, 1, 2, or 3;
   n is 0, 1, 2, or 3; and
   the sum of m and n is at least 1.

In some embodiments, x in U-CFGLKLDRIGSxSGLGC [SEQ ID NO. 12] is not a methionine residue, is not an asparagine residue, or is neither a methionine nor an asparagine residue. In some embodiments, x is not any one of the 20 natural amino acid residues encoded by the mammalian genetic code, such as amino acids A, C, D, E, F, G, H, I, K, L, M, N, P, Q, R, S, T, V, W, and Y. In some embodiments, x is an unnatural amino acid residue (*i.e*., an amino acid residue not encoded by the mammalian genetic code). In some embodiments, x is homoglutamine (also referred to herein as homoQ).

In some embodiments, X is a 4-7 amino acid residue or peptide sequence, wherein each amino acid residue is independently selected from lysine (K), arginine (R), and glycine (G).

In some embodiments, the long acting CNP derivative or the very long acting CNP derivative includes U-GLSKGCFGLKLDRIGSMSGLGC [SEQ ID NO. 2],
wherein:
U is (aliphatic)ₐ-(X)-;
wherein:
   a is 1;
   aliphatic is an optionally substituted C₄₋₂₄ chain (*e.g*., an optionally substituted C₁₀₋₂₄ chain, an optionally substituted C₁₂₋₁₈ chain), covalently bound to X via a chemical linkage, such as a carbonyl (*e.g*., as part of an amide or an ester linkage), a thioether, an ether, a thioether, a carbamate moiety, a bond, or the like with X; preferably via a carbonyl as part of an amide or an ester linkage; or more preferably via a carbonyl as part of an amide linkage with X;
   X is a 1-10 amino acid residue or peptide sequence, wherein each amino acid residue is independently selected from lysine (K), arginine (R), glycine (G), alanine (A), glutamic acid (E), and aspartic acid (D).

In some embodiments, the long acting CNP derivative or the very long acting CNP derivative includes U-CFGLKLDRIGSxSGLGC [SEQ ID NO. 13], x is homoglutamine; U is (aliphatic)ₐ-(X)-, wherein a is 0 or 1 (preferably a is 1), aliphatic is a branched or straight optionally substituted C₁₈ chain covalently bound to X via a carbonyl (*e.g*., CH₃(CH₂)₁₆C(=O)) or an optionally substituted C₁₈ chain covalently bound to X via a chemical linkage, such a thioether, an ether, a thioether, a carbamate moiety, a bond, or the like with X; preferably aliphatic is a branched or straight optionally substituted C₁₈ chain covalently bound to X via a carbonyl (*e.g*., CH₃(CH₂)₁₆C(=O)) as part of an amide or ester linkage with X; or more preferably aliphatic is a branched or straight optionally substituted C₁₈ chain covalently bound to X via a carbonyl (*e.g*., CH₃(CH₂)₁₆C(=O)) as part of an amide linkage with X, or aliphatic is HOC(=O)(CH₂)₁₆C(=O)); X is a linker (γE)ₘ-(B)ₙ; B is Gly; m is 0, 1, or 2; and n is 1.

In some embodiments, the long acting CNP derivative or the very long acting CNP derivative includes U-CFGLKLDRIGSxSGLGC [SEQ ID NO. 14], x is homoglutamine; U is (aliphatic)ₐ-(X)-, wherein a is 0 or 1 (preferably a is 1), aliphatic is a branched or straight optionally substituted C₁₈ chain covalently bound to X via a carbonyl (*e.g*., CH₃(CH₂)₁₆C(=O)) or an optionally substituted C₁₈ chain covalently bound to X via a chemical linkage, such a thioether, an ether, a thioether, a carbamate moiety, a bond, or the like with X; preferably aliphatic is a branched or straight optionally substituted C₁₈ chain covalently bound to X via a carbonyl (*e.g*., CH₃(CH₂)₁₆C(=O)) as part of an amide or ester linkage with X; or more preferably aliphati c is a branched or straight optionally substituted C₁₈ chain covalently bound to X via a carbonyl (*e.g.,* CH₃(CH₂)₁₆C(=O)) as part of an amide linkage with X, or aliphatic is HOC(=O)(CH₂)₁₆C(=O)); X is a linker (γE)ₘ-(B)ₙ; B is Gly; m is 1; and n is 1.

In some embodiments, the long acting CNP derivative or the very long acting CNP derivative includes U-CFGLKLDRIGSxSGLGC[SEQ ID NO. 15], x is homoglutamine; U is (aliphatic)ₐ-(X)-, wherein a is 0 or 1 (preferably a is 1), aliphatic is a branched or straight optionally substituted C₁₈ chain covalently bound to X via a carbonyl (*e.g*., CH₃(CH₂)₁₆C(=O)) or an optionally substituted C₁₈ chain covalently bound to X via a chemical linkage, such a thioether, an ether, a thioether, a carbamate moiety, a bond, or the like with X; preferably aliphatic is a branched or straight optionally substituted C₁₈ chain covalently bound to X via a carbonyl (*e.g*., CH₃(CH₂)₁₆C(=O)) as part of an amide or ester linkage with X; or more preferably aliphati c is a branched or straight optionally substituted C₁₈ chain covalently bound to X via a carbonyl (*e.g*., CH₃(CH₂)₁₆C(=O)) as part of an amide linkage with X, or aliphatic is HOC(=O)(CH₂)₁₆C(=O)); X is a linker (γE)ₘ-(B)ₙ; m is 1; and n is 0.

In some embodiments, the long acting CNP derivative or the very long acting CNP derivative includes U-CFGLKLDRIGSxSGLGC, where x is a natural or unnatural amino acid residue [SEQ ID NO. 12], wherein U is (aliphatic)ₐ-(X)-; a is 0 or 1 (preferably a is 1); aliphatic is an optionally substituted C₄₋₂₄ chain (*e.g*., an optionally substituted C₁₀₋₂₄ chain, an optionally substituted C₁₂₋₁₈ chain), covalently bound to X via a chemical linkage, such as a carbonyl (*e.g*., as part of an amide or an ester linkage), a thioether, an ether, a thioether, a carbamate moiety, a bond, or the like with X; preferably via a carbonyl as part of an amide or an ester linkage; or more preferably via a carbonyl as part of an amide linkage with X, or aliphatic is HOC(=O)(CH₂)₁₆C(=O)); X is a linker (γE)ₘ-(B)ₙ, wherein B is a 1-8 amino acid residue or peptide sequence wherein each amino acid residue is independently selected from 2-[2-(2-aminoethoxy)ethoxy]acetic acid residue, Gly, Ala, Leu, Ser, Arg, and Lys; m is 0, 1, 2, or 3; n is 0, 1, 2, or 3; and the sum of m and n is at least 1. In some embodiments, x in U-CFGLKLDRIGSxSGLGC [SEQ ID NO. 12] is not a methionine residue, is not an asparagine residue, or is neither a methionine nor an asparagine residue. In some embodiments, x is not any one of the 20 natural amino acid residues encoded by the mammalian genetic code, such as amino acids A, C, D, E, F, G, H, I, K, L, M, N, P, Q, R, S, T, V, W, and Y. In some embodiments, x is an unnatural amino acid residue (*i.e*., an amino acid residue not encoded by the mammalian genetic code). In some embodiments, x is homoglutamine (also referred to herein as homoQ).

In some embodiments, the long acting CNP derivative or the very long acting CNP derivative includes U-CFGLKLDRIGSxSGLGC, where x is homoglutamine (homoQ) [SEQ ID NO. 16], U is (aliphatic)ₐ-(X)-; wherein a is 0 or 1 (preferably a is 1); aliphatic is a branched or straight optionally substituted C₁₈ chain covalently bound to X via a carbonyl (*e.g*., CH₃(CH₂)₁₆C(=O)) or an optionally substituted C₁₈ chain covalently bound to X via a chemical linkage, such a thioether, an ether, a thioether, a carbamate moiety, a bond, or the like with X; preferably aliphatic is a branched or straight optionally substituted C₁₈ chain covalently bound to X via a carbonyl (*e.g*., CH₃(CH₂)₁₆C(=O)) as part of an amide or ester linkage with X; or more preferably aliphatic is a branched or straight optionally substituted C₁₈ chain covalently bound to X via a carbonyl (*e.g*., CH₃(CH₂)₁₆C(=O)) as part of an amide linkage with X, or aliphatic is HOC(=O)(CH₂)₁₆C(=O)); X is a linker (γE)ₘ-(B)ₙ; B is 2-[2-(2-aminoethoxy)ethoxy]acetic acid residue, m is 0, and n is 2.

In some embodiments, the long acting CNP derivative or the very long acting CNP derivative includes U-CFGLKLDRIGSxSGLGC, where x is homoglutamine (homoQ) [SEQ ID NO. 17], U is (aliphatic)ₐ-(X)-; wherein a is 0 or 1 (preferably a is 1); aliphatic is a branched or straight optionally substituted C₁₈ chain covalently bound to X via a carbonyl (e.g., CH₃(CH₂)₁₆C(=O)) or an optionally substituted C₁₈ chain covalently bound to X via a chemical linkage, such a thioether, an ether, a thioether, a carbamate moiety, a bond, or the like with X; preferably aliphatic is a branched or straight optionally substituted C₁₈ chain covalently bound to X via a carbonyl (*e.g*., CH₃(CH₂)₁₆C(=O)) as part of an amide or ester linkage with X; or more preferably aliphatic is a branched or straight optionally substituted C₁₈ chain covalently bound to X via a carbonyl (*e.g*., CH₃(CH₂)₁₆C(=O)) as part of an amide linkage with X, or aliphatic is HOC(=O)(CH₂)₁₆C(=O)); X is a linker (γE)ₘ-(B)ₙ; B is 2-[2-(2-aminoethoxy)ethoxy]acetic acid residue, m is 1, and n is 2.

In some embodiments, the long acting CNP derivative or the very long acting CNP derivative includes U-CFGLKLDRIGSxSGLGC, where x is homoglutamine (homoQ) [SEQ ID NO. 18], U is (aliphatic)ₐ-(X)-; wherein a is 0 or 1 (preferably a is 1); aliphatic is a branched or straight optionally substituted C₁₈ chain covalently bound to X via a carbonyl (*e.g*., CH₃(CH₂)₁₆C(=O)) or an optionally substituted C₁₈ chain covalently bound to X via a chemical linkage, such a thioether, an ether, a thioether, a carbamate moiety, a bond, or the like with X; preferably aliphatic is a branched or straight optionally substituted C₁₈ chain covalently bound to X via a carbonyl (*e.g*., CH₃(CH₂)₁₆C(=O)) as part of an amide or ester linkage with X; or more preferably aliphatic is a branched or straight optionally substituted C₁₈ chain covalently bound to X via a carbonyl (*e.g*., CH₃(CH₂)₁₆C(=O)) as part of an amide linkage with X, or aliphatic is HOC(=O)(CH₂)₁₆C(=O)); X is a linker (γE)ₘ-(B)ₙ; B is (2-[2-(2-aminoethoxy)ethoxy]acetic acid)-(2-[2-(2-aminoethoxy)ethoxy]acetic acid)-(Gly), m is 0, and n is 1.

In some embodiments, the long acting CNP derivative or the very long acting CNP derivative includes U-CFGLKLDRIGSxSGLGC, where x is homoglutamine (homoQ) [SEQ ID NO. 19], U is (aliphatic)ₐ-(X)-; wherein a is 0 or 1 (preferably a is 1); aliphatic is a branched or straight optionally substituted C₁₈ chain covalently bound to X via a carbonyl (*e.g*., CH₃(CH₂)₁₆C(=O)) or an optionally substituted C₁₈ chain covalently bound to X via a chemical linkage, such a thioether, an ether, a thioether, a carbamate moiety, a bond, or the like with X; preferably aliphatic is a branched or straight optionally substituted C₁₈ chain covalently bound to X via a carbonyl (*e.g.*, CH₃(CH₂)₁₆C(=O)) as part of an amide or ester linkage with X; or more preferably aliphatic is a branched or straight optionally substituted C₁₈ chain covalently bound to X via a carbonyl (*e.g*., CH₃(CH₂)₁₆C(=O)) as part of an amide linkage with X, or aliphatic is HOC(=O)(CH₂)₁₆C(=O)); X is a linker (γE)ₘ-(B)ₙ; B is (2-[2-(2-aminoethoxy)ethoxy]acetic acid)-(2-[2-(2-aminoethoxy)ethoxy]acetic acid)-(Gly), m is 1, and n is 1.

In some embodiments, the long acting CNP derivative or the very long acting CNP derivative U-CFGLKLDRIGSxSGLGC is HOC(=O)(CH₂)₁₆C(=O)-γE-Aeea-Aeea-GCFGLKLDRIGShomoQSGLGC with a disulfide bond between the cysteine residues (homoQ: homoGlutamine; Aeea: 2-[2-(2-aminoethoxy)ethoxy]acetic acid residue; HOC(=O)(CH₂)₁₆C(=O): octadecadioic acid reacted with γE so that a carbonyl (C(=O)) remains from one of the original octadecadioic acid carboxylic acid terminus; yE: glutamic acid conjugated through gamma-carboxy group [SEQ ID NO. 20].

In some embodiments, the long acting CNP derivative or the very long acting CNP derivative U-CFGLKLDRIGSxSGLGC is HOC(=O)(CH₂)₁₆C(=O)-Aeea-Aeea-GCFGLKLDRIGShomoQSGLGC with a disulfide bond between the cysteine residues (homoQ: homoGlutamine; Aeea: 2-[2-(2-aminoethoxy)ethoxy]acetic acid residue; HOC(=O)(CH₂)₁₆(CO): octadecadioic acid reacted with the amino terminus of Aeea so that a carbonyl (C(=O)) remains from the original octadecadioic acid carboxylic acid terminus; [SEQ ID NO. 21].

In some embodiments, in any of the definitions herein, aliphatic does not include one or more of a straight or branched optionally substituted C₄₋₉ chain (*e.g*., an optionally substituted C₃₋₈ chain-C(=O)- moiety, and/or an optionally substituted C₄₋₉ chain that is covalently bound to the peptide via a linkage such as a thioether, an ether, a thioether, a carbamate moiety, a bond, or the like). In certain embodiments, aliphatic is not a straight or branched C₈ chain (*e.g*., a straight or branched C₈ chain covalently bound to the peptide via a linkage such as a carbonyl, thioether, an ether, a thioether, a carbamate moiety, a bond, or the like).

In some embodiments, U as described above includes CH₃(CH₂)₁₄C(=O)KKKKGGG- [SEQ ID NO. 22]; CH₃(CH₂)₁₆C(=O)KKKKGGG-[SEQ ID NO. 23]; CH₃(CH₂)₁₈C(=O)KKKKGGG- [SEQ ID NO. 24]; CH₃(CH₂)₂₀C(=O)KKKKGGG- [SEQ ID NO. 25]; or CH₃(CH₂)₂₂C(=O)KKKKGGG [SEQ ID NO. 26].

In some embodiments, the long acting CNP derivatives of the present disclosure includes CH₃(CH₂)₁₄C(=O)KKKKGGGGLSKGCFGLKLDRIGSMSGLGC [SEQ ID NO. 5]; CH₃(CH₂)₁₆C(=O)KKKKGGGGLSKGCFGLKLDRIGSMSGLGC [SEQ ID NO. 6]; CH₃(CH₂)₁₈C(=O)KKKKGGGGLSKGCFGLKLDRIGSMSGLGC [SEQ ID NO. 7]; CH₃(CH₂)₂₀C(=O)KKKKGGGGLSKGCFGLKLDRIGSMSGLGC [SEQ ID NO. 8]; CH₃(CH₂)₂₂C(=O)KKKKGGGGLSKGCFGLKLDRIGSMSGLGC [SEQ ID NO. 9]; HOC(=O)(CH₂)₁₆C(-O)-γE-Aeea-Aeea-GCFGLKLDRIGShomoQSGLGC including a disulfide bond between the cysteine residues [SEQ ID NO. 20]; and/or HOC(=O)(CH₂)₁₆C(=O)-Aeea-Aeea-GCFGLKLDRIGShomoQSGLGC including a disulfide bond between the cysteine residues [SEQ ID NO. 21].

In certain embodiment, the long acting CNP derivatives of the present disclosure includes CH₃(CH₂)₁₆C(=O)KKKKGGGGLSKGCFGLKLDRIGSMSGLGC [SEQ ID NO. 6].

In some embodiments, the long acting CNP derivative or the very long acting CNP derivative includes U-GLSKGCFGLKLDRIGSMSGLGC [SEQ ID NO. 2], U-GLSKGCFGLK(U)LDRIGSMSGLGC [SEQ ID NO. 3], GLSKGCFGLK(U)LDRIGSMSGLGC [SEQ ID NO. 4], U-CFGLKLDRIGSxSGLGC, where x is a natural or unnatural amino acid residue [SEQ ID NO. 27], or any combination thereof,
wherein U is a moiety of Formula (II), where Formula (II) is

   (polymer)ₐ-(Y)-; (II)
wherein a is 0 or 1 (preferably a is 1);
polymer is cellulose, poly(ethylene glycol) (PEG), methoxy poly(ethylene glycol) (MPEG), poly(lactic-co-glycolic acid), or poly(N-vinyl pyrrolidone);
Y is:
   a 4-10 amino acid residue or peptide sequence, wherein each amino acid residue is independently selected from lysine (K), arginine (R), and glycine (G);
   a non-amino acid linker comprising an ester, an amide, a thioether, an ether, a thioether, a carbamate moiety, or a combination thereof; or
   a linker (γE)ₘ-(B)ₙ, wherein B is 1-8 amino acid residue or peptide sequence wherein each amino acid residue is independently selected from 2-[2-(2-aminoethoxy)ethoxy]acetic acid residue, Gly, Ala, Leu, Ser, Arg, and Lys; m is 0, 1, 2, or 3; n is 0, 1, 2, or 3; and the sum of m and n is at least 1.

In some embodiments, the long acting CNP derivative or the very long acting CNP derivative includes U-GLSKGCFGLKLDRIGSMSGLGC [SEQ ID NO. 2], U-GLSKGCFGLK(U)LDRIGSMSGLGC [SEQ ID NO. 3], or any combination thereof;
wherein U is a moiety of Formula (II), where Formula (II) is

   (polymer)ₐ-(Y)-; (II)
wherein a is 0 or 1 (preferably a is 1);
polymer is cellulose, poly(ethylene glycol) (PEG), methoxy poly(ethylene glycol) (MPEG), poly(lactic-co-glycolic acid), poly(N-vinyl pyrrolidone), or a derivative thereof;
Y is:
   a 1-10 amino acid residue or peptide sequence, wherein each amino acid residue is independently selected from lysine (K), arginine (R), glycine (G), alanine (A), glutamic acid (E), and aspartic acid (D);
   a non-amino acid linker comprising an ester, an amide, a thioether, an ether, a thioether, a carbamate moiety, or a combination thereof;
   an amino acid residue-containing linker, wherein the amino acid residue is covalently attached to (polymer)ₐ;
   a peptide linker different from the 1-10 amino acid residue or peptide sequence.

In some embodiments, Y in Formula (II) above is a linker -(γE)ₘ-(B)ₙ, wherein B is 1-8 amino acid residue or peptide sequence wherein each amino acid residue is independently selected from 2-[2-(2-aminoethoxy)ethoxy]acetic acid residue, Gly, Ala, Leu, Ser, Arg, and Lys; m is 0, 1, 2, or 3; n is 0, 1, 2, or 3; and the sum of m and n is at least 1.

In some embodiments, the polymer does not include poly(ethylene glycol), MPEG, or both poly(ethylene glycol) and MPEG.

In some embodiments, Y is a 4-10 amino acid residue or peptide sequence, wherein each amino acid residue is independently selected from lysine (K), arginine (R), and glycine (G); or a linker (γE)ₘ-(B)ₙ, wherein B is 1-8 amino acid residue or peptide sequence wherein each amino acid residue is independently selected from 2-[2-(2-aminoethoxy)ethoxy] acetic acid residue, Gly, Ala, Leu, Ser, Arg, and Lys; m is 0, 1, 2, or 3; n is 0, 1, 2, or 3; and the sum of m and n is at least 1.

In some embodiments, Y is a 4-10 amino acid residue sequence, wherein each amino acid residue is independently selected from lysine (K), arginine (R), and glycine (G).

In some embodiments, Y is a linker (γE)ₘ-(B)ₙ, wherein B is 1-8 amino acid residue or peptide sequence wherein each amino acid residue is independently selected from 2-[2-(2-aminoethoxy)ethoxy]acetic acid residue, Gly, Ala, Leu, Ser, Arg, and Lys; m is 0, 1, 2, or 3; n is 0, 1, 2, or 3; and the sum of m and n is at least 1.

In some embodiments, the CNP or derivatives thereof of the present disclosure does not include CNP that is modified with polyalkylene glycol at the lysine residues at positions 4 and 10 of SEQ ID NO. 10 and/or at the N-terminus of the CNP of SEQ ID NO. 10.

In some embodiments, the formulations including long acting CNP derivatives of the present disclosure includes one or more CNP or derivatives thereof formulated with a polymer excipient including a poly(amino acid) grafted with polyethylene glycol, fatty acid, and/or anionic moieties. The polymer is adapted to sequester or non-covalently bind to the CNP derivative(s).

In some embodiments, the formulations including very long acting CNP derivatives of the present disclosure includes one or more long acting CNP derivatives formulated with a polymer excipient including a poly(amino acid) grafted with polyethylene glycol, fatty acid, and/or anionic moieties. The polymer is adapted to sequester or non-covalently bind to the CNP derivative(s).

In some embodiments, the formulations including long acting NPRB agonist(s) of the present disclosure includes one or more CNP or derivatives thereof formulated with a polymer excipient including a poly(amino acid) grafted with polyethylene glycol, fatty acid, and/or anionic moieties. The polymer is adapted to sequester or non-covalently bind to the NPRB agonist(s).

In some embodiments, the formulations including very long acting NPRB agonist of the present disclosure includes one or more long acting CNP derivatives formulated with a polymer excipient including a poly(amino acid) grafted with polyethylene glycol, fatty acid, and/or anionic moieties. The polymer is adapted to sequester or non-covalently bind to the NPRB agonist(s).

The poly(amino acid) that is grafted with polyethylene glycol, fatty acid, and/or anionic moieties can include a poly(amino acid) which may have D- or L-chirality or both and is a straight chain homopolymer. In one specific embodiment, straight chain homopolymers include polylysine, polyomithine, polyarginine, polyglutamate, polyaspartate, polyserine, polytyrosine, or any other amide linked homopolymer made from amino acids. In another preferred embodiment, straight chain hydrophobic homopolymers comprise polyalanine, polyvaline, polyleucine, polyisoleucine, polyglycine, or polyphenylalanine. In some embodiments, the poly(amino acid) is polylysine.

### Methods of making the active pharmaceutical ingredients

The peptides of the present disclosure, such as the long acting CNP, long acting CNP derivative, and long acting NPRB agonist can be synthesized by solid phase peptide synthesis (SPPS) using methods known to a person of ordinary skill in the art. For example, a starting solid support, such as H-Cys(Trt)-2-Cl-Trt Resin (BLDPharm, Shanghai, China) could be used in a peptide synthesizer, such as an automated microwave peptide synthesizer (e.g., LibertyBlue HT12, CEM, Matthews, NC). Each amino acid, fatty acid, or protected alkyl carboxylic (di)acid can be anchored sequentially onto the peptide resin using Fmoc chemistry, known to those of ordinary skill in the art, resulting in a linear protected peptide linked to the resin. Linear crude peptide can be deprotected and released from the resin by acidolysis with trifluoroacetic acid in the presence of carbocation scavengers and ether precipitation. The resulting linear peptide can be cyclized, for example, by dissolving in DMSO and acetonitrile aqueous solution and reacted to form disulfide bond. Finally, the peptide can be purified and characterized by reversed phase HPLC (*e.g*., 1260 Infinity II Preparative LC Systems, Santa Clara, CA). Fractions with >90% purity of the final peptide product can be collected and dried as white powder.

In some embodiments, the formulations including the active pharmaceutical ingredient(s) ("APIs") of the present disclosure has a weight ratio of a polymer excipient relative to APIs such that the resulting mixture is a long acting, or very long acting. For example, the weight ratio of the polymer excipient to total API can be from 5:1 to 100:1, 10:1 to 50:1 or 20:1 to 5:1. The polymer excipient can include a poly(amino acid) grafted with polyethylene glycol, fatty acid, and/or anionic moieties. The polymer excipient is adapted to sequester or non-covalently bind to the APIs. Examples of polymer excipients are described, for example, in Castillo et al., Pharm. Res., (2012) 29(1); p 306-18; Castillo *et al.,* PLoS One, (2017) 12(2); e0171703; US patent nos. 10,507,248; 10,035,885; and 10,010,613, each of which is herein incorporated by reference in its entirety. The polymer excipient can be, but is not limited to polylysine grafted with PEG at the epsilon amino to a level between 10-55% (*e.g*., 10-35%, or 30-55%) of total epsilon amino and the remainder amino groups grafted with alkyl group and/or anionic moieties such as sulfate, sulfonate, carboxyl, phosphate, or phosphonate. Methods of making polymer excipients are known in the art.

Briefly, in some embodiments, the polymer excipient is a polymer made by the following procedure. Poly-L-lysine (20PL), hydrobromide (21 µmol or 1 g; Sigma, Average Mw=26 kDa; d.p.126) was dissolved and the amount of NH₂-groups determined by TNBS titration. Methoxy polyethylene glycol carboxymethyl (MPEG-CM; 10 g; Mw=5 kDa; 2 mmol; Laysan Bio) was coupled to the polylysine using NHSS and EDC to provide the polymer excipient intermediate. The percent amino groups remaining was determined by TNBS. The hydrodynamic diameter was determined by size exclusion chromatography. The crude product can be lyophilized. Stearyl-NHS (C18-NHS) was prepared by activating stearic acid with NHS. DCC coupling of stearyl-NHS to the polymer excipient intermediate can be conducted. Excess reagents and side products can be removed standard techniques. Additional C18-NHS (3.6 mmol) was added and allowed to react with the polymer intermediate overnight. The reaction mixture was concentrated by rotary evaporation under vacuum to remove volatile components until an oil is isolated. The oil can be dissolved in alcohol and water. The solution can be filtered, washed repeatedly to provide a retentate containing the polymer excipient (polylysine with C18 hydrophobic side chains and MPEG hydrophilic side chains) was collected, 0.2 um filtered (polysulfone filter, Nalgene, Rochester, NY) and lyophilized, to provide the dry polymer excipient.

While a polymer excipient having C₁₈ hydrophobic side chains is described above, it is understood that other hydrophobic side chain lengths (*e.g*., C₁₀₋₂₄, C₁₂₋₂₀, C₁₂₋₁₈, C₁₄₋₁₈, C₁₆₋₁₈, or C₁₈) and hydrophilic side chains (*e.g*., PEG, mPEG) can be adapted to make polymer excipients having other hydrophobic side chains and hydrophilic side chains.

The poly(amino acid) that is grafted with polyethylene glycol, fatty acid, and/or anionic moieties can include a poly(amino acid) which may have D- or L-chirality or both and is a straight chain homopolymer. In one specific embodiment, straight chain homopolymers include polylysine, polyomithine, polyarginine, polyglutamate, polyaspartate, polyserine, polytyrosine, or any other amide linked homopolymer made from amino acids. In another preferred embodiment, straight chain hydrophobic homopolymers comprise polyalanine, polyvaline, polyleucine, polyisoleucine, polyglycine, or polyphenylalanine. In some embodiments, the poly(amino acid) is polylysine.

Examples of hydrophilic side chains include poly(ethylene glycol), which may be esterified by dicarboxylic acid to form a poly(ethylene glycol) monoester; methoxy poly(ethylene glycol) monoester (MPEG) or a co-polymer of poly(ethylene glycol) and poly(propylene glycol) monoester in a form of an ester with a dicarboxylic acid giving the terminal of this co-polymers a carboxyl group that can be used to covalently link it to a poly(amino acid). Other forms include poly(ethylene glycol)-carboxyl; methoxy poly(ethylene glycol)-carboxyl; poly(ethylene glycol)-carboxymethyl; methoxy poly(ethylene glycol)-carboxymethyl; poly(ethylene glycol) monoamine; methoxy poly(ethylene glycol) monoamine; poly(ethylene glycol) hydrazide; methoxy poly(ethylene glycol) hydrazide; methoxy poly(ethylene glycol) imidazolide block-co-polymer of poly (ethylene glycol) and one or several polymers represented by polyaminoacid, polysaccharide, polyamidoamine, polyethyleneimine where these blocks are alternated to give a linear block-co-polymer. In one embodiment, the overall molecular weight of a protective chain may be larger than 300 Daltons but not exceeding 10,000 Daltons. In one embodiment, one or more protective chains are linked to the poly(amino acid) backbone by a single linkage.

Without wishing to be bound by theory, it is believed that the higher the weight ratio of polymer excipient to APIs, the more sustained the presence in the plasma and the more sustained the plasma cyclic-GMP elevation over the baseline, when the API composition is administered to a subject.

In some embodiments, the formulations including the long acting CNP, long acting CNP derivative, and/or the long acting NPRB agonist of the present disclosure has a weight ratio of a polymer excipient relative to CNP, CNP derivative, and/or NPRB agonist such that the resulting mixture is a long acting CNP, long acting CNP derivative, and/or long acting NPRB agonist. For example, the weight ratio of the polymer excipient to CNP, CNP derivative, and/or the NPRB agonist can be from 5:1 to 100:1, 10:1 to 50:1 or 20:1 to 5:1. The polymer excipient can include a poly(amino acid) grafted with polyethylene glycol, fatty acid, and/or anionic moieties. *See, e.g.,* Castillo et al., Pharm. Res., (2012) 29(1); p 306-18; Castillo et al., PLoS One, (2017) 12(2); e0171703; US patent nos. 10,507,248; 10,035,885; 10,010,613, each of which is herein incorporated by reference in its entirety. The polymer excipient is adapted to sequester or non-covalently bind to the CNP, CNP derivative, and/or the NPRB agonist. The polymer excipient can be, but is not limited to polylysine grafted with PEG at the epsilon amino to a level between 30-55% or 10-35% of total epsilon amino and the remainder amino groups grafted with alkyl group and/or anionic moieties such as sulfate, sulfonate, carboxyl, phosphate, or phosphonate. Methods of making polymer excipients are known in the art. Without wishing to be bound by theory, it is believed that the higher the weight ratio of polymer excipient to CNP, CNP derivative, and/or the NPRB agonist, the more sustained the CNP, CNP derivative, or NPRB agonist presence in the plasma and the more sustained the plasma cyclic-GMP elevation over the baseline, when the CNP, CNP derivative, and/or NPRB agonist composition is administered to a subject.

In some embodiments, the very long acting CNP, very long acting CNP derivative, and/or very long acting NPRB agonist formulations include CNP, CNP derivative, and/or NPRB agonist and a polymer excipient at a weight ratio of polymer excipient relative to CNP, CNP derivative, and/or NPRB agonist such that the resulting mixture is a very long acting CNP, a very long acting CNP derivative, and/or a very long acting NPRB agonist. For example, the weight ratio of the polymer excipient to CNP, CNP derivative, and/or NPRB agonist can be from 5:1 to 100:1, 10:1 to 50:1 or 20:1 to 5:1. The polymer excipient can include a poly(amino acid) grafted with polyethylene glycol, fatty acid, and/or anionic moieties. *See, e.g.,* Castillo et al., Pharm. Res., (2012) 29(1); p 306-18; Castillo et al., PLoS One, (2017) 12(2); e0171703; US patent nos. 10,507,248; 10,035,885; 10,010,613 each of which is herein incorporated by reference in its entirety. The polymer excipient is adapted to sequester or non-covalently bind to the CNP, CNP derivative, and/or NPRB agonist. The polymer excipient can be, but is not limited to polylysine grafted with PEG at the epsilon amino to a level between 30-55% or 10-35% of total epsilon amino and the remainder amino groups grafted with alkyl group and/or anionic moieties such as sulfate, sulfonate, carboxyl, phosphate, or phosphonate. Methods of making polymer excipients are known in the art. Without wishing to be bound by theory, it is believed that the higher the weight ratio of polymer excipient to CNP, CNP derivative, and/or NPRB agonist, the more sustained the CNP, CNP derivative, and/or NPRB agonist presence in the plasma and the more sustained the plasma cyclic-GMP elevation over the baseline, when the CNP, CNP derivative, and/or NPRB agonist composition is administered to a subject.

### Conditions

In some embodiments, any of the methods of the present disclosure includes treating ALI. In certain embodiment, any of the methods of the present disclosure includes treating ARDS. In some embodiments, the methods of the present disclosure include treating pulmonary edema. In some embodiments, the methods of the present disclosure include treating low arterial blood oxygenation. In certain embodiments, the methods of the present disclosure include treating elevated level of inflammatory cells in the lungs. In some embodiments, the methods of the present disclosure include treating sepsis. In some embodiments, the methods of the present disclosure include treating bacteremia. In yet some embodiments, the methods of the present disclosure include treating lung/pulmonary fibrosis. In some embodiments, the methods of the present disclosure include treating fibrosis in general (*e.g*., lung/pulmonary fibrosis, cirrhosis, and/or kidney glomerular sclerosis), and/or kidney injury.

In some embodiments, when the treated condition is ALI or ARDS, the ALI or ARDS is caused by, or associated with, any one of (i) a systemic insult selected from trauma, sepsis (*i.e*., body-wide infection), bacteremia (*i.e*., bacteria in the blood), pancreatitis, shock, multiple transfusions, disseminated intravascular coagulation, burns, drug overdose or toxicity, opioids, aspirin, phenothiazines, tricyclic antidepressant, amiodarone, chemotherapeutic agents, nitrofurantoin, protamine, thrombotic thrombocytopenia purpura, head injury, and/or paraquat; and/or (ii) a pulmonary insult selected from aspiration of gastric content, lung intubation, embolism (*e.g*., from thrombus, fat, air, or amniotic fluid), tuberculosis, viral pneumonia (*e.g*., SARS caused by coronavirus or influenza virus), bacterial pneumonia, cytogenic organizing pneumonitis, airway obstruction, smoking free-base cocaine, near-drowning, toxic gas inhalation, oxygen toxicity, lung contusion, radiation exposure, high-altitude exposure, lung re-expansion, and/or reperfusion.

In some embodiments, when the treated condition is ALI or ARDS, the ALI or ARDS can be caused by an infectious disease, wherein the infectious disease is caused by coronavirus or influenza virus, pulmonary fibrosis, sepsis; bacteremia; intubation; and/or a toxic gas selected from group consisting of chlorine gas, smoke, phosgene, and/or concentrated oxygen.

In certain embodiments, when the treated condition is ALI or ARDS, the ALI or ARDS is caused by an infectious disease (*e.g*., wherein the infectious disease is caused by coronavirus or influenza virus).

In certain embodiments, when the treated condition is ALI or ARDS, the ALI or ARDS is caused by pulmonary fibrosis.

In certain embodiments, when the treated condition is ALI or ARDS, the ALI or ARDS is caused by sepsis.

In further embodiment, when the treated condition is ALI or ARDS, the ALI or ARDS is caused by bacteremia.

In further embodiment, when the treated condition is ALI or ARDS, the ALI or ARDS is caused by intubation.

In further embodiment, when the treated condition is ALI or ARDS, the ALI or ARDS is caused by a toxic gas, such as chlorine gas, smoke, phosgene, concentrated oxygen, or any combinations thereof.

"Acute lung injury"/"acute respiratory distress syndrome" (ALI/ARDS) refers to a life-threatening clinical lung syndrome with a 28-day mortality of 30 to 50%. The prevalence of ALI/ARDS is about 200,000 cases / year in the US. *See, e.g.,* Johnson E.R., and Matthay MA, J Aerosol Med Pulm Drug Deliv. 23(4):243-52, 2010. ALI is a syndrome, or a condition characterized by lung alveolar injury, resulting from disruption of endothelial and epithelial barriers, a neutrophilic inflammatory response, pulmonary edema, and marked dysfunction in pulmonary blood oxygenation, lung compliance, and airway resistance. Acute respiratory distress Syndrome (ARDS) is a more severe form of ALI. As used herein, ALI includes both ALI and ARDS. For human clinical diagnostic purposes, ALI is defined by the North American-European consensus classification (*see*, *e.g.,* Henru et al., Intensive Care Med (2013) 39:2161-2170) based on the outcome or sum result of the conditions or syndrome described above, which is a decrease in blood oxygenation in a patient, in the absence of heart failure. This decrease is a PaO2:FiO2 (defined below) ratio of 200 to 300 mg Hg in the setting of a wedge pressure less than 18 mg Hg (*i.e*., not cardiovascular cause) along with radiographical presence (as determined by X-ray) of bilateral infiltrates ("infiltrates" term used by doctors reading chest X-rays) consistent with pulmonary edema. Patients with ARDS have rapid onset shortness of breath and very low oxygen levels in the blood with PaO2 below 63 mmHg or a PaO2:FIO2 ratio below 300 mg Hg, where PaO2 is partial pressure of oxygen in the arterial blood and FIO2 is the fractional concentration of inspired oxygen. Alternatively, or in addition, pulse oximetry, a sensor attached to a finger or an ear that uses light, may be used to determine blood oxygenation in terms of hemoglobin oxygen saturation, or SpO2. A well oxygenated blood has SpO2 of 96-99% and 90 and below is indicative of having PaO2:FiO2 ratio below 300 mg Hg. Other blood tests may be performed - including tests of kidney function, thyroid function, and blood count - as well as tests to exclude a heart attack (electro/echocardiogram) as the cause of any pulmonary edema rather than ALI.

ALI/ARDS can be caused by a wide variety of insults including sepsis (most common precipitating cause of ALI worldwide, *see, e.g*., Leonard D. Hudson, Arthur S. Slutsky, in Goldman's Cecil Medicine (Twenty Fourth Edition), 2012), aspiration of gastric content, shock, infection, lung contusion, non-thoracic trauma, toxic inhalation, near-drowning, and/or multiple blood transfusion. Pulmonary damage cause by influenza or corona virus (*e.g*., H1N1, SARS-Cov-1 & 2) can lead to life threatening ALI/ARDS. Mechanistically, these insults result in a disruption of alveolar endothelial and epithelial barriers, and leakage of fluid (pulmonary edema), plasma proteins and inflammatory cells (neutrophils, macrophages) into the lung/alveolar cavity limiting red blood cell access to oxygen from the alveolar air leading to severe hypoxemia, or very low hemoglobin oxygen saturation and respiratory distress. The accumulation of inflammatory cells is believed to be central in promoting and sustaining injury by generation of large amounts of oxygen-derived free radicals by these cells. Cytokines, growth factors, and degradative enzymes also are produced and released into the extracellular environment by inflammatory cells. These molecules and proteins can damage parenchymal cells in inflamed tissues and may lead to cell death. The severity of ALI correlates positively with the number of inflammatory cells (activated neutrophils) in the alveolar spaces. Early activation status of neutrophils in patients with ALI determines the clinical course of the disease. *See, e.g*., Yang et al., Am. J. Respir. Crit. Care Med 167:15671574, 2003. Many animal models of ALI are linked to the presence of elevated concentrations of neutrophils. *See*, *e.g*., Abraham et al., Am. J. Physiol. Lung Cell. Mol. Physiol. 279:1137-1145,2000; Flick et al., Circ. Res. 48:344-351, 1981; Heflin A C Jr and Brigham K L. J. Clin. Invest. 68:1253-1260, 1981; Shasby et al., Am. Rev. Respir. Dis. 125:443-447,1982.

Thus, "acute lung injury" (ALI) refers to a lung disorder in a mammal or a human resulting in very blood low oxygen levels with PaO2 (Partial pressure of arterial Oxygen) of less than about 60 mmHg or a PaO2:FiO2 ratio below 300 mg Hg (if the PaO2:FiO2 ratio is severely below 300 mg Hg, the condition is further characterized by shortness of breath), bilateral infiltrates radiographically consistent with pulmonary edema, in the absence of clinical evidence of cardiac failure. The term ALI also includes acute respiratory distress syndrome (ARDS), which is a more severe form of ALI. The PaO2:FiO2 ratio is the ratio of partial pressure of oxygen (PaO2) in the arterial blood and fractional concentration of inspired oxygen (FiO2) with no clinical evidence of cardiac failure. The FiO2 can be the fractional concentration of atmospheric oxygen of 0.21 (or 21%) but in hospital setting that uses oxygen as supportive care this can be up to 0.60. Because the FIO2 can vary depending on the use of oxygen in the inspired air, PaO2 alone below 60 mm Hg is an indicator of ALI, the extent of PaO2 lowering from 60mm Hg is indicative of a more severe the ALI/ARDS.

Because PaO2 require blood draw, pulse oximetry is also used to determine blood oxygenation. Pulse oximetry is a noninvasive method for monitoring a person's blood hemoglobin oxygen saturation (SO₂) now known as "fifth vital sign". It uses the absorbance of two wavelengths of light (in the extremities such as finger or ear), one for oxygenated hemoglobin and the other for deoxygenated hemoglobin to determine SO₂. In particular, deoxyhemoglobin absorbs light maximally in the red band of the spectrum (600 to 750 nm), and oxyhemoglobin absorbs maximally in the infrared band (850 to 1000 nm). The ratio of light absorbance between oxyhemoglobin and the sum of the absorbance of oxyhemoglobin plus deoxyhemoglobin is calculated and compared with previously calibrated direct measurements. ALI patients having an SO₂ below 90% correlates well with a PaO2 of below 60 mmHg and is diagnostic of ALI. All animal models of ALI are linked to presence of elevated concentrations of neutrophils and measurement of these is indicative of the severity and resolution of ALI. *See, e.g.,* Abraham et al., Am. J. Physiol. Lung Cell. Mol. Physiol. 279:1137-1145,2000; Flick et al., Circ. Res. 48:344-351, 1981; Heflin A. C. Jr. and Brigham K L. J. Clin. Invest. 68:1253-1260, 1981; Shasby et al., Am. Rev. Respir. Dis. 125:443-447,1982). This model is widely used by those skilled in the art in diagnosing the severity of ALI in mammalian subjects.

ALI is diagnosed or determined once the patient presents evidence of vital signs indicative of low blood oxygenation. These signs include shortness of breath, rapid breathing, blueish/cherry red skin, cough, wheezing, and sweating. The low blood oxygenation is confirmed by measuring blood oxygenation either by pulse oximetry or by blood draw and blood gas analysis to measure arterial blood oxygen partial pressure (PaO2). The blood gas analysis showing low blood oxygenation is a PaO2 of 60 mmHg and below or a ratio of arterial oxygen partial pressure to fractional inspired oxygen (PaO2/FiO2 ratio) below 300mmHg. Using oximetry, low blood oxygenation is indicated by a blood hemoglobin oxygen saturation (SpO2) below 90%. Pulse oximetry can also be used to monitor a person's blood hemoglobin oxygen saturation (SO₂). ALI patients have a SO₂ below 90%, which correlates well with PaO2 of below 300 mmHg and is diagnostic of ALI. The low oxygenation is due to pulmonary edema (also diagnostic of ALI) and can be determined by chest X-ray, which can confirm the diagnosis of pulmonary edema and exclude other possible causes of your shortness of breath.

ALI is also associated with an increase level of inflammatory cells in the lung as determined from bronchoalveolar lavage fluid (BALF). BALF is obtained and analyzed for the level and size of inflammatory cells. Bronchoalveolar lavage (BAL), performed during flexible bronchoscopy, has gained widespread acceptance as a minimally invasive method that provides important information about immunologic, inflammatory, and infectious processes taking place at the alveolar level. *See, e.g.,* Harbeck RJ, Clin Diagn Lab Immunol. 1998, 5(3):271-7]. In brief, the technique of BAL generally involves the introduction of a flexible fiber-optic bronchoscope transnasally while the patient is in a semi recumbent position. It is passed through the pharynx and vocal cords, into the trachea, and to the appropriate area of the lung. Aliquots of sterile saline (generally 30 to 40 ml) are instilled through the bronchoscope, which is immediately and gently withdrawn. One-hundred milliliters of saline can sample the constituents of about 1 million alveoli or about 1.5 to 3% of the lung and can recover about 1 ml of epithelial lining fluid. The total procedure takes less than 15 min. The cells recovered from the lung by lavage are much more heterogeneous than the cells obtained from peripheral blood. By flow cytometric examination the major cell populations include normal size macrophages, neutrophils, eosinophils, erythrocytes, and lymphocytes. During inflammation and ALI, epithelial cell number increases significantly. In addition to increase in number, pulmonary macrophage size can range from 8 to 30 mm or larger, while BAL fluid lymphocytes can be larger than their peripheral blood counterparts depending on the condition of the lung and especially if they are activated.

Sepsis is defined as life-threatening organ dysfunction due to dysregulated host response to infection. The consensus document describes organ dysfunction as an acute increase in total Sequential Organ Failure Assessment (SOFA) score two points consequently to the infection. *See, e.g.,* Gul et al., Turk J Anaesthesiol Reanim. 2017 Jun; 45(3): 129-138]. Septic shock occurs in a subset of patients with sepsis and includes an underlying circulatory and cellular/metabolic abnormality that is associated with increased mortality. Septic shock is defined by persisting hypotension requiring vasopressors to maintain a mean arterial blood pressure of 65 mm Hg or higher and a serum lactate level greater than 2 mmol/L (18 mg/dL) despite adequate volume resuscitation. *See, e.g.,* Singer et al., JAMA 2016; 315(8): 801-810. This definition, also called Sepsis-3, eliminates the requirement for the presence of systemic inflammatory response syndrome (SIRS) to define sepsis, and it removed the severe sepsis definition. What was previously called severe sepsis is now the new definition of sepsis. Severe sepsis is the most common precipitating cause of ALI worldwide. *See*, *e.g.,* Leonard D. Hudson, Arthur S. Slutsky, in Goldman's Cecil Medicine (Twenty Fourth Edition), 2012. Inflammation due to sepsis leads to ALI and therefore the progression to ALI must be mitigated as early as possible since no treatment is available for ALI other than supportive care. Endotoxin, or more accurately termed bacterial lipopolysaccharide (LPS), is recognized as the most potent microbial mediator implicated in the pathogenesis of sepsis and septic shock. *See, e.g.,* Opal SM. Contrib Nephrol. 2010;167:14-24. Therefore, the use of LPS to simulate sepsis in animal model is widely used in testing the effectiveness of treatment prior to human use. Because sepsis is known to be associated with a drop in blood pressure and natriuretic peptides are generally known to drop pressure it is counterintuitive to use that as a treatment. Yet, as discussed above, the present disclosure features the use of C-type natriuretic peptide (CNP) derivative, long acting CNP, long acting CNP derivative, very long acting CNP, very long acting CNP derivative, long acting NPRB agonist, and/or very long acting NPRB agonist, to treat sepsis, with beneficial effects.

Bacteremia is the presence of bacteria in the bloodstream and is known to those skilled in the art. If bacteria in the blood stream are present long enough time and in large enough numbers, particularly in people who have a weakened immune system, bacteremia can lead to other infections and sometimes trigger a serious body wide response called sepsis. Bacteremia may result from ordinary activities (such as vigorous toothbrushing), dental or medical procedures, or from infections (such as pneumonia or a urinary tract infection). Usually bacteremia (particularly if it occurs during ordinary activities) does not cause infections because bacteria typically are present only in small numbers and are rapidly removed from the bloodstream by the immune system.

Acute respiratory distress syndrome (ARDS) is a more severe form of ALI. It is a rapidly progressive disease occurring in critically ill patients, where fluid leaks into the lungs reaches the point where breathing is difficult or impossible.

Pulmonary edema is a condition where there is by excess fluid in the lungs arising from the lung itself and is defined and diagnosed by radiographical presence (by X-ray) of bilateral infiltrates ("infiltrates" term used by doctors and those skilled in the arts who reads chest X-rays) equivalent to pulmonary edema. This fluid collects in the numerous air sacs in the lungs, making it difficult to breathe. radiographical presence (by X-ray) of bilateral infiltrates ("infiltrates" is a term used by doctors reading chest X-rays) consistent with pulmonary edema. In most cases, heart problems cause pulmonary edema. But fluid can accumulate for other reasons, including pneumonia, exposure to certain toxins and medications, trauma to the chest wall, and visiting or exercising at high elevations. Pulmonary edema that develops suddenly (acute pulmonary edema) is a medical emergency requiring immediate care. Pulmonary edema can sometimes be fatal, but the outlook improves if it is treated quickly. Treatment for pulmonary edema varies depending on the cause but generally includes supplemental oxygen and medications.

Low arterial blood oxygenation or hypoxemia is a below-normal level of oxygen in blood with an oxygen partial pressure below 60mm Hg or pulse oximeter readings values below 90 percent. Normal arterial oxygen partial pressure is approximately 75 to 100 millimeters of mercury (mm Hg) or 10.5 to 13.5 kilopascal (kPa). Normal pulse oximeter readings usually range from 95 to 100 percent reflecting blood hemoglobin saturation. Hypoxemia is a sign of a problem that results in various symptoms, such as shortness of breath.

Elevated level of inflammatory cells in the lungs refers to an increase level or quantity or number of inflammatory cells (macrophages, neutrophils, eosinophils, and lymphocytes) in the lung by at least 3-fold compared to that of normal level healthy control subjects measured in the same manner. The level can be up to 10-fold compared to the normal healthy control subject. This level is determined from bronchoalveolar lavage fluid (BALF) by flow cytometric examination. The major cell populations in BALF include normal size macrophages, neutrophils, eosinophils, erythrocytes, and lymphocytes. During inflammation and ALI, the number and size (8-30 um or larger) of pulmonary macrophage increases along with significant increase in epithelial cell number. Similarly, lymphocytes can be larger than their peripheral blood counterparts depending on the condition of the lung and especially if they are activated.

In some embodiments, administering to the subject a therapeutically effective bolus dose of the composition decreases a total number of cells and total proteins in a BALF sample from the subject. In certain embodiments, administering to the subject a therapeutically effective bolus dose of the composition decreases MPO (an activated neutrophil marker) in a lung tissue from the subject. In certain embodiments, administering to the subject a therapeutically effective bolus dose of the composition attenuates (*i.e*., reduces, or decreases the expression of) inflammatory cytokine expression (*e.g*., IL-6, IL-1b, TNFα, MCP-1, and IFNg) in the subject. In certain embodiments, administering to the subject a therapeutically effective bolus dose of the composition decreases a fibrotic area in a lung in a subject having idiopathic pulmonary fibrosis. In certain embodiments, administering to the subject a therapeutically effective bolus dose of the composition decreases a fibrotic area in a lung, in a liver, or in a kidney. In some embodiments, administering to the subject a therapeutically effective bolus dose of the composition decreases cell numbers and protein levels, and attenuates (*i.e*., reduces, or decreases the expression of) any one of IL-6, IL-1b, TNFα, MCP-1, and IFNg or any combination thereof in a subject having idiopathic pulmonary fibrosis. In some embodiments, administering to the subject the therapeutically effective bolus dose of the composition decreases the expression of any one of AST, ALT, α-SMA, IL-6, IL-1b, TNFα, MCP-1, IFNg, iNOS, Elf-1, Tollip, IRAK-1, P-P38, P-P65, β-act, STAT1, P-STAT1, STAT2, STAT3, STAT6, a fibrotic area, serum creatinine, an albumin/creatinine ratio in urine, hydroxyproline in a lung, or any combination thereof, of the subject.

The following examples are provided to illustrate, not limit, the disclosure.

### EXAMPLES

All peptides used in the Examples were synthesized by solid phase peptide synthesis (SPPS) with H-Cys(Trt)-2-Cl-Trt Resin (0.54 mmol/g) as the starting solid support (BLDPharm, Shanghai, China) in an automated microwave peptide synthesizer (LibertyBlue HT12, CEM, Matthews, NC). Each constituent molecule of the peptide, such as amino acid, fatty acid, or protected alkyl dioic acid were anchored sequentially onto the peptide resin using Fmoc chemistry, which is known to a person of ordinary skill in the art, resulting in a linear protected peptide linked to the resin. Linear crude peptide was deprotected and released from the resin by acidolysis with trifluoroacetic acid in the presence of carbocation scavengers and ether precipitation. The resulting linear peptide was cyclized by dissolving in 10% DMSO and 20% acetonitrile aqueous solution and allowed to react for at least two days to provide disulfide bond formation. Finally, the peptide was purified and characterized by reversed phase HPLC (1260 Infinity II Preparative LC Systems, Santa Clara, CA) using a gradient of 10% acetonitrile in water with 0.1% trifluoroacetic acid (TFA) and acetonitrile with 0.1% TFA. This gradient was run on a Waters 30 x 150mm XBridge C18 column (P/N 186003284) with a Waters C18 prep column (P/N 186006893) at 40 mL/min over 24 minutes at room temperature and was acquired at 214 nm. The peptide fractions with >90% purity were collected and dried as white powder to provide the final peptide product.

### Example 1: Superior in vivo performance of long acting CNP compared to native CNP when administered as a bolus

All mice used for this study were maintained under a 12-hour light/12-hour dark cycle with free access to water and standard mouse diet (MF diet, Oriental Yeast Co., Ltd. Tokyo, Japan or PicoLab Rodent Diet 20, LabDiet Corp., St. Louis, Missouri).

For the pharmacokinetic study, female CD-1 mice (6-8 weeks old from Charles river laboratory) were treated with 2.0 mg/Kg of native human CNP (Chempep Inc. Wellington, FL), long acting CNP derivative (dCNP, Chempep Inc. Wellington, FL), or very long acting CNP derivative (VLA-dCNP) via subcutaneous administration between the shoulder blades. All test articles were formulated or dissolved in 100 mM sorbitol, 100 mM methionine, 20 mM histidine, pH 6.0. Blood sampling at various times (0, 0.5, 1, 2, 3, 4, 5, and 24 for native CNP; 0, 1, 2, 4, 8, 12, 24, 48, and 72 for dCNP and VLA-dCNP) was performed by retro-orbital bleed, two bleeding per animal at two different timepoints. Blood samples were processed in K₂EDTA tubes to obtain plasma. Plasma was analyzed by commercially available CNP ELISA kit from Phoenix Pharmaceuticals (cat# EKE-012-03). CNP is a native human CNP (GLSKGCFGLKLDRIGSMSGLGC [SEQ ID NO. 10] and dCNP is one of the addition derivatives of human CNP with the following sequence: CH₃(CH₂)₁₆C(=O)KKKKGGGGLSKGCFGLKLDRIGSMSGLGC [SEQ ID NO. 6]. The VLA-dCNP is a co-formulation of dCNP with PK extending polymer excipient at a dCNP: excipient weight ratio of 1:10. The details of the polymer are described in Castillo et al., Pharm. Res., (2012) 29(1); p 306-18, herein incorporated by reference in its entirety.

For pharmacodynamic study of cyclic-GMP response study, male C57BL/6J mice (6-week old from Kyudo; Saga, Japan) were treated with 1.0 mg/Kg of native human CNP, long acting CNP derivative (dCNP), and very long acting CNP derivative (VLA-dCNP) via subcutaneous bolus administration between the shoulder blades. All test articles were formulated or dissolved in 100 mM sucrose, 100 mM methionine, 50 mM histidine, pH 7.4. Blood sampling at various times (0, 1, 4, 8, 12, and 24 h for native CNP and dCNP; 0, 1, 2, 4, 8, 5, 24, and 48 h for dCNP and VLA-dCNP) was performed by abdominal aorta blood sampling after laparotomy, one bleeding per animal per timepoint. To obtain plasma, EDTA; final concentration 1.5 mg/mL (Dojindo, Kumamoto, Japan) and aprotinin; final concentration 500KIU/mL (Sigma Aldrich, St. Louis MO) were added to blood and centrifuged (x 2,000 g; 15 min, 4C). After supernatant was harvested, plasma samples were stored at -80 °C. Plasma samples were analyzed by commercially available cyclic-GMP kit from CisBio (Codolet, France). CNP is a native human CNP (GLSKGCFGLKLDRIGSMSGLGC [SEQ ID NO. 10]) and dCNP is one of the addition derivatives of human CNP with the following sequence: CH₃(CH₂)₁₆C(=O)KKKKGGGGLSKGCFGLKLDRIGSMSGLGC [SEQ ID NO. 6]. The VLA-dCNP is a co-formulation of dCNP with PK extending excipient at a dCNP: excipient weight ratio of 1:10. The details of the polymer are described in Castillo *et al.,* Pharm. Res., (2012) 29(1); p 306-18, herein incorporated by reference in its entirety. Specifically, the PK extending excipient was made using N-hydroxy sulfo-succinimide reagent and 1-Ethyl-3-(3-dimethylaminopropyl)carbodiimide to activate carboxyl group of polyetheylene glycol (PEG) for attachment to epsilon amino of linear polylysine ( at Epsilon amino: NHSS: EDC: PEG carboxyl group molar ratio of 0.2: 1: 1: 0.3) of 5kDa polyetheylene glycol (PEG) attached them to the epsilon amino group of the linear polylysine backbone with molecular weight ranging from 15 to 40kDa (polylysine average molecular weight of 25kDa, by multi-angle laser light scattering or MALLS). The product was characterized by trinitrobenzine sulfonic acid (TNBS) amino in process measurement. It is estimated to have 55% of epsilon amino group used up during the PEG addition reaction and the remaining epsilon amino groups was used up during the stearic acid addition reaction using NHS-stearic acid. Only trace amount of measurable amino groups (<5%) present at the end of the stearic acid addition as measured by TNBS. The PK extending excipient was purified by an ultrafiltration process that is well-known to those skilled in the art. Buffer formulation used for bolus administrations with and without PK extending excipient was 100 mM sucrose, 100 mM methionine, 50 mM histidine.

Referring to FIGURE 1A, the plasma CNP [mean (SD); n= 5] is shown for CD-1 mice after subcutaneous administration in an amount of 2.0 mg/Kg of a native CNP, a CNP derivative (dCNP), and a very long acting CNP derivative (VLA-dCNP). The inset is an enlarged scale of the left bottom comer to show the low plasma level of CNP (diamond) when native CNP is administered. Error bars represent standard deviation of n=5 plasma samples. Baseline CNP level prior to administration was 1.74 (0.6) ng/mL [mean (SD); n=15]. FIGURE 1B is a plot showing plasma cyclic-GMP in male C57BL/6J mice measured using a cyclic-GMP kit from CisBio (Codolet, France) after subcutaneous administration in an amount of 1.0mg/Kg of native CNP, long acting CNP derivative (dCNP), and very long acting CNP derivative (VLA-dCNP). Baseline plasma cyclic-GMP level was 20 (3.7) pmol/mL [mean (SEM); n=8] or 7 (1.3) ng/mL [mean (SEM); n=8]. At 2 hours and beyond, subcutaneous administration of native CNP did not show significant elevation of plasma cyclic-GMP compared to the baseline, while similar administration of long acting CNPs (dCNP and VLA-dCNP) showed significant elevation of cyclic-GMP for at least 24 hours.

### Example 2: Bolus administration of high dose of very long acting CNP derivative (VLA-dCNP) can increase plasma cyclic-GMP in the surprising absence of corresponding drop in blood pressure

For this study the cardiovascular and hemodynamic effects were assessed for three different long acting natriuretic peptides (very long acting ANP derivatives or VLA-dANP; ANP modified in a similar way as dCNP, where VLA-ANP was CH₃(CH₂)₁₆C(=O)KKKKGGG-SLRRSSCFGGRMDRIGAQSGLGCNSFRY [SEQ ID NO. 28] plus PK extending excipient and dANP was CH₃(CH₂)₁₆C(=O)KKKKGGG-SLRRSSCFGGRMDRIGAQSGLGCNSFRY [SEQ ID NO. 28] alone. The PK extending excipient was a polymeric excipient described in Example 1 above, and in Castillo et al., Pharm. Res., (2012) 29(1); p 306-18, and herein incorporated by reference in its entirety. The very long action BNP derivatives or VLA-dBNP was CH₃(CH₂)₁₆C(=O)KKKKGGG-SPKMVQGSGCFGRKMDRISSSSGLGCKVLRRH (dBNP) [SEQ ID NO. 29] plus PK extending excipient described above. dBNP, CH₃(CH₂)₁₆C(=O)KKKKGGG-SPKMVQGSGCFGRKMDRISSSSGLGCKVLRRH [SEQ ID NO. 29] is without the PK extending excipient described above. VLA-dCNP is dCNP as described in Example 1 plus PK extending polymeric excipient described above. dCNP is as described in Example 1 and is without the PK extending excipient. These formulations (in 100 mM Sucrose, 100 mM methionine, 50 mM histidine buffer) were administered to Beagle dogs [n=12 animals/test article; the same animals were for other test articles after a washout period of at least a week]. These test articles were administered by a single subcutaneous injection containing 25 µg/Kg of peptide and 1 mg/Kg of PK extending polymer (2.5% loading). 12 animals were previously instrumented with Data Sciences International (St. Paul, MN) telemetry transmitters to continuously record heart rate, mean arterial pressure, systolic arterial pressure, diastolic arterial pressure, PR interval, QRS duration, QT interval and body temperature. All animals were monitored for 7 days after each dose. At 4, 6, 8, 16, 20, 24, 28, 32, 40, 48, 66, 78, 90, 102, 114, 126, 138, 150, 162, and 174 hours after each dose, a 3mL blood sample was taken in a K₃ EDTA collection tube and then stored on wet ice until spun in a refrigerated centrifuge. Plasma was harvested and treated with plasma preservation reagent (phosphoric acid in deionized water, 15:85, v/v). The samples were inverted several times and then frozen on dry ice. The samples were stored in a freezer (-80C) then shipped on dry ice for LC-MS analysis of cyclic-GMP.

All natriuretic peptides act by causing an increase in cytoplasmic cyclic-GMP generation which is believed to cause a corresponding drop in blood pressure. However, when the bolus doses of very long acting versions of 3 main natriuretic peptides were compared, it was surprisingly found that a high bolus dose (sufficient to increase blood cyclic-GMP for 3 days) of very long acting CNP derivative of the present disclosure could increase in plasma cyclic-GMP without causing a dangerous drop in blood pressure. While a similarly developed very long acting ANP and BNP derivative, when given as a bolus dose (enough to increase blood cyclic-GMP for 3 days), caused a significant drop in blood pressure. For very long acting ANP derivative, the blood pressure drop was as much as 45%, while for the very long acting BNP derivative, the blood pressure drop was as much as 20%. For all 3 long acting natriuretic peptide derivatives, the increase in cyclic-GMP was more than 1.5-fold and as much as 6-fold the baseline. The cyclic-GMP AUC are VLA-dANP 3,483 ng*h/mL, VLA-dBNP 2,585 ng*h/mL, VLA-dCNP 2,627 ng*h/mL.

FIGURE 2A shows the corresponding increase in plasma cyclic-GMP [mean (SEM); n=12] as monitored after a bolus administration of 25ug/Kg of very long acting CNP derivative (VLA-dCNP), very long acting BNP derivative (VLA-dBNP), and very long acting BNP derivative (VLA-dANP). Baseline plasma cyclic-GMP level was 8 (0.2) ng/mL [mean (SEM); n=12], a level which is similar to healthy human. *See, e.g.,* Igaki et al., Hypertens Res 1998; 21: 7-13. All very long acting formulations of natriuretic peptide increased cyclic-GMP above the baseline of 8ng/ml. The cyclic-GMP AUC values were VLA-dANP 3,483 ng*h/mL, VLA-dBNP 2,585 ng*h/mL, VLA-dCNP 2,627 ng*h/mL. The very long acting CNP derivative (VLA-dCNP) increased plasma cyclic-GMP for 3 days without an associated drop in blood pressure.

FIGURE 2B shows the mean arterial pressure in dogs [mean (SEM); n=12] as monitored after a bolus administration of 25ug/Kg of very long acting CNP derivative (VLA-dCNP), very long acting BNP derivative (VLA-dBNP), and very long acting BNP derivative (VLA-dANP). VLA-dCNP did not cause significant drop in blood pressure from baseline (0 hr) after administration at a very high dose. In comparison, other very long acting natriuretic peptides such as VLA-dBNP and VLA-dANP derivatives caused more than a 15% drop in blood pressure. This was especially true for VLA-dANP where a drop in blood pressure could be as much as 50% for similar increase in cyclic-GMP. The very long acting CNP derivative (VLA-dCNP) increased plasma cyclic-GMP for 3 days without an associated drop in blood pressure.

### Example 3: Bolus administration of very long acting CNP derivative suppress lung injury

Increased cells in bronchoalveolar lavage fluid (BALF), especially neutrophils are seen in ALI and ARDS. Therefore, the number of cells and total proteins in (Fig 3) and MPO (Fig 4) which is a neutrophil marker are measured. The decrease in number of MPO positive cells (neutrophils) and total proteins indicates resolution of ALI/ARDS. Male C57BL/6J mice (6 week) were purchased from Kyudo (Saga, Japan) and maintained under a 12-hour light/12-hour dark cycle with free access to water and standard mouse diet (MF diet, Oriental Yeast Co., Ltd. Tokyo, Japan). Mice were treated with lipopolysaccharide (LPS) (Sigma-Aldrich; 0.05 mg/kg intratracheal administration) and treated with various test articles. Test articles were very long acting CNP derivative or VLA-dCNP (described in Example 1) (Low (L) 0.1 mg/kg s.c.; Medium (M) 0.3 mg/kg s.c.; High (H) 1.0 mg/kg s.c.), native C-type natriuretic peptide or CNP (High 1.0 mg/kg s.c.), long acting CNP derivative or dCNP (described in Example 1) (High 1.0 mg/kg s.c.), atrial natriuretic peptide (ANP) (High 1.0 mg/kg s.c.), B-type natriuretic peptide or BNP (High 1.0 mg/kg s.c.), anti-Tumor necrosis factor alpha antibody or TNFα ab which is an anti-inflammatory drug (clone XT3.11; BioXcell West Lebanon, NH) (1.0 mg/kg s.c.), and cyclic-GMP degradation inhibitor or PDE5 inhibitor called Vardenafil (VDN, Cayman Chemicals Ann Arbor, MI) (1.0 mg/kg s.c.). Test articles were administered right after LPS administration. Normal control (NC) without LPS treatment and LPS treated groups without test article treatment (Control) were included. After 24 hrs from treatment, mice were sacrificed under isoflurane anesthesia and then bronchoalveolar lavage fluid (BALF) was harvested. The total cell number in BALF was counted with counting chamber. Total protein concentration in BALF was measured with Pierce^{™} BCA Protein Assay Kit (Thermo Fisher Scientific). Statistical analysis was based on Dunnett's test performed by using GraphPad InStat 3 (n = 15, 23, 7, 7, 7, 7, 7, 7 and 9; NC, Control, CNP (H), dCNP (H), ANP (H), BNP (H),anti-TNFa ab, VDN, VLA-dCNP (H). * P < 0.01 vs VLA-dCNP (H)). Increased cells in BALF (especially neutrophils) are seen in ALI and ARDS. Therefore, the number of cells in BALF and MPO which is an activated neutrophil marker are measured.

FIGURE 3A a timeline for the protocol for evaluating dCNP-suppressed LPS-induced acute lung injury. FIGURE 3B shows an increase in cells in BALF, especially neutrophils, in ALI and ARDS, following the protocol shown in FIGURE 3A. The decrease in cells indicated resolution of ALI/ARDS. Statistical analysis was based on Dunnett's test performed by using GraphPad InStat 3 (n = 15, 23, 7, 7, 7, 7, 7, 7 and 9; NC, Control, CNP (H), dCNP (H), ANP (H), BNP (H), TNFα ab, VDN, VLA-dCNP (H). * P < 0.01 vs VLA-dCNP (H)). FIGURE 3C shows the total proteins in BALF, in ALI and ARDS, following the protocol shown in FIGURE 3A. The decrease in total proteins indicated resolution of ALI/ARDS. Statistical analysis was based on Dunnett's test performed by using GraphPad InStat 3 (n = 15, 23, 7, 7, 7, 7, 7, 7 and 9; NC, Control, CNP (H), dCNP (H), ANP (H), BNP (H), TNFα ab, VDN, VLA-dCNP (H). * P < 0.01 vs VLA-dCNP (H)).

### Example 4. Lung treatment using dCNP and VLA-dCNP

### A) dCNP and VLA-dCNP decreased neutrophil infiltration in the lung indicating resolution of ALI/ARDS

Male C57BL/6J mice (6 week) were purchased from Kyudo (Saga, Japan) and maintained under a 12-hour light/12-hour dark cycle with free access to water and standard mouse diet (MF diet, Oriental Yeast Co., Ltd. Tokyo, Japan). Mice were treated with LPS (Sigma-Aldrich; 0.05 mg/kg intratracheal administration) and treated with very long acting CNP derivative or VLA-dCNP (described in Example 1) (1.0 mg/kg s.c.), native C-type natriuretic peptide or CNP (1.0 mg/kg s.c.), CNP derivative or dCNP (described in Example 1) (1.0 mg/kg s.c.), Atrial Natriuretic peptide (ANP) (1.0 mg/kg s.c.), B-Type natriuretic peptide or BNP (1.0 mg/kg s.c.), anti-Tumor necrosis factor alpha antibody or TNFα ab (clone XT3.11; BioXcell West Lebanon, NH) (1.0 mg/kg s.c.), and cyclic-GMP degradation inhibitor or PDE5 inhibitor called Vardenafil (VDN) (Cayman Chemicals Ann Arbor, MI) (1.0 mg/kg s.c.). Test articles were administered right after LPS administration. After 24 hrs from treatment, mice were sacrificed under isoflurane anesthesia and lung tissue was harvested and fixed by 4% paraformaldehyde. Paraffin section of fixed lung tissue was stained immunohistochemically with anti-MPO rabbit polyclonal antibody (Agilent Technologies Santa Clara, CA), horseradish peroxidase (HRP)-labeled anti-rabbit IgG goat polyclonal antibody (Nichirei bioscience Inc. Tokyo, Japan) and 3,3'-diaminobenzidine-4HCl (DAB) (Agilent Technologies Santa Clara, CA). The number of MPO-positive cells per field were counted. Statistical analysis was based on Dunnett's test performed by using GraphPad InStat 3 (n = 18, 6, 6, 6, 6, 6, 6 and 6; Control, CNP, dCNP, ANP, BNP, anti-TNFα ab, VDN, VLA-dCNP. * P < 0.01 vs VLA-dCNP and ** P< 0.05 vs VLA-dCNP). Increased cells in BALF (especially neutrophils) are seen in ALI and ARDS. Therefore, the number of cells in BALF (FIGURE 3B) and MPO (FIGURE 4A) which is an activated neutrophil marker are measured. Inflammatory cell infiltration as seen by HE stains indicates inflammation in the lung.

FIGURE 4A shows that VLA-dCNP treatment decreased the number of MPO positive neutrophils., MPO is marker neutrophil granulocyte pro-inflammatory cell. Statistical analysis was based on Dunnett's test performed by using GraphPad InStat 3 (n = 18, 6, 6, 6, 6, 6, 6 and 6; Control, CNP, dCNP, ANP, BNP, TNFα ab, VDN, VLA-dCNP. * P < 0.01 vs VLA-dCNP and ** P< 0.05 vs VLA-dCNP).

### B) dCNP and VLA-dCNP decreased inflammatory cell infiltration or inflammation in the lung

Increased H&E staining indicates inflammatory cell infiltration or inflammation in the lung. Male C57BL/6J mice (6 week) were purchased from Kyudo (Saga, Japan) and maintained under a 12-hour light/12-hour dark cycle with free access to water and standard mouse diet (MF diet, Oriental Yeast Co., Ltd. Tokyo, Japan). Mice were treated with LPS (Sigma-Aldrich; 0.05 mg/kg intratracheal administration) and treated with very long acting CNP derivative or VLA-dCNP (described in Example 1) (1.0 mg/kg s.c.), native C-type natriuretic peptide or CNP (1.0 mg/kg s.c.), CNP derivative or dCNP (described in Example 1) (1.0 mg/kg s.c.), Atrial Natriuretic peptide (ANP) (1.0 mg/kg s.c.), B-Type natriuretic peptide or BNP (1.0 mg/kg s.c.), anti-Tumor necrosis factor alpha antibody or TNFα ab (1.0 mg/kg s.c.), and cyclic-GMP degradation inhibitor or PDE5 inhibitor called Vardenafil (VDN) (1.0 mg/kg s.c.). Test articles were administered right after LPS administration. After 24 hrs from treatment, mice were sacrificed under isoflurane anesthesia and lung tissue was harvested and fixed by 4% paraformaldehyde. Paraffin section of fixed lung tissue was stained by hematoxylin-eosin stain. Hematoxylin and eosin (H&E) stains are essential for recognizing various tissue types and the morphologic changes. The stain displays a broad range of cytoplasmic, nuclear, and extracellular matrix features. Hematoxylin stains cell nuclei blue, reflecting number of cells or multinucleated cells, whereas eosin stains protein pink in general and shows cytoplasmic- and extracellular matrix-protein. Increase H&E staining indicated inflammatory cell infiltration or inflammation in the lung.

FIGURE 4B shows micrographs of hematoxylin-eosin (HE) staining of paraffin-sections of lung tissue showing an increase in nucleated cells, number of cells, extracellular matrix and protein in general, scarring, and/or protein permeation in the alveolar space. Inflammatory cell infiltration as seen by HE stains indicated inflammation in the lung (right panel showing darker staining as cell numbers and protein increase). For these studies, mice were treated with LPS (Sigma-Aldrich; 0.05 mg/kg intratracheal administration) and treated with very long acting CNP derivative or VLA-dCNP (1.0 mg/kg s.c.), native C-type natriuretic peptide or CNP (1.0 mg/kg s.c.), CNP derivative or dCNP (1.0 mg/kg s.c.), Atrial natriuretic peptide (ANP) (1.0 mg/kg s.c.), B-Type natriuretic peptide or BNP (1.0 mg/kg s.c.), anti-tumor necrosis factor alpha antibody or TNFα ab (1.0 mg/kg s.c.), and cyclic-GMP degradation inhibitor or PDE5 inhibitor called Vardenafil (VDN) (1.0 mg/kg s.c.). Test articles were administered right after LPS administration. At 24 hours after treatment, mice were sacrificed under isoflurane anesthesia and lung tissue was harvested and fixed by 4% paraformaldehyde. Paraffin section of fixed lung tissue was stained by anti-MPO antibody (brown to dark brown color) and hematoxylin-eosin stain (nuclei is blue-purple and proteins are pink).

### Example 5: VLA-dCNP and dCNP treatment attenuated LPS-induced upregulation of inflammatory cytokines in BALF

Male C57BL/6J mice (6 week) were purchased from Kyudo (Saga, Japan) and maintained under a 12-hour light/12-hour dark cycle with free access to water and standard mouse diet (MF diet, Oriental Yeast Co, Ltd. Tokyo, Japan). Mice were treated with LPS (Sigma-Aldrich; 0.05 mg/kg intratracheal administration) and treated with very long acting CNP derivative or VLA-dCNP (described in Example 1) (1.0 mg/kg s.c.), native C-type natriuretic peptide or CNP (1.0 mg/kg s.c.), CNP derivative or dCNP (described in Example 1) (1.0 mg/kg s.c.), Atrial Natriuretic peptide (ANP) (1.0 mg/kg s.c.), B-Type natriuretic peptide or BNP (1.0 mg/kg s.c.), anti-Tumor necrosis factor alpha antibody or TNFα ab (1.0 mg/kg s.c.), and cyclic-GMP degradation inhibitor or PDE5 inhibitor called Vardenafil (VDN) (1.0 mg/kg s.c.). Test articles were administered right after LPS administration. After 24 hours from treatment, mice were sacrificed under isoflurane anesthesia and then bronchoalveolar lavage fluid (BALF) was harvested. Each cytokine concentration, specifically interleukin-6 (IL-6), tumor necrosis factor a (TNF-a) and interleukin-1β (IL-1β) concentrations were measured with commercially available Time Resolution FRET Kits (Cisbio, Bedford MA). Macrophage chemoattractant protein-1 (MCP-1) was measured by using ELISA kit (R&D SYSTEMS, Minneapolis MN). Previous studies showed the role of TNFα (PLoS One, 2014 Jul22;9(7):e102967) and the elevation of TNFα, IL-6 in non-survivors (Chest, 1997:111:1306-21), and MCP-1 in the patients who developed ARDS/ALI (International Journal of Molecular Sciences, 2019:20 (9): 2218). Statistical analysis was based on Student's t-test performed by using GraphPad.

FIGURE 5A shows that VLA-dCNP and dCNP treatments attenuated LPS-induced upregulation of inflammatory cytokines (IL6) in BALF to facilitate resolution of ARDS/ALI. Male C57BL/6J mice (6 week) were treated with LPS (0.05 mg/kg intratracheal administration) and treated with very long acting CNP derivative or VLA-dCNP (1.0 mg/kg s.c.), native C-type natriuretic peptide or CNP (1.0 mg/kg s.c.), CNP derivative or dCNP (1.0 mg/kg s.c.), atrial natriuretic peptide (ANP) (1.0 mg/kg s.c.), B-Type natriuretic peptide or BNP (1.0 mg/kg s.c.), anti-Tumor necrosis factor alpha antibody or TNFα ab (1.0 mg/kg s.c.), and cyclic-GMP degradation inhibitor or PDE5 inhibitor called Vardenafil (VDN) (1.0 mg/kg s.c.). After 24 hours from treatment, bronchoalveolar lavage fluid (BALF) was harvested and IL-6 cytokines were measured. Statistical analysis was based on Student's t-test. (n = 15, 23, 7, 7, 7, 7, 7, 7 and 9; NC, Control, CNP, dCNP, ANP, BNP, TNFα ab, VDN, and VLA-dCNP. * P < 0.01 vs. VLA-dCNP and ** P< 0.05 vs. VLA-dCNP).

FIGURE 5B shows that VLA-dCNP and dCNP treatment attenuated LPS-induced up-regulation of inflammatory cytokines (TNFα) in BALF to facilitate resolution of ARDS/ALI. The protocol was the same as that described in FIGURE 5A, except that bronchoalveolar lavage fluid (BALF) was harvested and measured for TNFα cytokines.

FIGURE 5C shows that VLA-dCNP and dCNP treatment attenuated LPS-induced upregulation of inflammatory cytokines (MCP-1) in BALF to facilitate resolution of ARDS/ALI. The protocol was the same as that described in FIGURE 5A, with the exception that bronchoalveolar lavage fluid (BALF) was harvested and measured for MCP-1 cytokines.

### Example 6: VLA-dCNP treatment attenuated LPS-induced upregulation of inflammatory cytokines in lung tissue

Male C57BL/6J mice (6 week) were purchased from Kyudo (Saga, Japan) and maintained under a 12-hour light/12-hour dark cycle with free access to water and standard mouse diet (MF diet, Oriental Yeast Co. Ltd, Tokyo Japan). Mice were treated with LPS (Sigma-Aldrich; 0.05 mg/kg intratracheal administration) and treated with VLA-dCNP (1.0 mg/kg s.c.). VLA-dCNP (described in Example 1) was administered right after LPS administration. After 24 hours from treatment, mice were anesthetized with isoflurane before sacrifice. Protein in harvested lung tissue was extracted in cell-lysis buffer (50 mM Tris-HCl, 150 mM NaCl, 1% Triton X-100, 1 mM EDTA, 50 mM NaF, 30 mM Na₄P₂O₇) supplemented with 1 mM PMSF, 2 µg/ml aprotinin, and 1 mM pervanadate. Each cytokine concentration in extracted lung protein, Interleukin-6 (IL-6), Tumor Necrosis Factor a (TNF-a), interleukin-1β (IL-1β) and Macrophage chemoattractant protein-1 (MCP-1), was measured by using ELISA kits (R&D SYSTEMS, Minneapolis MN). Statistical analysis was based on Student's t-test performed by using GraphPad Prism 6.

FIGURES 6A-6D show that VLA-dCNP treatment attenuated LPS-induced upregulation of inflammatory cytokines in lung tissue to facilitate resolution of ARDS/ALI. Male C57BL/6J mice (6 week) were treated with LPS (0.05 mg/kg intratracheal administration) and treated with VLA-dCNP (1.0 mg/kg s.c.). 24 hours after treatment, lung tissue was harvested. Each cytokine concentration in extracted lung protein, interleukin-6 (IL-6) (FIGURE 6A), tumor necrosis factor a (TNF-α) (FIGURE 6B), interleukin-1β (IL-1β) (FIGURE 6C) and macrophage chemoattractant protein-1 (MCP-1) (FIGURE 6D), was measured by using ELISA kits. Statistical analysis was based on Student's t-test (n = 10, 10, 9; NC, Control, VLA-dCNP. * P < 0.05 vs Control).

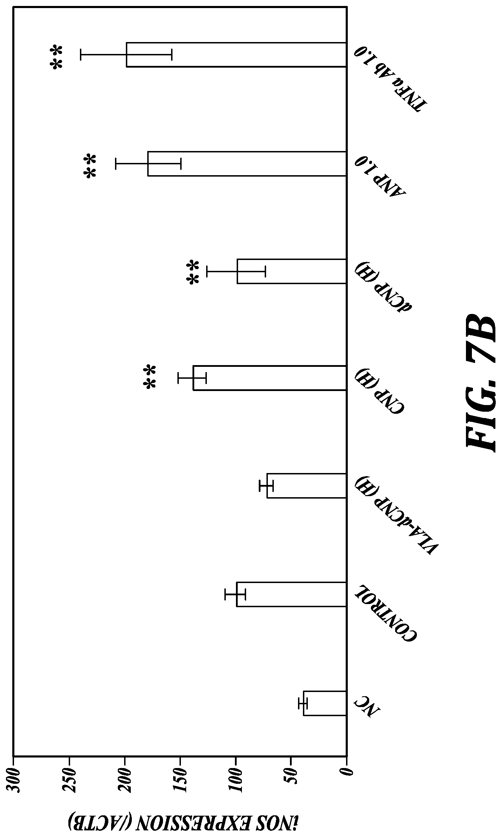

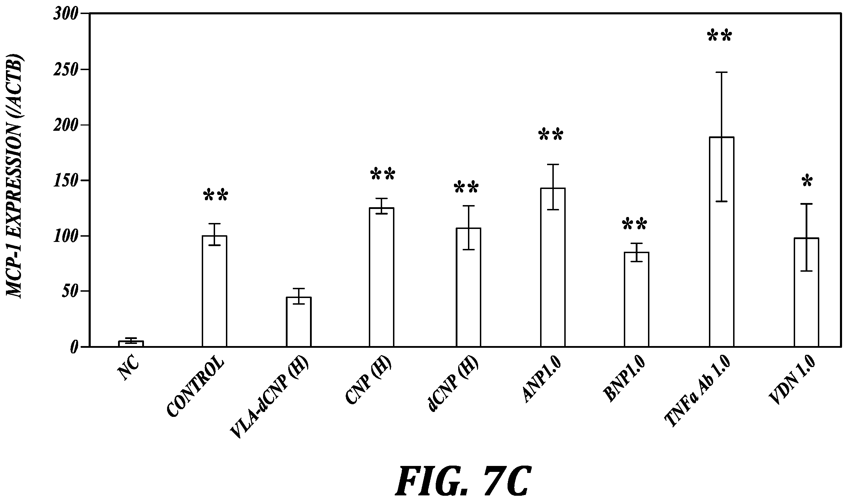

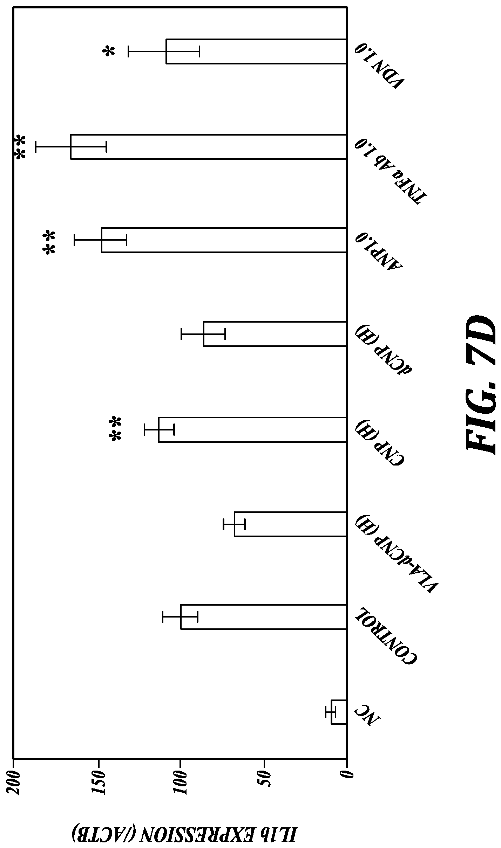

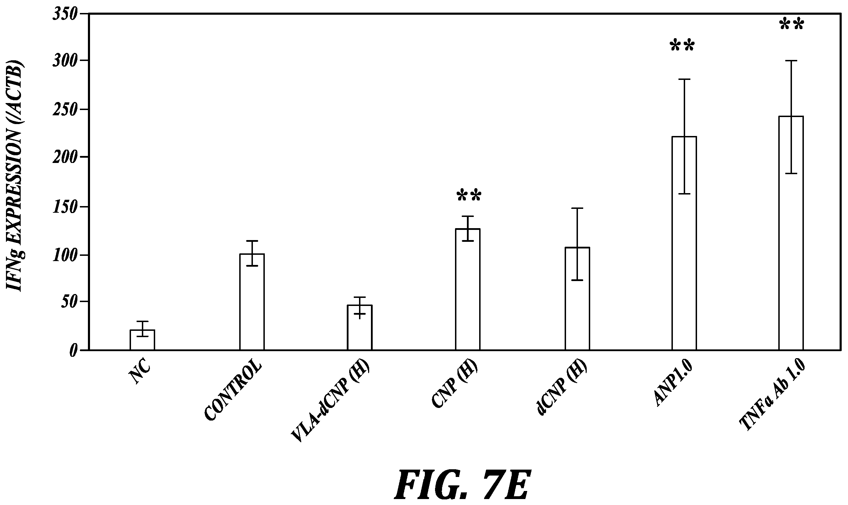

### Example 7: VLA-CNP attenuated LPS-elicited inflammatory cytokine expression including IL-6, TNFα, IL1b that are commonly regulated by NFkb systems, the master regulator of inflammation systems suggesting that VLA-dCNP broadly suppresses inflammation response in the subject's body to resolve ARDS/ALI (Fig 7)

Male C57BL/6J mice (6 week) were purchased from Kyudo (Saga, Japan) and maintained under a 12-hour light/12-hour dark cycle with free access to water and standard mouse diet (MF diet, Oriental Yeast Co. Ltd Tokyo, Japan). Mice were treated with LPS (Sigma-Aldrich; 0.05 mg/kg intratracheal administration) and treated with very long acting CNP derivative or VLA-dCNP (described in Example 1) (1.0 mg/kg s.c.), native C-type natriuretic peptide or CNP (1.0 mg/kg s.c.), CNP derivative or dCNP (described in Example 1) (1.0 mg/kg s.c.), atrial natriuretic peptide or ANP (1.0 mg/kg s.c.), B-Type natriuretic peptide or BNP (1.0 mg/kg s.c.), Tumor necrosis factor alpha antibody or TNFα ab (1.0 mg/kg s.c.), and cyclic-GMP degradation inhibitor or PDE5 inhibitor called Vardenafil (VDN) (1.0 mg/kg s.c.). Test articles were administered right after LPS administration. After 24 hours from treatment, mice were anesthetized with isoflurane before sacrifice, then lung tissue was harvested and shredded in TRI Reagent (Molecular Research Center, Inc. Cincinnati, OH) and kept at -80°C until analysis. Total RNA was extracted from harvested lung tissue by the chloroform-phenol method. Complementary DNA (cDNA) was synthesized from extracted mRNA with cDNA Kit (Qiagen, Hilden Germany). Quantitative RT- PCR analysis was performed by premix kit (Takara bio, Shiga Japan). Several studies indicated that using specific iNOS inhibitors and/or iNOS-knockout animals have supported the contention that NO/iNOS is responsible for the oxidative stress and endothelial damage in the ARDS/ALI caused by endotoxin (World Journal of Critical Care Medicine, 2012 1(2): 50-60). Statistical analysis was based on Student's t-test performed by using GraphPad.

FIGURE 7A shows that VLA-dCNP attenuated LPS-elicited inflammatory cytokine expression including IL-6 that is commonly regulated by NFkb systems, the master regulator of inflammation systems suggesting that VLA-dCNP broadly suppressed inflammation response in the subject's body to facilitate resolution of ARDS/ALI. Measurement of inflammatory related gene expression in ALI lung tissue. Male C57BL/6J mice (6 week) were treated with LPS (0.05 mg/kg intratracheal administration) and then treated with very long acting CNP derivative or VLA-dCNP (1.0 mg/kg s.c.), native C-type natriuretic peptide or CNP (1.0 mg/kg s.c.), CNP derivative or dCNP (1.0 mg/kg s.c.), atrial natriuretic peptide or ANP (1.0 mg/kg s.c.), B-Type natriuretic peptide or BNP (1.0 mg/kg s.c.), tumor necrosis factor alpha antibody or TNFα ab (1.0 mg/kg s.c.), and cyclic-GMP degradation inhibitor or PDE5 inhibitor called Vardenafil (VDN) (1.0 mg/kg s.c.). After 24 hours from the treatment, lung tissue was harvested. Total RNA was extracted from harvested lung tissue. Statistical analysis was based on Student's t-test. (n = 15, 22, 6, 6, 6, 6, 6, 5 and 9; NC, Control, CNP, dCNP, ANP, BNP, TNFα ab, VDN, and VLA-dCNP. * P < 0.01 vs. VLA-dCNP and ** P< 0.05 vs. VLA-dCNP).

FIGURE 7B shows that VLA-dCNP attenuated LPS-elicited inflammatory cytokine expression including iNOS, suggesting that VLA-dCNP broadly suppressed inflammation response in a subject to facilitate resolution of ARDS/ALI. The protocol was as described for FIGURE 7A.

Referring to FIGURE 7C, VLA-dCNP attenuated LPS-elicited inflammatory cytokine expression including MCP-1, suggesting that VLA-dCNP broadly suppresses inflammation response in the subject's body to facilitate resolution of ARDS/ALI. The protocol was as described for FIGURE 7A.

Referring to FIGURE 7D, VLA-dCNP attenuated LPS-elicited inflammatory cytokine expression including IL1b, suggesting that VLA-dCNP broadly suppressed inflammation response in the subject's body to facilitate resolution of ARDS/ALI. The protocol was as described for FIGURE 7A.

Referring to FIGURE 7E is a bar graph showing that VLA-dCNP attenuated LPS-elicited inflammatory cytokine expression including IFNg, suggesting that VLA-dCNP broadly suppressed inflammation response in the subject's body to facilitate resolution of ARDS/ALI. The protocol was as described for FIGURE 7A.

### Example 8: VLA-dCNP suppressed inflammation levels in lung tissue

Tollip is the negative regulator of TLR-dependent inflammatory pathway. This data indicated that VLA-dCNP (described in Example 1) upregulate the negative regulator of TLR-dependent inflammatory pathway and that may contribute to the anti-inflammatory effect of that compound (Journal of Biological Chemistry, 2002; 227:7059-7065). IRAK1, P-P38, and P-P65 were measured and are the well-known crucial mediators in toll like receptor 4 (TLR-4) dependent inflammatory pathway that is essential in lipopolysaccharide (LPS) induced ALI. TLR-4 is the receptor for LPS and plays the crucial effect in LPS-induced inflammation response including ALI and sepsis. Tollip is a built-in negative regulator that can attenuate TLR4-dependent signaling and ELF-1 suppresses Tollip expression in the cell. If Elf-1 were down-regulated, the Tollip expression could be up-regulated and that may suppress LPS-elicited inflammation. Male C57BL/6J mice (6 week) were purchased from Kyudo (Saga, Japan) and maintained under a 12-hour light/12-hour dark cycle with free access to water and standard mouse diet (MF diet, Oriental Yeast Co. Ltd, Tokyo Japan). Mice were treated with LPS (Sigma-Aldrich; 0.05 mg/kg intratracheal administration) and treated with very long acting CNP derivative or VLA-dCNP (1.0 mg/kg s.c.). VLA-dCNP was administered right after LPS administration. After 24 hours from treatment, mice were anesthetized with isoflurane before sacrifice. Lung tissue were lysed in cell lysis buffer (50 mM Tris-HCl, 150 mM NaCl, 1% Triton X-100, 1 mM EDTA, 50 mM NaF, 30 mM Na₄P₂O₇) supplemented with 1 mM PMSF, 2 µg/ml aprotinin, and 1 mM pervanadate and samples were added with 2-mercaptoethanol (Fujifilm, Tokyo Japan) and sodium dodecyl sulfate (SDS) solution and then boiled. Western blot analysis was performed by using SDS-gel (Bio-rad, Hercules CA) and PVDF membrane (Merck Millipore, Burlington MA). After blocking step by 2.5% BSA, membranes were detected by using antibody against Elf-1 (Santa Cruz Biotechnology, Dallas Texas), Tollip (Protein Tech, Tokyo Japan), IRAK-1 (Cell Signaling technology, Danvers, MA), P-P38 (Cell Signaling technology, Danvers, MA), P-P65 (Santa Cruz Biotechnology, Dallas Texas) and β-actin (Sigma-Aldrich, St. Louis MO) followed by incubation with a secondary antibody (Abcam, Cambridge, UK) and washed with 1% Tween TBS. Membranes were detected by image analyzer (Vilber Lourmat, Collegien France). Statistical analysis was based on Student's t-test performed by using GraphPad Prism 6.

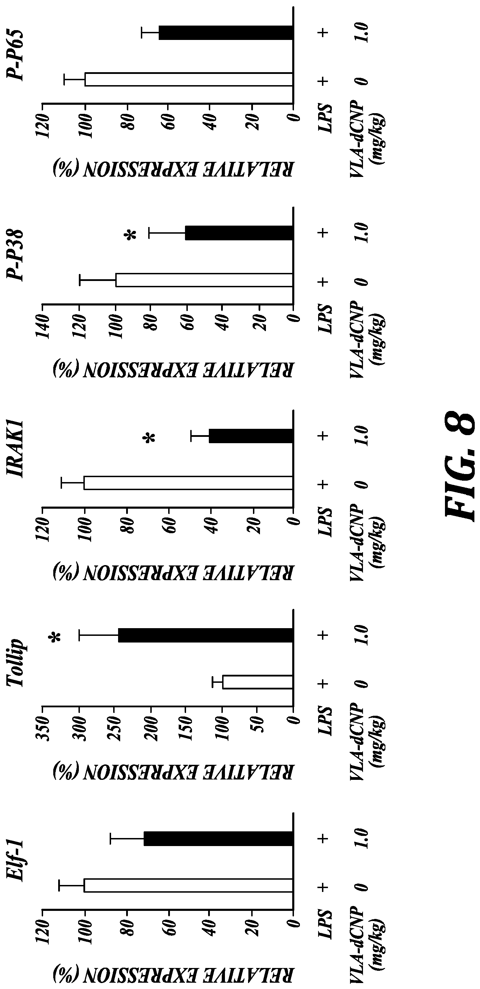

Referring to FIGURE 8, VLA-dCNP suppressed inflammation levels in lung tissue to facilitate resolution of ARDS/ALI. Male C57BL/6J mice (6 week) were treated with LPS (0.05 mg/kg intratracheal administration) and treated with VLA-dCNP (1.0 mg/kg s.c.). At 24 hours after treatment, lung tissue was harvested. Western blot analysis was performed by using antibody against Elf-1, Tollip, IRAK-1, P-P38, P-P65 and β-actin (internal standard). Statistical analysis was based on Student's t-test (n = 5, * P < 0.05 vs Cont.).

### Example 9: VLA-dCNP suppressed STAT levels in lung tissue indicating attenuation of inflammation

STAT1, P-STAT1, STAT2, STAT3 are also involved in iNOS expression that is contributed to ARDS/ALI. Several studies indicated that using specific iNOS inhibitors and/or iNOS-knockout animals have supported the contention that NO/iNOS is responsible for the oxidative stress and endothelial damage in the ARDS/ALI caused by endotoxin (World Journal of Critical Care Medicine, 2012 1(2): 50-60). In addition, STAT6 deficient mice exhibit attenuation of airway inflammation (Journal of Immunology, 2013, 190:904-912). Male C57BL/6J mice (6 week) were purchased from Kyudo (Saga, Japan) and maintained under a 12-hour light/12-hour dark cycle with free access to water and standard mouse diet (MF diet, Oriental Yeast Co. Ltd, Tokyo Japan). Mice were treated with LPS (Sigma-Aldrich; 0.05 mg/kg intratracheal administration) and treated with VLA-dCNP (described in Example 1) (1.0 mg/kg s.c.). VLA-dCNP was administered right after LPS administration. After 24 hours from treatment, mice were anesthetized with isoflurane before sacrifice. Lung tissue were lysed in cell lysis buffer (50 mM Tris-HCl, 150 mM NaCl, 1% Triton X-100, 1 mM EDTA, 50 mM NaF, 30 mM Na₄P₂O₇) supplemented with 1 mM PMSF, 2 µg/ml aprotinin, and 1 mM pervanadate and samples were added with 2-mercaptoethanol (Fujifilm, Tokyo Japan) and sodium dodecyl sulfate solution and then boiled. Western blot analysis was performed by using SDS-gel (Bio-Rad, Hercules CA) and PVDF membrane (Merck Millipore, Burlington MA). After blocking step by 2.5% BSA, membranes were detected by using antibody against STAT-1 (Cell signaling Technology (CST), Danvers MA), P-STAT-1 (CST), STAT-2 (CST), STAT-3 (CST), STAT-6 (CST) and β-actin (Sigma-Aldrich, St. Louise MO) followed by incubation with a secondary antibody (Abcam, Cambridge UK) and washed with 1% Tween TBS. Membranes were detected by image analyzer (Vilber Lourmat, Collegien France). Statistical analysis was based on Student's t-test performed by using GraphPad Prism 6.

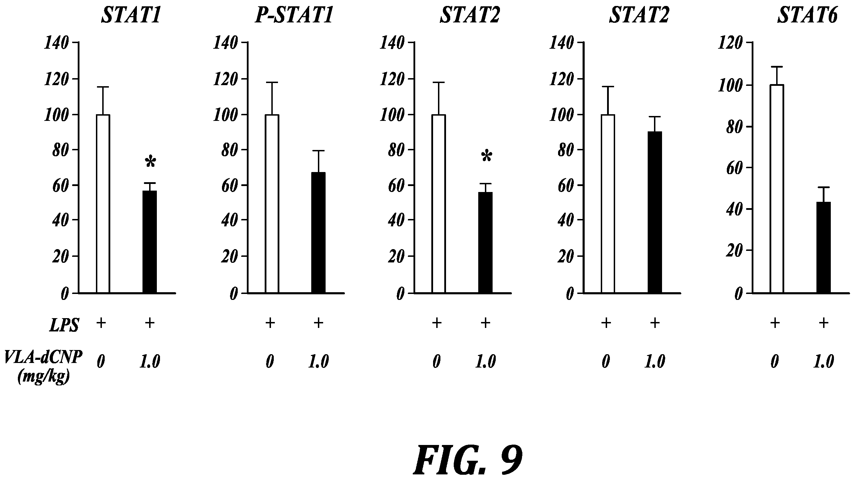

Referring to FIGURE 9, VLA-dCNP suppressed STAT levels in lung tissue to facilitate resolution of ARDS/ALI. Male C57BL/6J mice (6 week) were treated with LPS (0.05 mg/kg intratracheal administration) and treated with VLA-dCNP (1.0 mg/kg s.c.). At 24 h after treatment, lung tissue was harvested. Western blot analysis was performed by using antibody anti-STAT-1, P-STAT-1, STAT-2, STAT-3, STAT-6 and β-actin (internal standard). Statistical analysis was based on Student's t-test performed (n = 5, * P < 0.05 vs Control).

### Example 10. VLA-dCNP suppressed Elf-1 expression in human umbilical vein endothelial cells indicating suppression of TLRs-dependent inflammation including LPS or of TLR-dependent Damage-associated molecular patterns (DAMPs)/Pathogen-associated molecular patterns (PAMPs)-elicited inflammation

Toll like receptor (TLR4) is the receptor for Lipopolysaccharide (LPS) and plays the crucial effect in LPS-induced inflammation response including ALI and Sepsis. Tollip is a built-in negative regulator that can attenuate TLR4-dependent signaling and ELF-1 suppresses Tollip expression in the cell. If Elf-1 were down-regulated, the Tollip expression could be up-regulated and that can suppress LPS-elicited inflammation.

Human umbilical vein endothelial cells (HUVECs) was purchased from Takara Bio (Shiga, Japan). Cells were maintained in HuMedia-EG2 medium purchased from Kurabo. Cells were plated into 12-well plates (Nunc, Roskilde Denmark) at the density of 1 X 10⁵ cells/well in 2 mL in HuMedia-EG2. After 24 hours, cells were treated with each concentration of VLA-dCNP (described in Example 1) in M199 (Thermo Fisher Scientific, Waltham MA) supplemented with 1%BSA (Sigma-Aldrich, St. Louis MO) for 6 hours. Cells were lysed in cell lysis buffer (50 mM Tris-HCl, 150 mM NaCl, 1% Triton X-100, 1 mM EDTA, 50 mM NaF, 30 mM Na₄P₂O₇) supplemented with 1 mM PMSF, 2 µg/ml aprotinin, and 1 mM pervanadate and samples were added with 2-mercaptoethanol and sodium dodecyl sulfate (SDS) solution and then boiled. Western blot analysis was performed by using SDS-gel (Bio-rad, Hercules CA) and PVDF membrane (Merck Millipore, Burlington MA). After blocking step by 2.5% BSA, membranes were detected by using an antibody against Elf-1 (Santa Cruz Biotechnology, Dallas TX) and β-actin (Sigma-Aldrich, St. Louis MO) followed by incubation with a secondary antibody (Abcam, Cambridge UK) and washed with 1% Tween TBS. Membranes were detected by image analyzer (Vilber Lourmat, Collegien France). Statistical analysis was based on Student's t-test performed by using GraphPad Prism 6.

Referring to FIGURE 10, VLA-dCNP suppressed Elf-1 expression in human umbilical vein endothelial cells. Human umbilical vein endothelial cells (HUVECs) was maintained in HuMedia-EG2 and inoculated into 12 well plates (1 X 105 cells/well in 2 mL in HuMedia-EG2). After 24 hours, cells were treated with each concentration of VLA-dCNP (0.07uM (0.21 µg/mL) or 0.7uM (2.1 µg/mL)) (in M199 1%BSA) for 6 hours. Protein levels were assessed by western blot analysis by using an antibody against Elf-1 and β-actin (internal standard). Statistical analysis was based on Student's t-test (n = 4, * P < 0.05 vs Control).

### Example 11. VLA-dCNP suppressed Elf-1 levels in nuclei of human umbilical vein endothelial cells

Human umbilical vein endothelial cells (HUVECs) was purchased from Takara Bio (Shiga Japan). Cells were maintained in HuMedia-EG2 medium purchased from Kurabo (Osaka Japan). Cells were plated into grass bottom dish at the density of 1 X 10⁵ cells/well in 2 mL in HuMedia-EG2. After 24 hours, cells were treated with each concentration of VLA-dCNP (described in Example 1) in M199 (Thermo Fisher Scientific, Waltham MA) supplemented with 1%BSA (Sigma-Aldrich, St. Louis MO) for 6 hours. Cells were fixed by 4% paraformaldehyde (Fujifilm, Tokyo Japan) and treated with anti-Elf-1 Ab (Santa Cruz Biotechnology, Dallas TX) followed by incubation with Alexa Fluor 488 labeled-secondary antibody (Thermo Fisher Scientific, Waltham MA) and Hoechst 33342. Pictures were taken by florescence microscope (Keyence, Osaka Japan). The overlay the green (Elf-1) and blue (nuclei) and the mean fluorescence intensity of green at blue were evaluated. Statistical analysis was based on Student's t-test performed by using GraphPad Prism 6.

Referring to FIGURE 11, VLA-dCNP suppressed Elf-1 levels in nuclei of human umbilical vein endothelial cells. Human umbilical vein endothelial cells (HUVECs) was maintained in HuMedia-EG2. Cells were plated into grass bottom dish at the density of 1 X 10⁵ cells/well in 2 mL in HuMedia-EG2. After 24 hours, cells were treated with each concentration of VLA-dCNP (0.07uM (0.21 µg/mL)) or CNP 0.1µM (0.21µg/mL)) in M199 (Thermo Fisher Scientific, Waltham MA) supplemented with 1%BSA (Sigma-Aldrich, St. Louis MO) for 6 hours. Cells were fixed by 4% paraformaldehyde and treated with an antibody against Elf-1 Ab (Santa Cruz Biotechnology, Dallas TX) followed by incubation with Alexa Fluor 488 labeled-secondary antibody (Thermo Fisher Scientific, Waltham MA) and Hoechst 33342.

### Example 12 VLA-dCNP elicits Tollip expression in human lung fibroblast cell line HFL1

Tollip is the negative regulator of TLR-dependent inflammatory pathway. This data indicated that VLA-dCNP (described in Example 1) upregulate the negative regulator of TLR-dependent inflammatory pathway and that may contribute to the anti-inflammatory effect in vivo. Human lung fibroblast cell line HFL1 was purchased from ATCC (Old Town Manassas, VA). Cells were maintained in Dulbecco's Modified Eagle's Medium (Fujifilm, Tokyo Japan) supplemented with 10% fetal bovine serum (FBS) purchased from (Sigma Aldrich, St. Louis MO). Cells were plated into 12-well plates (Nunc, Roskilde Denmark) at the density of 1 X 10⁵ cells/well in 2 mL in 10% FBS DMEM. After 16 hours, cells were treated with each concentration of VLA-dCNP in M199 (Thermo Fisher Scientific, Waltham MA) supplemented with 1%BSA (Sigma-Aldrich, St. Louis MO) for 12 hours and added LPS (final concentration of 1.0 µg/mL) for 2 hours. Cells were lysed in cell lysis buffer (50 mM Tris-HCl, 150 mM NaCl, 1% Triton X-100, 1 mM EDTA, 50 mM NaF, 30 mM Na₄P₂O₇) supplemented with 1 mM PMSF, 2 µg/ml aprotinin, and 1 mM pervanadate) and samples were added with 2-mercaptoethanol and sodium dodecyl sulfate (SDS) solution and then boiled. Western blot analysis was performed by using SDS-gel (Bio-rad Hercules CA) and PVDF membrane (Merck millipore Burlington MA). After blocking step by 2.5% BSA, membranes were detected by using an antibody against Tollip (Proteintech) and β-actin (Sigma-Aldrich, St. Louis MO) followed by incubation with a secondary antibody (Abcam, Cambridge UK) and washed with 1% Tween TBS. Membranes were detected by image analyzer (Vilber Lourmat, Collegien France). Statistical analysis was based on Student's t-test performed by using GraphPad Prism 6.

Referring to FIGURE 12, VLA-dCNP elicits Tollip expression in human lung fibroblast cell line HFL1. Human lung fibroblast HFL1 (1.0 x 105 cells /well) was cultured with DMEM medium for 16 hours and then incubated with 1% BSA-M199 medium with 0.21 µM (0.66 ug/mL) VLA-dCNP and without VLA-dCNP (N.C.). After a 12-hour incubation, cells were stimulated with LPS (final concentration of 1.0 µg/mL). After another 2-hour incubation, cells were harvested and lysed. The amount of protein expression in the cells were evaluated by western blotting with an antibody against Tollip and β-actin (internal standard). Statistical analysis was based on Student's t-test (n = 4, * P < 0.05 vs Cont.).

### Example 13: VLA-dCNP had protective effect on the lethality of LPS-induced sepsis (Table 1)

Male Balb/c mice (11 week) were purchased from Kyudo (Saga, Japan) and maintained under a 12-hour light/12-hour dark cycle with free access to water and standard mouse diet (MF diet, Oriental Yeast Co., Ltd. Tokyo, Japan). Mice were treated with LPS (Sigma-Aldrich 10 mg/kg i.p) and treated with each dose of VLA-dCNP (described in Example 1) (Low 0.1 mg/kg s.c.; Medium 0.3 mg/kg s.c.; High 1.0 mg/kg s.c.), native C-type natriuretic peptide or CNP (High 1.0 mg/kg s.c.), CNP derivative or dCNP (High 1.0 mg/kg s.c.), B-Type natriuretic peptide or BNP (High 1.0 mg/kg s.c.), Anti-Tumor necrosis factor alpha antibody or TNFα ab (1.0 mg/kg s.c.), and cyclic-GMP degradation inhibitor or PDE5 inhibitor called vardenafil (VDN) (1.0 mg/kg s.c.). Test articles were administered right after LPS administration. Survival was observed every 2 hours.

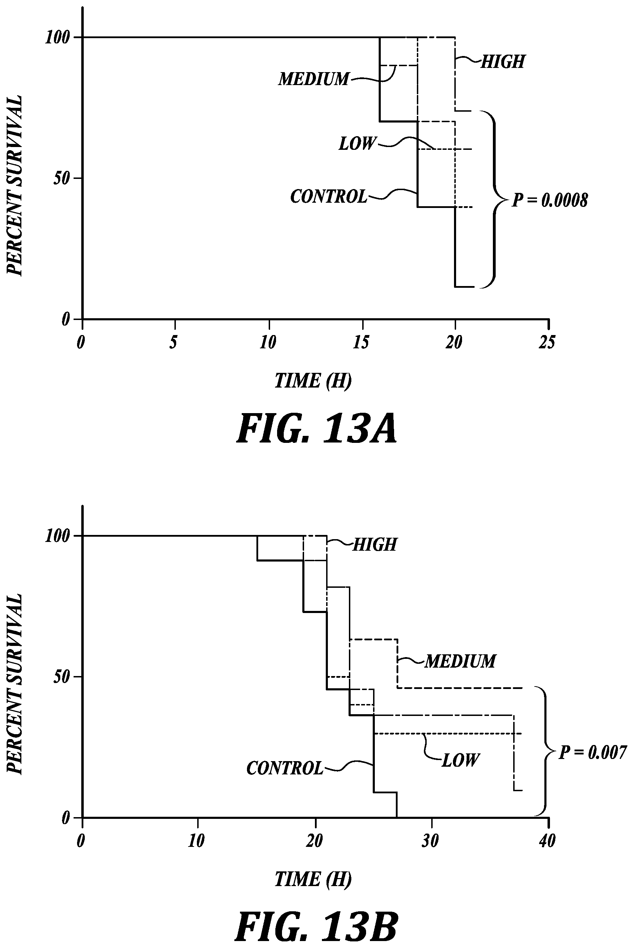

Referring to FIGURE 13A, VLA-dCNP had protective effect on LPS-induced sepsis. Balb/c (11 week-old male) mice were treated with LPS (10 mg/kg i.p.) and treated with each dose of VLA-dCNP (Low 0.1 mg/kg s.c.; Medium 0.3 mg/kg s.c.; High 1.0 mg/kg s.c.). Survival was observed every 2 hours. Statistical analysis was performed by Log rank test based on Graphpad Prism 6.0 (n = 10, 10, 10, 11).

Referring to FIGURE 13B, C57BL/6J (6 week-old male) mouse treated with LPS (15 mg/kg i.p.) and treated with a given dose of VLA-dCNP (Low 0.1 mg/Kg s.c.; Medium 0.3 mg/kg s.c.; High 1.0 mg/Kg s.c.). Survival was observed every 2 hours. Statistical analysis was performed by Log rank test. (n = 11, 10, 11, 11). VLA-dCNP had protective effect on LPS-induced sepsis.

Table 1. VLA-dCNP had protective effect on LPS-induced sepsis mortality. Shown is the % survival by hours. Balb/c (11-week male) mice were treated with LPS (10 mg/kg i.p.) and treated with each dose of VLA-dCNP (L; 0.1 mg/kg s.c.; M; 0.3 mg/kg s.c.; H; 1.0 mg/kg s.c.), native C-type natriuretic peptide or CNP (High 1.0 mg/kg s.c.), CNP derivative or dCNP (High 1.0 mg/kg s.c.), B-Type natriuretic peptide or BNP (High 1.0 mg/kg s.c.), Anti-Tumor necrosis factor alpha antibody or TNFα ab (1.0 mg/kg s.c.), and cyclic-GMP degradation inhibitor or PDE5 inhibitor called Vardenafil (VDN) (1.0 mg/kg s.c.). Survival was observed every 2 hours (FIGURES 13A and 13B). First mortality observed was in Italicized and bold and the last observation survival % was in bold in the table.

**Table 1. Percent animal survival over time showing VLA-dCNP had protective effect on LPS-induced sepsis.**

| | **Percent animal survival at various time after LPS administration** | | | | | | | | | |
|---|---|---|---|---|---|---|---|---|---|---|
| rs | Control (n=10) | H; CNP (n=10) | H; dCNP (n=10) | L; VLA-dCNP (n=10) | M; VLA-dCNP (n=10) | H; VLA-dCNP (n=11) | H; ANP (n=10) | H; BNP (n=10) | TNFα ab (n=10) | VDN (n=10) |
| 0 | 100 | 100 | 100 | 100 | 100 | 100 | 100 | 100 | 100 | 100 |
| 2 | 100 | 100 | 100 | 100 | 100 | 100 | 100 | 100 | 100 | 100 |
| 3 | ***50*** | ***50*** | 100 | 100 | 100 | 100 | 100 | 100 | 100 | 100 |
| 5 | 10 | 30 | ***50*** | 100 | 100 | 100 | ***90*** | 100 | 100 | 100 |
| 6 | 10 | 30 | 50 | 100 | 100 | 100 | 60 | ***60*** | ***70*** | ***60*** |
| 7 | 0 | 10 | 10 | 100 | ***90*** | 100 | 60 | 60 | 70 | 60 |
| 8 | 0 | 10 | 10 | 100 | 90 | 100 | 30 | 20 | 40 | 20 |
| 9 | 0 | 10 | 10 | ***60*** | 70 | 100 | 30 | 20 | 40 | 20 |
| 0 | 0 | 10 | 10 | 60 | 70 | 100 | 10 | 10 | 0 | 0 |
| **1** | **0** | **0** | **0** | **40** | **50** | ***73*** | **10** | **10** | **0** | **0** |

### Example 14. VLA-dCNP decreased fibrotic area in the lung indicating resolution of Idiopathic Pulmonary Fibrosis (IPF)

Male C57BL/6J mice (6 week) were purchased from Kyudo (Saga, Japan) and maintained under a 12-hour light/12-hour dark cycle with free access to water and standard mouse diet (MF diet; Oriental Yeast Co., Ltd. Tokyo, Japan). Mice were treated with bleomycin (Nippon Kayaku Tokyo, Japan; 1.0 mg/kg intratracheal administration). VLA-dCNP (described in Example 1) (0.3 or 0.1 mg/kg, 5 times/week, subcutaneous bolus administration) was administered from 7th day after bleomycin administration. After 21 day from bleomycin treatment, mice were sacrificed under isoflurane anesthesia and lung tissue was harvested and fixed by 4% paraformaldehyde (Fuji film, Tokyo, Japan). Paraffin section of fixed lung tissue was stained by Masson's Trichrome Stain reagent (Kyodo Byori, Kobe, Japan) (B). Masson's Trichrome Stain showed decrease in fibrotic area in the lung tissue. Fibrosis area was measured by using Image J (NIH, Bethesda, Maryland, USA) (A). Statistical analysis was based on Dunnett's multiple comparisons test performed by using GraphPad Prism 6 (GraphPad Software Inc. San Diego, CA, USA).

Referring to FIGURE 14A, VLA-dCNP decreased fibrotic area in the lung indicating resolution of idiopathic pulmonary fibrosis (IPF), or interstitial lung disease (ILD). Male C57BL/6J mice (6 week) were treated with bleomycin (1.0 mg/kg intratracheal administration) and treated with each dose of VLA-dCNP (0.1 mg/kg s.c. and 0.3 mg/kg s.c). VLA-dCNP was administered at 7^{th} day after bleomycin administration (5 times/week). At 21^{st} day, mice were sacrificed, and lung tissue was harvested and performed Masson's Trichrome staining. Statistical analysis was based on Dunnett's test performed by using GraphPad Prism 6. (n = 5, 8, 9, 7; Negative Control, Control, VLA-dCNP 0.1, and VLA-dCNP 0.3. * P < 0.05 vs Control.). FIGURE 14B show the Masson's trichrome stained lung tissue samples of FIGURE 14A.

### Example 15: VLA-dCNP decreased cell numbers and protein levels and attenuated TNFα and IL-6 in BALF from Acute exacerbations of idiopathic pulmonary fibrosis (IPF-AE) model

Considering IL-6 is upregulated in the patient with IPF-AE (American Journal of Physiology; Lung Cellular and Molecular Physiology, 2010 299: L3-L7) and TNFα showed a trend towards statistical significance in the patient with IPF-AE (PLoS One, 2015 10(1):e0116775). Together, VLA-dCNP has potential beneficial effect on the patient with IPF-AE. Male C57BL/6J mice (6 week) were purchased from Kyudo (Saga, Japan) and maintained under a 12-hour light/12-hour dark cycle with free access to water and standard mouse diet (MF diet; Oriental Yeast Co., Ltd. Tokyo, Japan). Mice were treated with bleomycin (Nippon Kayaku Tokyo Japan; 1.0 mg/kg intratracheal administration). After 3 weeks, mice were treated with LPS (0.05 mg/kg intratracheal administration Sigma Aldrich, St. St. Louis, MO, USA) and treated with each dose of VLA-dCNP (described in Example 1) (Medium 0.3 mg/kg s.c.; High 1.0 mg/kg subcutaneous bolus administration.). VLA-dCNP was administered right after LPS administration. After 24 hours from treatment, mice were sacrificed under isoflurane anesthesia and then bronchoalveolar lavage fluid (BALF) was harvested. The total cell number in BALF was counted with counting chamber. Total protein concentration in BALF was measured with PierceTM BCA Protein Assay Kit (Thermo Fisher Scientific). Each cytokine concentration Interleukin-6 (IL-6), Tissue Necrosis Factor a (TNF-a) was measured with commercially available Time Resolution FRET Kits (Cisbio, Bedford MA).

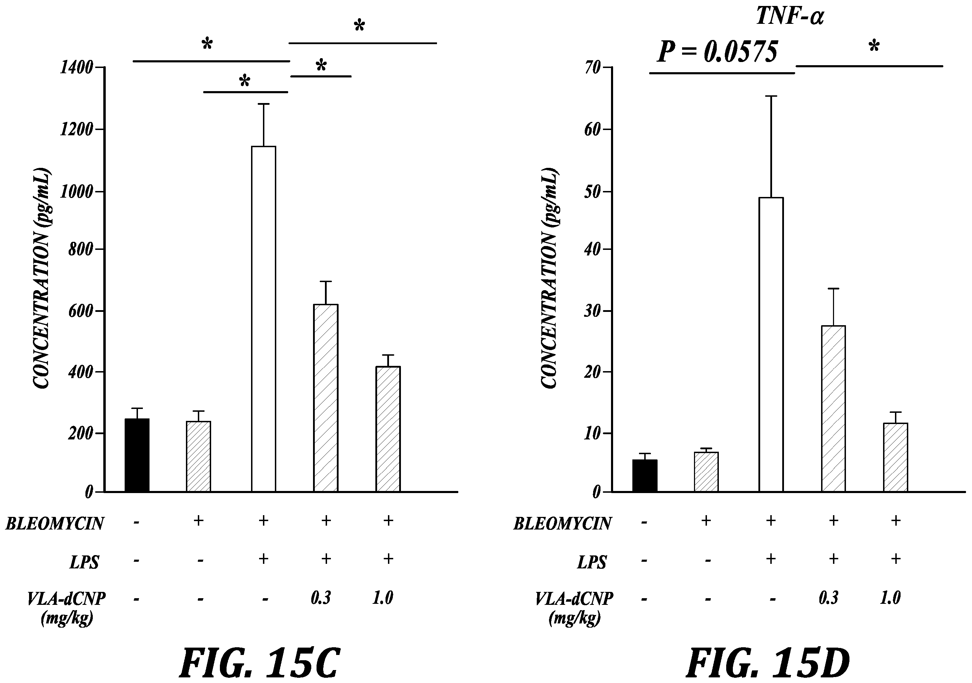

Referring to FIGURE 15A, VLA-dCNP decreased cell numbers in BALF from Acute exacerbations of idiopathic pulmonary fibrosis (IPF-AE) model. Referring to FIGURE 15B, VLA-dCNP decreased protein levels in BALF from Acute exacerbations of idiopathic pulmonary fibrosis (IPF-AE) model. Referring to FIGURE 15C, VLA-dCNP attenuated IL-6 in BALF from acute exacerbations of idiopathic pulmonary fibrosis (IPF-AE) model. Referring to FIGURE 15D, VLA-dCNP decreased cell numbers and protein levels and attenuated TNFα in BALF from acute exacerbations of idiopathic pulmonary fibrosis (IPF-AE) model.

### Example 16. VLA-dCNP decreased tubular injury in cisplatin (CDDP) induced acute kidney injury (AKI).

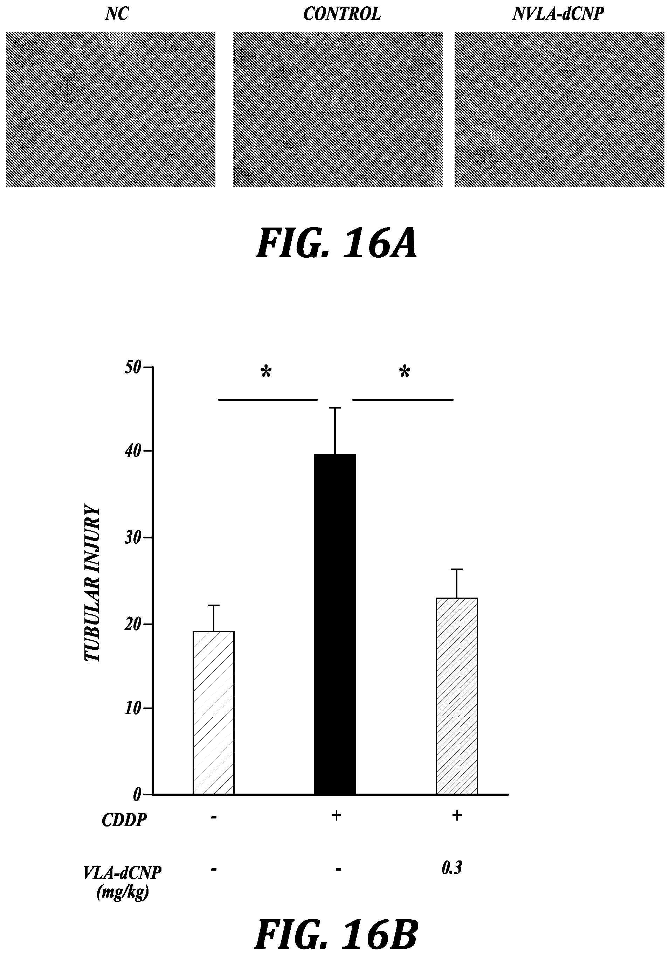

Referring to FIGURES 16A and 16B, VLA-dCNP decreased tubular injury in the cisplatin (CDDP) induced Acute Kidney Injury (AKI).

C57BL/6J mice (8 weeks old, male, n = 8,7,8/group) were purchased from Kyudo (Saga, Japan) and maintained under a 12-hour light/12-hour dark cycle with free access to water and standard mouse diet (MF diet; Oriental Yeast Co., Ltd. Tokyo, Japan). Mice were treated with CDDP (TCI Tokyo Japan; 6 mg/kg b.w. IP saline(Otsuka Pharmaceutical, Tokushima, Japan)) at day 2, day 9 and day16 and VLA-dCNP (described in Example 1) (0.3 mg/kg) in buffer (methionine 100 mM (Tokyo Chemical Industry Co., Ltd., Tokyo, Japan); sucrose 100 mM (Tokyo Chemical Industry Co., Ltd.); histidine 50 mM (Tokyo Chemical Industry Co., Ltd.); in H₂O (Otsuka Pharmaceutical, Tokushima, Japan)) or buffer (for control group) (subcutaneous injection under isoflurane anesthesia, 5 times/week). Mice were sacrificed at day 19 under isoflurane vapor. Kidney were fixed by paraformaldehyde. Deparaffinized tissue sections were immersed in 0.5% orthoperiodic acid for 7 minutes at room temperature and washed by purified water for 2 minutes each 2 times and sections were stained with Schiff's reagent for 15 minutes at room temperature. After that, sections were immersed in sulfite water (10% sodium hydrogen sulfite 10 mL, 1N hydrochloric acid 10 mL, purified water 180 mL) for 2 minutes each 3 times at room temperature and washed by running water for 5 minutes. Finally, sections were stained with Mayer's hematoxylin solution for 4 minutes at room temperature and washed by running water for 5 minutes and kidney injury were evaluated by using a fluorescence microscope with brightfield mode (BZ-X700, Keyence Tokyo, Japan), the magnification was x20.

### Example 17. VLA-dCNP and long acting CNP suppress liver enzymes and inflammation/fibrosis markers in diet-induced liver fibrosis

This Example employs a choline-deficient, amino acid-defined, high fat diet model known to rapidly induce fibrosis. *See, e.g.,* Matsumoto et.al., Int J Exp Pathol. 2013 Apr; 94(2):93-103, incorporated herein by reference in its entirety. Asparate transaminase (AST) elevation shows damage in liver, or other organs that can sustain inflammation and fibrotic process. Alanine transaminase (ALT) elevation indicates a liver injury that can sustain inflammation and fibrotic process of the liver. Activated hepatic stellate cells, which are the main collagen producing cells in liver fibrogenesis, display an increase in alpha-Smooth Muscle Actin (α-SMA) during fibrogenesis. Additionally, liver tissue shows an increase in inflammation markers such as tumor necrosis factor alpha (TNFa) and monocyte chemoattractant protein 1 (MCP-1) during the process of fibrogenesis. All these markers (AST, ALT, α-SMA, TNFα, and MCP1) were suppressed when the subject was given a high bolus dose (1mg/Kg) of long acting CNP derivatives and medium bolus dose (0.3mg/Kg) and high bolus dose (1mg/Kg) of VLA-dCNP (described in Example 1). Taken together long-acting CNP derivative and VLA-dCNP suppressed tissue injury, inflammation, and the fibrotic process.

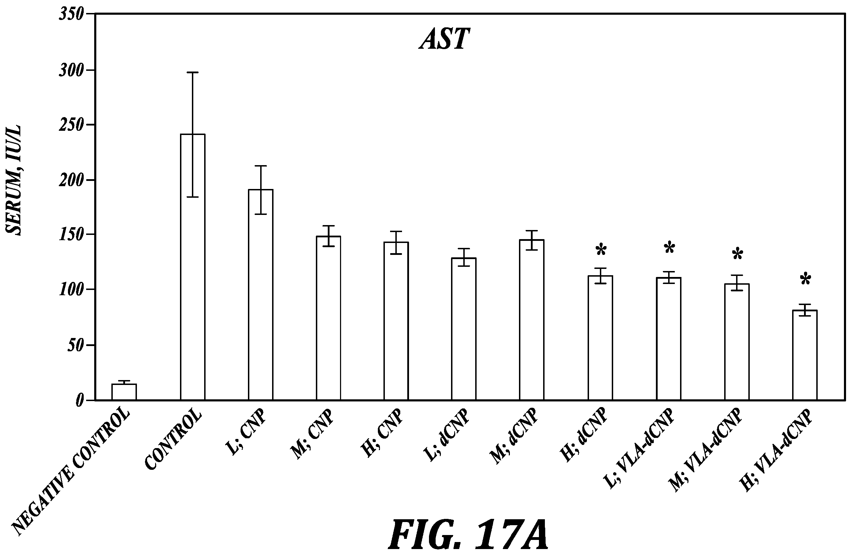

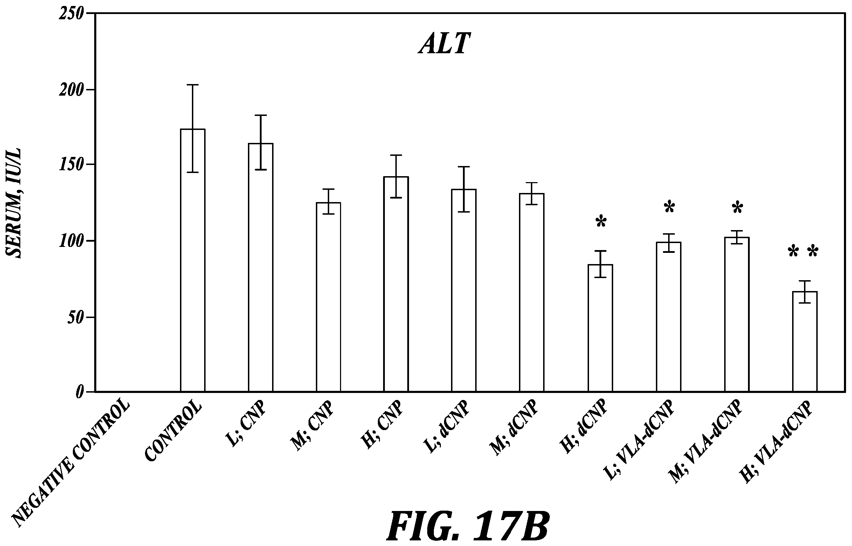

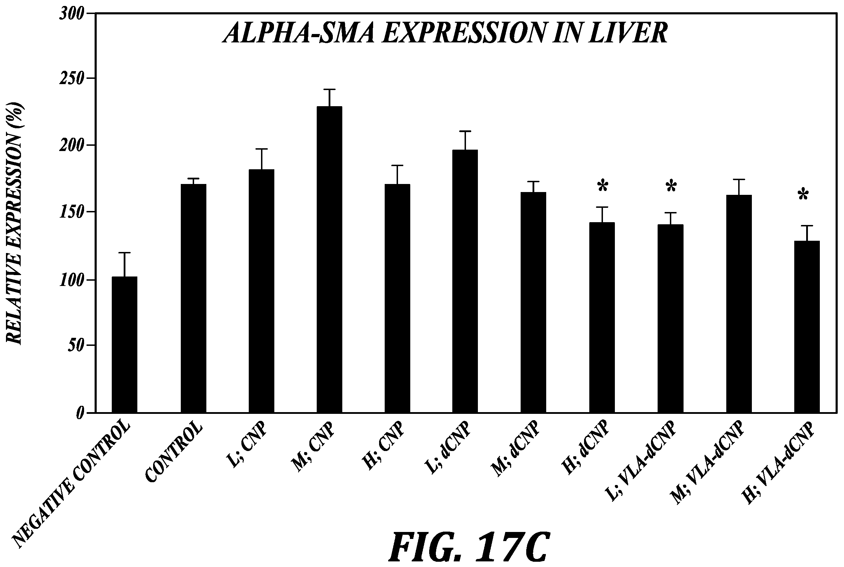

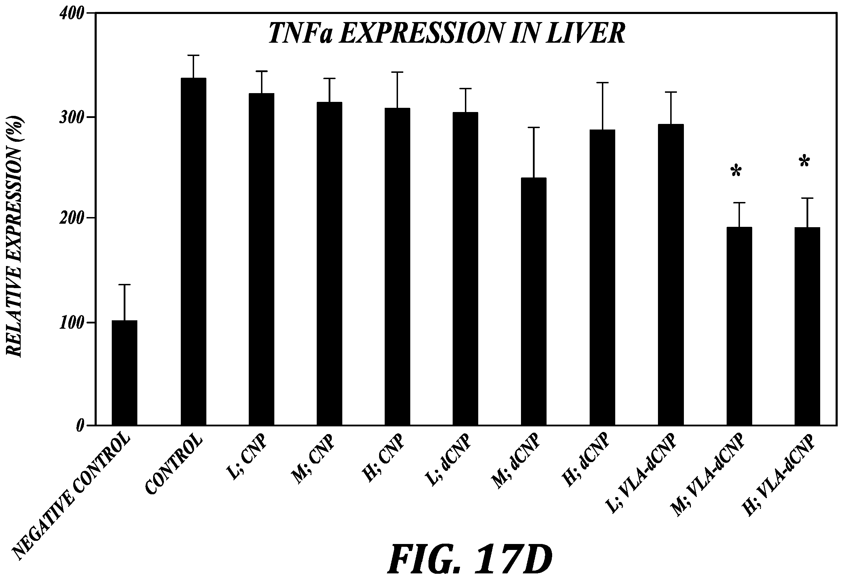

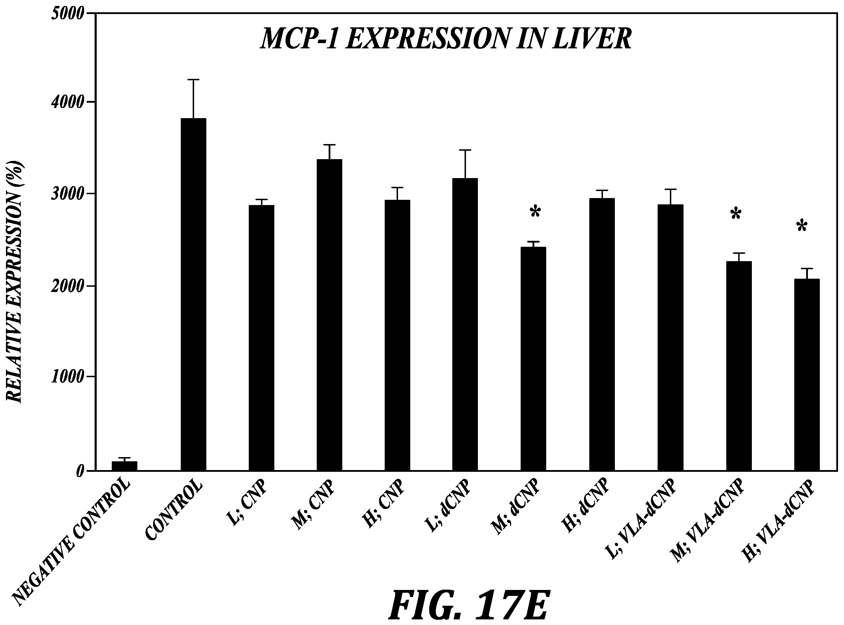

Referring to FIGURES 17A-17E, VLA-dCNP and long acting CNP suppressed Liver enzymes and inflammation/fibrosis markers in diet-induced liver fibrosis. FIGURE 17A shows a significant decrease in liver enzyme aspartate aminotransferase (AST); FIGURE 17B shows a significant decrease in liver enzyme alanine aminotransferase (ALT); FIGURE 17C shows significant decrease in alpha smooth muscle actin (a-SMA), a marker of fibrotic cells; FIGURE 17D shows significant decrease in tumor necrosis growth factor alpha (TNF-a), a marker of inflammation inducing fibrosis; and FIGURE 17E shows significant decrease in monocytes chemoattractant protein 1 (MCP-1), mediator of macrophage induced inflammation in liver tissue, when the subject is administered with long-acting CNP derivative and/or VLA-dCNP.

In this study, C57BL/6J mice (6 weeks old, male, n = 10/group) were purchased from Kyudo (Saga, Japan) and maintained under a 12-hour light/12-hour dark cycle with free access to water and standard mouse diet (MF diet; Oriental Yeast Co., Ltd. Tokyo, Japan) or Choline-deficient, amino acid-defined high fat diet, (CDAHFD) (Research Diet, New Brunswick, NJ). Mice were treated with and VLA-dCNP (0.1, 0.3, or 1.0 mg/kg), dCNP (0.1, 0.3, or 1.0 mg/kg), and CNP (0.1, 0.3, 1.0 mg/Kg) in buffer 100 mM (Tokyo Chemical Industry Co., Ltd.); histidine 50 mM (Tokyo Chemical Industry Co., Ltd.); in H₂O (Otsuka Pharmaceutical, Tokushima, Japan)) or buffer (for control group) (subcutaneous bolus injection under isoflurane anesthesia over less than 30 seconds, 5 times/week (week-day only administration) for two weeks starting on Day 5^{th}. Blood/plasma samples were collected from cardiac puncture under isoflurane and liver was harvested after the puncture at Day 17 (at 8.5 weeks old). AST and ALT were evaluated using enzyme substrate assay (Fuji film, Wako, Japan).

### Example 18. Long-acting CNP derivatives (dCNP) and VLA-dCNP decrease kidney fibrosis and improve kidney function in cisplatin induced acute kidney injury

Cisplatin is a common potent chemotherapeutic agent for the treatment of cancer, but the dose-limiting side effect is nephrotoxicity leading to acute kidney injury. Serum creatinine provide an indication of how well the kidneys are working. The albumin-to-creatinine ratio in urine can provide a more accurate indication of the how much albumin is being released into the urine. The presence of a small amount of albumin in the urine can be an early indicator of kidney disease.

Renal tissue injury initiates inflammatory and fibrotic processes that occur to promote regeneration and repair. After renal injury, damaged tissue releases cytokines (TNF-α, tumor necrosis factor-a; IL(s), interleukin(s); and TGF-β, transforming growth factor-β) and chemokines (SDF-1, stromal cell-derived factor-1; MCP-1, monocyte chemoattractant protein-1; CCL2; CX3CL1, Fractalkine; and CXCL10, C-X-C motif chemokine 10) which stimulate activation and infiltration of inflammatory cells (Neutrophils; Monocytes; Mϕ, macrophage; NK cells, natural killer cells; T cells; B cells) to the kidney. Normal tissue repair processes occur simultaneously with activation of myofibroblasts, collagen deposition, and wound healing responses; however, prolonged activation of proinflammatory and pro-fibrotic cell types (Fibroblast/Fibrocytes, Myofibroblast/Pericytes) causes excess extracellular matrix deposition (*see*, *e.g*., Black et al., Renal Inflammation and Fibrosis: A Double-edged Sword, Journal of Histochemistry & Cytochemistry 2019, Vol. 67(9) 663-681, incorporated herein by reference in its entirety), leading to chronic kidney disease (CKD). *See, e.g.,* Eoghainin Ó hAinmhire, Benjamin D. Humphreys; Fibrotic Changes Mediating Acute Kidney Injury to Chronic Kidney Disease Transition Nephron 2017;137:264-267, incorporated herein by reference in its entirety.

The therapeutic agents used for cancer treatment can cause damages to major organ systems, including the heart (*i.e*., cardiotoxicity), lungs (*e.g*., pulmonary fibrosis), and bone (*e.g*., bone marrow suppression). Cancer and its treatment can increase the likelihood of acute kidney injury that can lead to fibrosis and chronic kidney disease. Cancer cells can cause urinary tract obstruction that leads to acute kidney injury leading to inflammation, and fibrosis (*e.g*., prostate or urothelial cancer, cancer of the uterus or ovary, compression of the urinary tract by retroperitoneal node enlargement, a tumor mass, and/or retroperitoneal fibrosis). Systemic anticancer treatment can damage the kidney directly (*e.g*., cisplatin-induced necrosis of the proximal tubule) or indirectly (*e.g*., methotrexate-induced crystal nephropathy and tumor lysis syndrome) both leading to inflammation, fibrosis, and chronic kidney disease. acute kidney injury is a serious adverse drug reaction of conventional cytotoxic chemotherapeutic agents and can affect the efficacy of cancer treatment and the survival of the patient. *See, e.g.,* Perazella M.A., Onco-nephrology: renal toxicities of chemotherapeutic agents. Clin J Am Soc Nephrol 2012; 7: 1713-21; Malyszko *et al.,* Kozlowska K, Kozlowski L, Malyszko J. Nephrotoxicity of anticancer treatment. Nephrol Dial Transplant 2017; 32: 924-36. Cisplatin, which is used as part of chemotherapeutic regimens for a wide array of different cancers, can cause acute kidney injury in 20-30% of cases due to mitochondrial damage from reactive oxygen species. *See, e.g.,* Miller et al., Mechanisms of cisplatin nephrotoxicity. Toxins (Basel) 2010; 2: 2490-518; and Brooks et al., Regulation of mitochondrial dynamics in acute kidney injury in cell culture and rodent models. J Clin Invest 2009; 119: 1275-85. Cisplatin accumulates in the S3 segment of the proximal tubule and promotes glutathione depletion and high amounts of mitochondrial reactive oxygen species. This accumulation could be related to the selective uptake of cisplatin via active basolateral-to-apical transporters, such as CTR1 and SLC22A2 (previously known as OCT2), which are both expressed on the basolateral membrane of the S3 segment.

Another notable adverse effect of cisplatin is hearing loss or ototoxicity. Otoptoxicity results from similar mitochondrial damage from reactive oxygen species (ROS) that occurs in the inner ear upon exposure to cisplatin leading to inflammation. *See, e.g.,* Yu et al., Current Strategies to Combat Cisplatin-Induced Ototoxicity Front. Pharmacol., 03 July 2020. Studies show that ROS could stimulate cochlear inflammation. Inner ear Inflammation may trigger inner ear cell death through endoplasmic reticulum stress, autophagy, and necroptosis, which induce apoptosis. *See, e.g.,* Sheth et al., Mechanisms of Cisplatin-Induced Ototoxicity and Otoprotection, Frontiers in Cellular Neuroscience, 27 Oct., Vol 11, 2017.

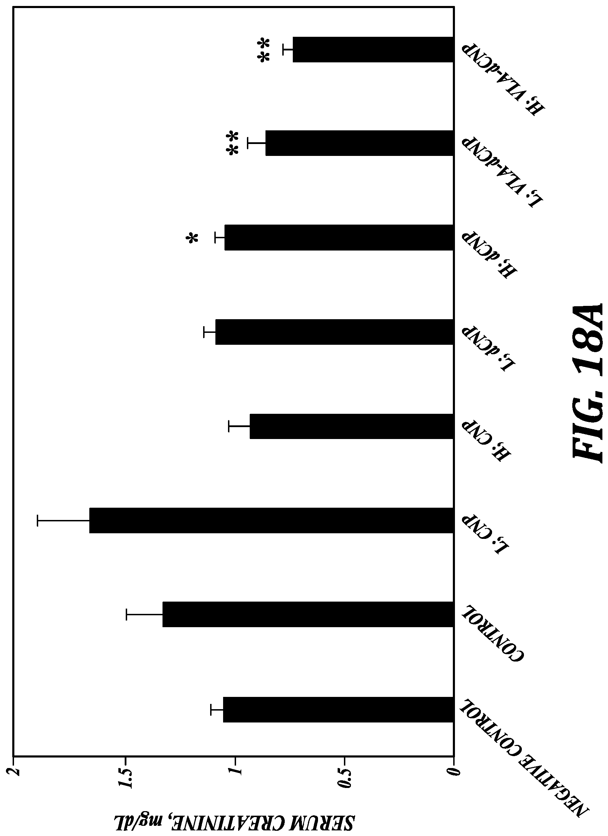

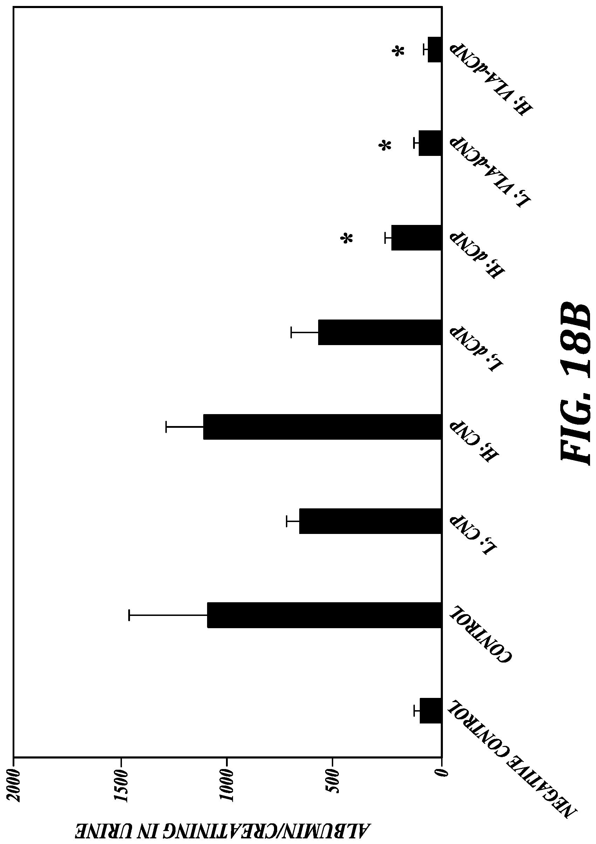

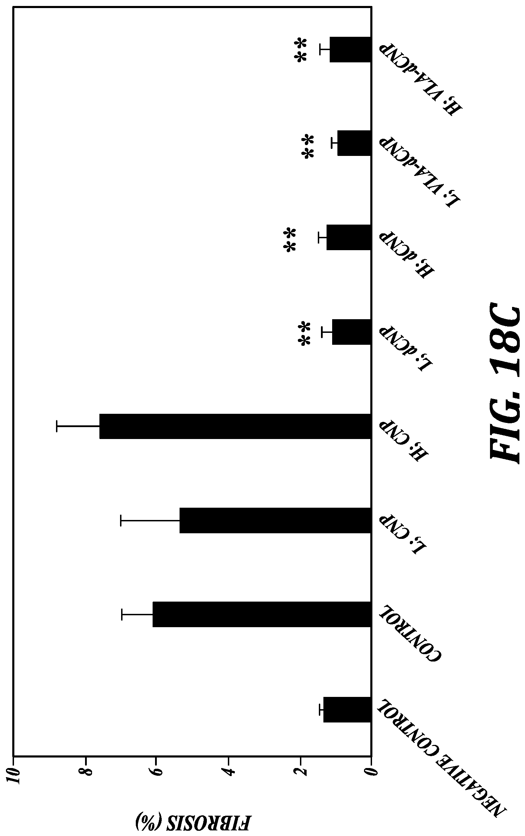

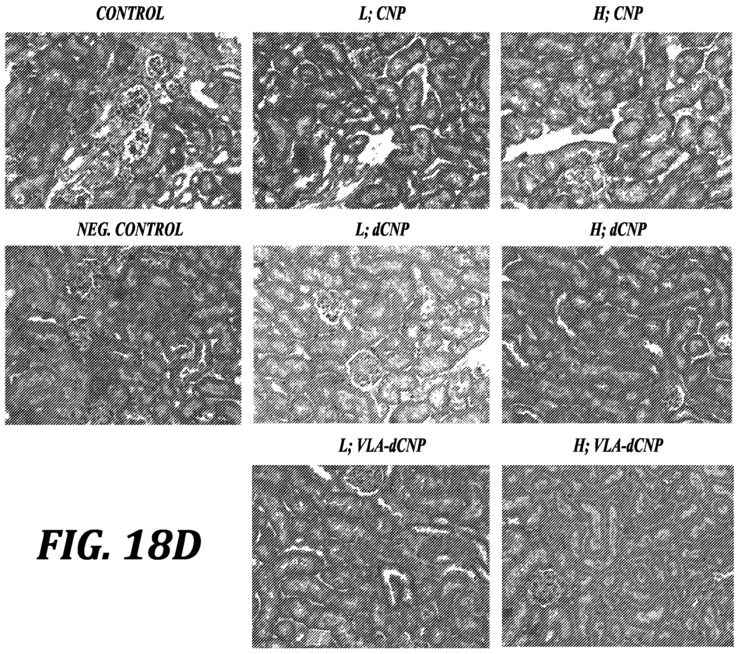

FIGURE 18A shows significant improvement in kidney function based on decrease in serum creatinine; FIGURE 18B shows significant improvement in kidney function based on decreased albumin level in urine by calculating albumin-to-creatinine ratio; FIGURE 18C shows significant decrease in % fibrosis area in kidney; Fibrosis area was measured by using Image J (NIH, Bethesda, Maryland, USA); FIGURE 18D is a series of representative images of Masson's Trichrome (MT) stain of kidneys. Magnification is X20. In this Masson's Trichrome stain, nucleus is stained with iron hematoxylin (brown/black color in the image), cytoplasm is stained with acid fuchsin (pink/red color in the image), and collagen fibrotic area is stained with aniline blue (blue color in the image).

In this study, mice were treated with CDDP (TCI Tokyo Japan; 10 mg/kg b.w. IP saline (Otsuka Pharmaceutical, Tokushima, Japan)) at day 0, day 7, day 14, and day 21 and CNP (low dose (L): 0.1 mg/Kg; and high dose (H): 1.0 mg/Kg), dCNP (described in Example 1) (L: 0.1 mg/Kg; and H: 1.0 mg/Kg), or VLA-dCNP (described in Example 1) (L: 0.1 mg/Kg; and H: 1.0 mg/kg) in buffer (methionine 100 mM (Tokyo Chemical Industry Co., Ltd., Tokyo, Japan); sucrose 100 mM (Tokyo Chemical Industry Co., Ltd.); histidine 50 mM (Tokyo Chemical Industry Co., Ltd.); in H₂O (Otsuka Pharmaceutical, Tokushima, Japan)) or buffer (for control group) (subcutaneous bolus injection under isoflurane anesthesia over less than 30 seconds, 5 times/week for 4 weeks). In this study, negative control is no CDDP inductions and administered buffer for 5 times/week for 4 weeks. Blood/serum samples were collected from cardiac puncture under isoflurane at Day 28. Serum Creatinine, BUN, and Urine creatine were measured by colorimetric method (Arbor Assays, Ann Arbor MI), (Thermo Fisher Scientific, Waltham MA), and (R&D systems, Detroit, MN) respectively. Urine samples were harvested, and kidney were fixed by paraformaldehyde. The kidney section was stained with Masson's Trichrome stain and fibrosis area % was evaluated by using a fluorescence microscope in brightfield mode (BZ-X700, Keyence Tokyo, Japan), the magnification was x20. In this Masson's Trichrome stain, nucleus is stained with iron hematoxylin (brown/black color in the image), cytoplasm is stained with acid fuchsin (Pink/red color in the image), and collagen fibrotic area is stained with aniline blue (blue color in the image). The fibrosis area % calculation was done as follow. First, the area of the tissue was calculated by (total pixels-pixels in an empty area (the highest brightness area), then the difference in blue light intensity minus red light intensity was calculated by the Image J to convert it to pixels. In the end, the fibrosis area (%) = (fibrosis area/total tissue area) x100. Statistical analysis was based on Student's t-test performed by using GraphPad Prism 6. *P<0.05 or **P<0.01 vs. control.

### Example 19. Both long acting CNP derivative (dCNP) and VLA-dCNP suppress fibrosis in bleomycin-induced acute exacerbations of idiopathic pulmonary fibrosis (AE-IPF) model in mice

Acute exacerbation of IPF (AE-IPF) is defined as sudden acceleration of the disease or progressive form of lung disease of unknown etiology. *See, e.g.,* J Thorac Dis 2015 7(3) 499-519. Hydroxyproline is a major component of the collagen and plays a key role in the stability of the collagen triple helix. In this study, it was used to assess collagen content in the lung tissues.

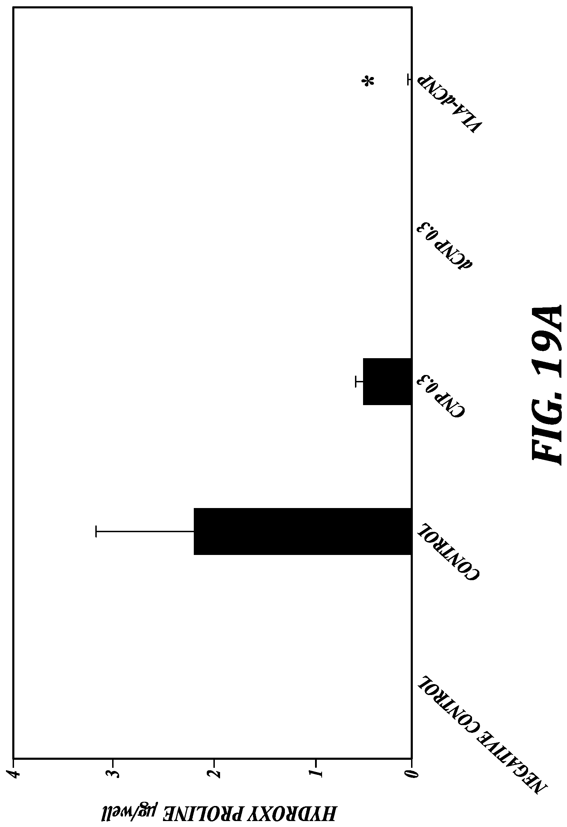

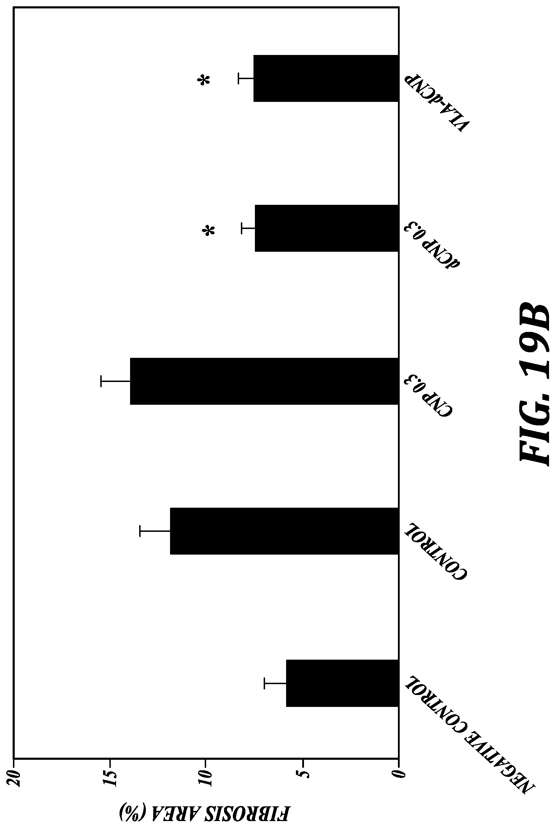

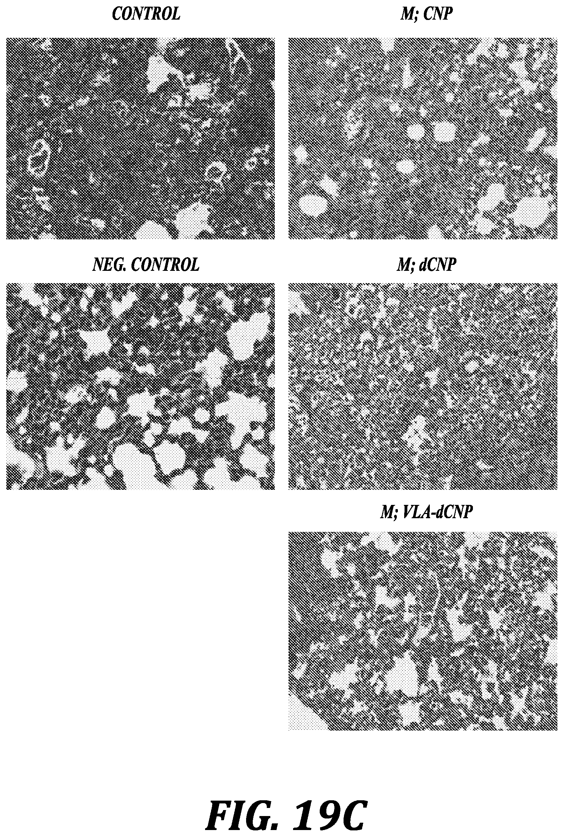

FIGURE 19A shows a significant decrease in fibrosis based on a decrease in hydroxyproline, a major component of the collagen, in lung tissue; FIGURE 19B shows a significant decrease in the % fibrosis area in lung-based quantification of evaluation of histological Masson's Trichrome staining of lung tissue sections. Fibrosis area was measured by using Image J (NIH, Bethesda, Maryland, USA); FIGURE 19C shows representative images of Masson's Trichrome (MT) stained kidneys at magnification is X20.

In this study, male C57BL/6J mice (6 week old, male, n=6/group) were purchased from Kyudo (Saga, Japan) and maintained under a 12-hour light/12-hour dark cycle with free access to water and standard mouse diet (MF diet; Oriental Yeast Co., Ltd. Tokyo, Japan). Mice were treated with bleomycin (Nippon Kayaku Tokyo Japan; 1.0 mg/kg intratracheal administration). After 2 weeks, mice were treated with LPS (0.05 mg/kg intratracheal administration Sigma Aldrich, St. Louis, MO, USA) and treated with each bolus dose of CNP (0.3 mg/Kg), dCNP (described in Example 1) (0.3 mg/Kg), or VLA-dCNP (described in Example 1) (0.3 mg/Kg) in buffer (methionine 100 mM (Tokyo Chemical Industry Co., Ltd., Tokyo, Japan); sucrose 100 mM (Tokyo Chemical Industry Co., Ltd.); histidine 50 mM (Tokyo Chemical Industry Co., Ltd.); in H₂O (Otsuka Pharmaceutical, Tokushima, Japan)) or buffer (for control group) (subcutaneous bolus injection under isoflurane anesthesia). Test articles and control were administered one day before LPS for 3 consecutive days. The day after the last treatment, the animals were euthanized, and part of the lung was harvested and the other part was fixed with paraformaldehyde. The part of the lung (20mg) was homogenized and extract was measured for hydroxyproline (Abcam Cambridge, UK). The fixed lung section was stained Masson's Trichrome stain and evaluated by using a fluorescence microscope with brightfield mode (BZ-X700, Keyence Tokyo, Japan), the magnification was x20. In this Masson's Trichrome stain, nucleus is stained with iron hematoxylin (brown/black color in the image), cytoplasm is stained with acid fuchsin (Pink/red color in the image), and collagen fibrotic area is stained with aniline blue (blue color in the image). The fibrosis area % calculation was done as follow. First, the area of the tissue was calculated by (total pixels-pixels in an empty area (the highest brightness area), then the difference in blue light intensity minus red light intensity was calculated by the Image J to convert it to pixels. In the end, the fibrosis area (%) = (fibrosis are/total tissue area) x100. Statistical analysis was based on Student's t-test performed by using GraphPad Prism 6. *P<0.05 vs. control

### Example 20. Pharmacokinetic profile of long acting CNP derivative s1 (dCNP-s1) and CNP derivative s2 (dCNP-s2) from a bolus administration showed sustain presence in the blood over time

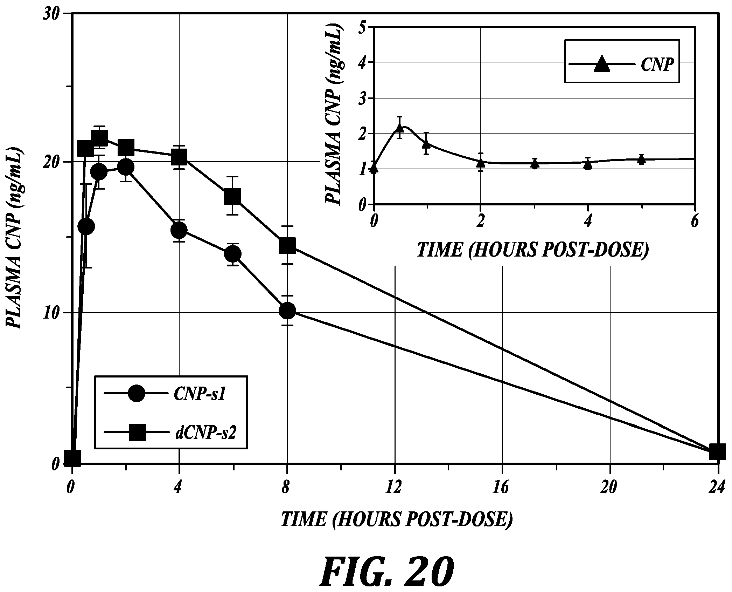

Referring to FIGURE 20, shown is a graph of plasma CNP [mean (SEM); n= 5] in CD-1 mice after subcutaneous bolus administration of 2.0mg/Kg of CNP derivative s1 (dCNP-s1), and CNP derivative s2 (dCNP-s2). For comparison, the inset shows the low plasma level of CNP (diamond) when native CNP was administered. Error bars represent standard error of the mean of n=5 plasma samples. Baseline CNP level prior to administration was 0.391 (0.02) ng/mL [mean (SEM); n=10]. Long acting dCNP-s1 and dCNP-s2 provides 10-fold higher blood level of CNP in a sustain manner (at least 8 hours) than native CNP when given at similar dose weight/Kg dose.

For this pharmacokinetic study, all animals (mice) for this study were maintained under a 12-hour light/12-hour dark cycle with free access to water and standard mouse diet (Lab Pico Rodent #5053; Animal Specialties, Woodburn, OR). Male CD-1 mice (6-8 weeks old; Charles River, Hollister, CA) were treated with 2.0 mg/Kg of CNP derivative s1 (dCNP-sl; PharmaIN Corp, Bothell, WA), and CNP derivative s2 (dCNP-s2; PharmaIN Corp, Bothell, WA) via subcutaneous administration between the shoulder blades. All test articles were formulated or dissolved in 100 mM sucrose, 100 mM methionine, 50 mM histidine, pH 7.4. Blood sampling at various times (0 hour, 0.5 hour, 1 hour, 2 hours, 4 hours, 6 hours, 8 hours, and 24 hours) was performed by retro-orbital bleed, two bleeding per animal at two different timepoints. Blood samples were processed in K2EDTA tubes to obtain plasma. Plasma was analyzed by commercially available CNP ELISA kit from Phoenix Pharmaceuticals (cat# EKE-012-03). CNP is a native human CNP (GLSKGCFGLKLDRIGSMSGLGC [SEQ ID NO. 10] and dCNP-s1 and dCNP-s2 are derivatives of human CNP with the following sequences: HOC(=O)(CH₂)₁₆C(=O)-Aeea-Aeea-GCFGLKLDRIGShomoQSGLGC [SEQ ID NO. 21], and HOC(=O)(CH₂)₁₆C(=O)-γE-Aeea-Aeea-GCFGLKLDRIGShomoQSGLGC [SEQ ID NO. 20], each with a disulfide bond between the 2 cysteine residues, and where homoQ: homoGlutamine residue; Aeea: 2-[2-(2-aminoethoxy)ethoxy]acetic acid residue, where the amino and carboxylic acid groups are used in forming amide bonds to provide the CNP derivatives; HOC(=O)(CH₂)₁₆C(=O)- was derived from octadecadioic acid; yE: gamma glutamic acid residue.

By example and without limitation, embodiments are disclosed according to the following enumerated paragraphs:
A1. A method of treating a subject having a lung, liver, and/or kidney injury, or a symptom associated with a lung, liver, and/or kidney injury, comprising:
   administering to the subject a therapeutically effective bolus dose of a composition comprising a long acting CNP, a long acting CNP derivative, a long acting NPRB agonist, a very long acting CNP, a very long acting CNP derivative, a very long acting NPRB agonist, a long acting CNP agonist, a very long acting CNP agonist, or any combination thereof,
   wherein the composition does not decrease blood pressure by more than 20% (*e.g.,* by more than 15%, by more than 10%, or by more than 5%) of a baseline blood pressure measurement taken prior to administration of the therapeutically effective bolus dose of the composition,
   wherein the composition increases plasma cyclic-GMP level at from 1 hour to 12 hours (e.g., 2 to 12 hours, 4 to 12 hours, 1 hour to-24 hours, 2 to 24 hours, 4 to 24 hours, 1 hour to 84 hours, 2 to 84 hours, 4 to 84 hours, 12 to 84 hours, 1 hour to 168 hours, 2 to 168 hours, 4 to 168 hours, or 12 to 168 hours) after administration to above 1.5x of a baseline plasma cyclic-GMP level, the baseline plasma cyclic-GMP level is an average plasma cyclic-GMP level prior to administration of the composition or the average plasma cyclic-GMP level of a healthy subject (preferably the average plasma cyclic-GMP level prior to administration of the composition for the subject), and
   wherein the lung, liver, and/or kidney injury, or the symptom associated with lung, kidney, and/or kidney injury is selected from
      i) acute lung injury (ALI),
      ii) acute respiratory distress syndrome (ARDS),
      iii) pulmonary edema,
      iv) elevated level of inflammatory cells in the lung,
      v) increased level or expression of inflammatory cytokines in the lung as compared to healthy lung,
      vi) increased protein level in lung alveolar space as compared to healthy lung,
      vii) low arterial blood oxygenation, wherein low arterial blood oxygenation is a blood PaO2 of below 60 mm Hg and/or a blood hemoglobin oxygen saturation (SpO2) of below 90%,
      viii) pneumonia,
      ix) fibrosis,
      x) kidney injury,
   and any combination thereof (e.g., a combination of two, three, four, five, six, seven, eight, nine, or ten of i) to x)).
A2. The method of Paragraph A1, wherein the long acting CNP derivative or the very long acting CNP derivative comprises U-GLSKGCFGLKLDRIGSMSGLGC [SEQ ID NO. 2], U-GLSKGCFGLK(U)LDRIGSMSGLGC [SEQ ID NO. 3], GLSKGCFGLK(U)LDRIGSMSGLGC [SEQ ID NO. 4], U-CFGLKLDRIGSxSGLGC, where x is a natural or unnatural amino acid residue [SEQ ID NO. 11], or any combination thereof,
   wherein:
   U is a moiety of Formula (I) or (II), where Formula (I) is

      (aliphatic)ₐ-(X)-; (I)
   wherein
      a is 0 or 1 (preferably a is 1);
      aliphatic is an optionally substituted C₄₋₂₄ chain (*e.g*., an optionally substituted C₁₀₋₂₄ chain, an optionally substituted C₁₂₋₁₈ chain), covalently bound to X via a chemical linkage, such as a carbonyl (*e.g*., as part of an amide or an ester linkage), a thioether, an ether, a thioether, a carbamate moiety, a bond, or the like with X; preferably via a carbonyl as part of an amide or an ester linkage; or more preferably via a carbonyl as part of an amide linkage with X;
      X is a 1-10 amino acid residue or peptide sequence, wherein each amino acid residue is independently selected from lysine (K), arginine (R), glycine (G), alanine (A), glutamic acid (E), and aspartic acid (D); or
      X is a linker (γE)ₘ-(B)ₙ,
      wherein B is a 1-8 amino acid residue sequence wherein each amino acid residue is independently selected from 2-[2-(2-aminoethoxy)ethoxy]acetic acid residue, Gly, Ala, Leu, Ser, Arg, and Lys;
      m is 0, 1, 2, or 3;
      n is 0, 1, 2, or 3; and
      the sum of m and n is at least 1,
   and Formula (II) is

      (polymer)ₐ-(Y)-; (II)
   wherein
      a is 0 or 1 (preferably a is 1);
      polymer is cellulose, poly(ethylene glycol) (PEG), methoxy poly(ethylene glycol) (MPEG), poly(lactic-co-glycolic acid), poly(N-vinyl pyrrolidone), or a derivative thereof;
      Y is:
         a 1-10 amino acid residue or peptide sequence, wherein each amino acid residue is independently selected from lysine (K), arginine (R), glycine (G), alanine (A), glutamic acid (E), and aspartic acid (D);
         a non-amino acid linker comprising an ester, an amide, a thioether, an ether, a thioether, a carbamate moiety, or a combination thereof;
         an amino acid residue-containing linker, wherein the amino acid residue is covalently attached to (polymer)ₐ; or
         a peptide linker different from the 1-10 amino acid residue or peptide sequence.
A3. The method of Paragraph A2, wherein Y is a linker (γE)ₘ-(B)ₙ, wherein B is a 1-8 amino acid residue or peptide sequence wherein each amino acid residue is independently selected from 2-[2-(2-aminoethoxy)ethoxy]acetic acid residue, Gly, Ala, Leu, Ser, Arg, and Lys; m is 0, 1, 2, or 3; n is 0, 1, 2, or 3; and the sum of m and n is at least 1.
A4. The method of any one of Paragraphs A1 to A3, wherein the long acting CNP derivative or the very long acting CNP derivative comprises U-GLSKGCFGLKLDRIGSMSGLGC [SEQ ID NO. 2], U-GLSKGCFGLK(U)LDRIGSMSGLGC [SEQ ID NO. 3], GLSKGCFGLK(U)LDRIGSMSGLGC [SEQ ID NO. 4], U-CFGLKLDRIGSxSGLGC, where x is a natural or unnatural amino acid residue [SEQ ID NO. 12], or any combination thereof; and;
   wherein:
   U is a moiety of Formula (I), where Formula (I) is

      (aliphatic)ₐ-(X)-; (I)
   wherein
      a is 0 or 1 (preferably a is 1);
      aliphatic is an optionally substituted C₁₀₋₂₄ chain (*e.g*., an optionally substituted C₁₂₋₁₈ chain), covalently bound to X via a chemical linkage, such as a carbonyl (*e.g.,* as part of an amide or an ester linkage), a thioether, an ether, a thioether, a carbamate moiety, a bond, or the like with X; preferably via a carbonyl as part of an amide or an ester linkage; or more preferably via a carbonyl as part of an amide linkage with X;
      X is a 1-10 amino acid residue or peptide sequence, wherein each amino acid residue is independently selected from lysine (K), arginine (R), glycine (G), alanine (A), glutamic acid (E), and aspartic acid (D); or
      X is a linker (γE)ₘ-(B)ₙ,
      wherein B is a 1-8 amino acid residue or peptide sequence wherein each amino acid residue is independently selected from 2-[2-(2-aminoethoxy)ethoxy]acetic acid residue, Gly, Ala, Leu, Ser, Arg, and Lys;
      m is 0, 1, 2, or 3;
      n is 0, 1, 2, or 3; and
      the sum of m and n is at least 1.
A5. The method of any one of Paragraphs A2 to A4, wherein X is a 4-7 amino acid sequence, wherein each amino acid residue is independently selected from lysine (K), arginine (R), and glycine (G), or
   X is a linker (γE)ₘ-(B)ₙ wherein B is a 1-8 amino acid residue sequence wherein each amino acid residue is independently selected from 2-[2-(2-aminoethoxy)ethoxy]acetic acid residue, Gly, Ala, Leu, Ser, Arg, and Lys; m is 0, 1, 2, or 3; n is 0, 1, 2, or 3; and the sum of m and n is at least 1.
A6. The method of any one of Paragraphs A1 to A5, wherein the long acting CNP derivative or the very long acting CNP derivative comprises U-GLSKGCFGLKLDRIGSMSGLGC [SEQ ID NO. 2],
   wherein:
   U is (aliphatic)ₐ-(X)-;
   wherein
      a is 1;
      aliphatic is an optionally substituted C₄₋₂₄ chain (*e.g*., an optionally substituted C₁₀₋₂₄ chain, an optionally substituted C₁₂₋₁₈ chain), covalently bound to X via a chemical linkage, such as a carbonyl (*e.g.,* as part of an amide or an ester linkage), a thioether, an ether, a thioether, a carbamate moiety, a bond, or the like with X; preferably via a carbonyl as part of an amide or an ester linkage; or more preferably via a carbonyl as part of an amide linkage with X; and
      X is a 1-10 amino acid residue or peptide sequence, wherein each amino acid residue is independently selected from lysine (K), arginine (R), glycine (G), alanine (A), glutamic acid (E), and aspartic acid (D); or
      X is a linker (γE)ₘ-(B)ₙ wherein B is a 1-8 amino acid residue or peptide sequence wherein each amino acid residue is independently selected from 2-[2-(2-aminoethoxy)ethoxy]acetic acid residue, Gly, Ala, Leu, Ser, Arg, and Lys; m is 0, 1, 2, or 3; n is 0, 1, 2, or 3; and the sum of m and n is at least 1.
A7. The method of any one of Paragraphs A2 to A6, wherein aliphatic does not comprise a straight or branched optionally substituted C₄₋₉ chain (*e.g.,* an optionally substituted C₃₋₈ alkyl-C(=O)- moiety, and/or an optionally substituted C₄₋₉ alkyl that is covalently bound to the peptide via a linkage such as a thioether, an ether, a thioether, a carbamate moiety, a bond, or the like).
A8. The method of any one of Paragraphs A1 to A7, wherein the long acting CNP derivative is selected from
   CH₃(CH₂)₁₄C(=O)KKKKGGGGLSKGCFGLKLDRIGSMSGLGC [SEQ ID NO. 5];
   CH₃(CH₂)₁₆C(=O)KKKKGGGGLSKGCFGLKLDRIGSMSGLGC [SEQ ID NO. 6];
   CH₃(CH₂)₁₈C(=O)KKKKGGGGLSKGCFGLKLDRIGSMSGLGC [SEQ ID NO. 7];
   CH₃(CH₂)₂₀C(=O)KKKKGGGGLSKGCFGLKLDRIGSMSGLGC [SEQ ID NO. 8];
   CH₃(CH₂)₂₂C(=O)KKKKGGGGLSKGCFGLKLDRIGSMSGLGC [SEQ ID NO. 9];
   HOC(=O)(CH₂)₁₆C(=O)-γE-Aeea-Aeea-GCFGLKLDRIGShomoQSGLGC comprising a disulfide bond between the cysteine residues [SEQ ID NO. 20]; and
   HOC(=O)(CH₂)₁₆C(=O)-Aeea-Aeea-GCFGLKLDRIGShomoQSGLGC comprising a disulfide bond between the cysteine residues [SEQ ID NO. 21].
A9. The method of any one of Paragraphs A1 to A8, wherein the long acting CNP derivative is CH₃(CH₂)₁₄C(=O)KKKKGGGGLSKGCFGLKLDRIGSMSGLGC [SEQ ID NO. 5].
A10. The method of any one of Paragraphs A1 to A8, wherein the long acting CNP derivative is CH₃(CH₂)₁₆C(=O)KKKKGGGGLSKGCFGLKLDRIGSMSGLGC [SEQ ID NO. 6].
A11. The method of any one of Paragraphs A1 to A8, wherein the long acting CNP derivative is CH₃(CH₂)₁₈C(=O)KKKKGGGGLSKGCFGLKLDRIGSMSGLGC [SEQ ID NO. 7].
A12. The method of any one of Paragraphs A1 to A8, wherein the long acting CNP derivative is CH₃(CH₂)₂₀C(=O)KKKKGGGGLSKGCFGLKLDRIGSMSGLGC [SEQ ID NO. 8].
A13. The method of any one of Paragraphs A1 to A8, wherein the long acting CNP derivative is CH₃(CH₂)₂₂C(=O)KKKKGGGGLSKGCFGLKLDRIGSMSGLGC [SEQ ID NO. 9].
A14. The method of any one of Paragraphs A1 to A8, wherein the long acting CNP derivative is HOC(=O)(CH₂)₁₆C(=O)-γE-Aeea-Aeea-GCFGLKLDRIGShomoQSGLGC comprising a disulfide bond between the cysteine residues [SEQ ID NO. 20].
A15. The method of any one of Paragraphs A1 to A8, wherein the long acting CNP derivative is HOC(=O)(CH₂)₁₆C(=O)-Aeea-Aeea-GCFGLKLDRIGShomoQSGLGC comprising a disulfide bond between the cysteine residues [SEQ ID NO. 21].
A16. The method of any one of Paragraphs A1 to A3, wherein the long acting CNP derivative or the very long acting CNP derivative comprises U-GLSKGCFGLKLDRIGSMSGLGC [SEQ ID NO. 2], U-GLSKGCFGLK(U)LDRIGSMSGLGC [SEQ ID NO. 3], GLSKGCFGLK(U)LDRIGSMSGLGC [SEQ ID NO. 4], U-CFGLKLDRIGSxSGLGC, where x is a natural or unnatural amino acid residue [SEQ ID NO. 27], or any combination thereof;
   wherein:
   U is a moiety of Formula (II), where Formula (II) is

      (polymer)ₐ-(Y)-; (II)
   wherein
      a is 0 or 1 (preferably a is 1);
      polymer is cellulose, poly(ethylene glycol) (PEG), methoxy poly(ethylene glycol) (MPEG), poly(lactic-co-glycolic acid), or poly(N-vinyl pyrrolidone);
      Y is:
         a 4-10 amino acid residue or peptide sequence, wherein each amino acid residue is independently selected from lysine (K), arginine (R), and glycine (G);
         a non-amino acid linker comprising an ester, an amide, a thioether, an ether, a thioether, a carbamate moiety, or a combination thereof; or
         a linker (γE)ₘ-(B)ₙ, wherein B is 1-8 amino acid residue sequence wherein each amino acid residue is independently selected from 2-[2-(2-aminoethoxy)ethoxy]acetic acid residue, Gly, Ala, Leu, Ser, Arg, and Lys; m is 0, 1, 2, or 3; n is 0, 1, 2, or 3; and the sum of m and n is at least 1.
A17. The method of any one of Paragraphs A1 to A3 and A16, wherein the long acting CNP derivative or the very long acting CNP derivative comprises U-GLSKGCFGLKLDRIGSMSGLGC [SEQ ID NO. 2], U-GLSKGCFGLK(U)LDRIGSMSGLGC [SEQ ID NO. 3], or any combination thereof;
   wherein:
   U is a moiety of Formula (II), where Formula (II) is

      (polymer)ₐ-(Y)-; (II)
   wherein
      a is 1;
      polymer is cellulose, poly(ethylene glycol) (PEG), methoxy poly(ethylene glycol) (MPEG), poly(lactic-co-glycolic acid), poly(N-vinyl pyrrolidone), or a derivative thereof;
      Y is:
         a 1-10 amino acid residue or peptide sequence, wherein each amino acid residue is independently selected from lysine (K), arginine (R), glycine (G), alanine (A), glutamic acid (E), and aspartic acid (D);
         a non-amino acid linker comprising an ester, an amide, a thioether, an ether, a thioether, a carbamate moiety, or a combination thereof;
         an amino acid residue-containing linker, wherein the amino acid residue is covalently attached to (polymer)ₐ;
         a peptide linker different from the 1-10 amino acid residue or peptide sequence; or
         a linker (γE)ₘ-(B)ₙ, wherein B is 1-8 amino acid residue or peptide sequence wherein each amino acid residue is independently selected from 2-[2-(2-aminoethoxy)ethoxy]acetic acid residue, Gly, Ala, Leu, Ser, Arg, and Lys; m is 0, 1, 2, or 3; n is 0, 1, 2, or 3; and the sum of m and n is at least 1.
A18. The method of any one of Paragraphs A1 to A3, A14, and A15, wherein the polymer does not include poly(ethylene glycol), MPEG, or both poly(ethylene glycol) and MPEG.
A19. The method of any one of Paragraphs A1 to A3, and A16 to A18, wherein Y is:
   a 4-10 amino acid residue or peptide sequence, wherein each amino acid residue is independently selected from lysine (K), arginine (R), and glycine (G); or
   a linker (γE)ₘ-(B)ₙ, wherein B is 1-8 amino acid residue or peptide sequence wherein each amino acid residue is independently selected from 2-[2-(2-aminoethoxy)ethoxy]acetic acid residue, Gly, Ala, Leu, Ser, Arg, and Lys; m is 0, 1, 2, or 3; n is 0, 1, 2, or 3; and the sum of m and n is at least 1.
A20. The method of any one of Paragraphs A1 to A19, wherein the bolus dose administration occurs at most twice a day and the route of administration comprises subcutaneous, intravenous, intramuscular, nasal, by inhalation, enteral, or any combination thereof, or
   wherein the route of administration is subcutaneous; or
   wherein the route of administration is intravenous; or
   wherein the route of administration is intramuscular; or
   wherein the route of administration is by inhalation; or
   wherein the route of administration is nasal; or
   wherein the enteral route of administration is oral.
A21. The method of any one of Paragraphs A1 to A20, wherein the subject has ALI or ARDS associated with pulmonary edema; low arterial blood oxygenation; elevated level of inflammatory cells in the lung; increase level or expression of inflammatory cytokine in the lung; sepsis; bacteremia; pneumonia, pulmonary fibrosis, or any combination thereof.
A22. The method of any one of Paragraphs A1 to A21, wherein inflammatory cytokine comprises IL-6, IL-1b, TNFα, MCP-1, IFNg, or any combination thereof.
A23. The method of any one of Paragraphs A1 to A22, wherein the pneumonia comprises bacterial pneumonia, viral pneumonia, aseptic pneumonia,
A24. The method of any one of Paragraphs A1 to A23, wherein the ALI or ARDS is caused by:
   (i) a systemic insult selected from trauma, sepsis (*i.e.,* body-wide infection), bacteremia (*i.e.,* bacteria in the blood), pancreatitis, shock, multiple transfusions, disseminated intravascular coagulation, bums, drug overdose or toxicity, opioids, aspirin, phenothiazines, tricyclic antidepressant, amiodarone, chemotherapeutic agents, nitrofurantoin, protamine, thrombotic thrombocytopenia purpura, head injury, paraquat, and any combination thereof; or
   (ii) a pulmonary insult selected from aspiration of gastric content, lung intubation, embolism, tuberculosis, viral pneumonia, bacterial pneumonia, cytogenic organizing pneumonitis, airway obstruction, smoking free-base cocaine, near-drowning, toxic gas inhalation, oxygen toxicity, lung contusion, radiation exposure, high-altitude exposure, lung re-expansion, reperfusion, and any combination thereof.
A25. The method of Paragraph A24, wherein the embolism is caused by a thrombus, fat, air, or amniotic fluid.
A26. The method of Paragraph A23 or A24, wherein the viral pneumonia is SARS caused by a coronavirus or an influenza virus.
A27. The method of any one of Paragraphs A1 to A26, wherein:
   ALI or ARDS caused by an infectious disease, or
   ALI or ARDS caused by PF, or
   ALI or ARDS caused by sepsis; or
   ALI or ARDS caused by bacteremia; or
   ALI or ARDS caused by intubation; or
   ALI or ARDS caused by a toxic gas selected from group consisting of chlorine gas, smoke, phosgene, concentrated oxygen, and any combination thereof.
A28. The method of Paragraph A27, wherein the infectious disease is caused by a coronavirus or an influenza virus.
A29. The method of any one of Paragraphs A1 to A21, wherein the fibrosis comprises lung or pulmonary fibrosis, liver fibrosis, cirrhosis, and kidney glomerular sclerosis.
A30. The method of any one of Paragraphs A1 to A29, wherein the composition comprises a long acting CNP composition or a very long acting CNP composition, comprising a CNP, a CNP derivative, or a long acting CNP derivative and a polymer excipient, the polymer excipient comprising a poly(amino acid) grafted with polyethylene glycol, fatty acid, and/or anionic moieties; wherein the polymer excipient is adapted to sequester or non-covalently bind to any of the CNP or CNP derivatives.
A31. The method of any one of Paragraphs A1 to A30, wherein the composition comprises a very long acting CNP derivative composition comprising a long acting CNP derivative and a polymer excipient, the polymer excipient comprising a poly(amino acid) grafted with polyethylene glycol, fatty acid, anionic moieties, or any combination thereof; and wherein the polymer excipient is adapted to sequester or non-covalently bind to the long acting CNP derivative.
A32. The method of any one of Paragraphs A1 and A20 to A30, wherein the long acting NPRB agonist or the very long acting NPRB agonist comprises a polypeptide.
A33. The method of Paragraph A32, wherein the polypeptide comprises an antibody.
A34. The method of any one of Paragraphs A1 and A20 to A32, wherein the long acting NPRB agonist or the very long acting NPRB agonist comprises a molecule of a molecular weight of less than 2kDa.
A35. A method of treating a subject having, or at risk of developing ALI or ARDS, comprising
   administering to the subject a therapeutically effective bolus dose of a composition comprising a long acting CNP derivative or a very long acting CNP derivative comprising U-GLSKGCFGLKLDRIGSMSGLGC [SEQ ID NO. 2], U-GLSKGCFGLK(U)LDRIGSMSGLGC [SEQ ID NO. 3], or GLSKGCFGLK(U)LDRIGSMSGLGC [SEQ ID NO. 4], U-CFGLKLDRIGSxSGLGC, where x is a natural or unnatural amino acid residue [SEQ ID NO. 11], or any combination thereof,
   wherein:
      U is a moiety of Formula (I) or (II), where Formula (I) is

         (aliphatic)ₐ-(X)-; (I)
      wherein
         a is 0 or 1 (preferably a is 1);
         aliphatic is an optionally substituted C₄₋₂₄ chain (*e.g*., an optionally substituted C₁₀₋₂₄ chain, an optionally substituted C₁₂₋₁₈ chain), covalently bound to X via a chemical linkage, such as a carbonyl (*e.g*., as part of an amide or an ester linkage), a thioether, an ether, a thioether, a carbamate moiety, a bond, or the like with X; preferably via a carbonyl as part of an amide or an ester linkage; or more preferably via a carbonyl as part of an amide linkage with X;
         X is a 1-10 amino acid residue or peptide sequence, wherein each amino acid residue is independently selected from lysine (K), arginine (R), glycine (G), alanine (A), glutamic acid (E), and aspartic acid (D); or
         X is a linker (γE)ₘ-(B)ₙ wherein B is 1-8 amino acid residue or peptide sequence wherein each amino acid residue is independently selected from 2-[2-(2-aminoethoxy)ethoxy]acetic acid residue, Gly, Ala, Leu, Ser, Arg, and Lys; m is 0, 1, 2, or 3; n is 0, 1, 2, or 3; and the sum of m and n is at least 1;
      and Formula (II) is

         (polymer)ₐ-(Y)-; (II)
      wherein
         a is 0 or 1 (preferably a is 1);
         polymer is cellulose, poly(ethylene glycol) (PEG), methoxy poly(ethylene glycol) (MPEG), poly(lactic-co-glycolic acid), poly(N-vinyl pyrrolidone), or a derivative thereof;
         Y is:
            a 1-10 amino acid residue or peptide sequence, wherein each amino acid residue is independently selected from lysine (K), arginine (R), glycine (G), alanine (A), glutamic acid (E), and aspartic acid (D);
            a non-amino acid linker comprising an ester, an amide, a thioether, an ether, a thioether, a carbamate moiety, or a combination thereof;
            an amino acid residue-containing linker, wherein the amino acid residue is covalently attached to (polymer)ₐ; or
            a peptide linker different from the 1-10 amino acid residue or peptide sequence;
   wherein the composition does not decrease blood pressure by more than 15% of a baseline blood pressure measurement, where the baseline blood pressure measurement is an average blood pressure prior to administration of the composition; and
   wherein the composition increases plasma cyclic-GMP level at from 1 hour to 12 hours (e.g., 2 to 12 hours, 4 to 12 hours, 1 hour to-24 hours, 2 to 24 hours, 4 to 24 hours, 1 hour to 84 hours, 2 to 84 hours, 4 to 84 hours, 12 to 84 hours, 1 hour to 168 hours, 2 to 168 hours, 4 to 168 hours, or 12 to 168 hours) after administration to above 1.5x *(e.g.,* above 2x, above 3x, above 4x, or above 5x) of a baseline plasma cyclic-GMP level, and the baseline plasma cyclic-GMP level is an average plasma cyclic-GMP level prior to administration of the composition or the average plasma cyclic-GMP level of a healthy subject (preferably the average plasma cyclic-GMP level prior to administration of the composition for the subject).
A36. The method of Paragraph A35, wherein Y is a linker (γE)ₘ-(B)ₙ, wherein B is 1-8 amino acid residue or peptide sequence wherein each amino acid residue is independently selected from 2-[2-(2-aminoethoxy)ethoxy]acetic acid residue, Gly, Ala, Leu, Ser, Arg, and Lys; m is 0, 1, 2, or 3; n is 0, 1, 2, or 3; and the sum of m and n is at least 1.
A37. The method of Paragraph A35 or Paragraph A36, wherein the long acting CNP derivative is selected from:
   CH₃(CH₂)₁₄C(=O)KKKKGGGGLSKGCFGLKLDRIGSMSGLGC [SEQ ID NO. 5];
   CH₃(CH₂)₁₆C(=O)KKKKGGGGLSKGCFGLKLDRIGSMSGLGC [SEQ ID NO. 6];
   CH₃(CH₂)₁₈C(=O)KKKKGGGGLSKGCFGLKLDRIGSMSGLGC [SEQ ID NO. 7];
   CH₃(CH₂)₂₀C(=O)KKKKGGGGLSKGCFGLKLDRIGSMSGLGC [SEQ ID NO. 8];
   CH₃(CH₂)₂₂C(=O)KKKKGGGGLSKGCFGLKLDRIGSMSGLGC [SEQ ID NO. 9];
   HOC(=O)(CH₂)₁₆C(=O)-γE-Aeea-Aeea-GCFGLKLDRIGShomoQSGLGC comprising a disulfide bond between the cysteine residues [SEQ ID NO. 20]; and
   HOC(=O)(CH₂)₁₆C(=O)-Aeea-Aeea-GCFGLKLDRIGShomoQSGLGC comprising a disulfide bond between the cysteine residues [SEQ ID NO. 21].
A38. The method of any one of Paragraphs A35 to A37, wherein the long acting CNP derivative is CH₃(CH₂)₁₄C(=O)KKKKGGGGLSKGCFGLKLDRIGSMSGLGC [SEQ ID NO. 5].
A39. The method of any one of Paragraphs A35 to A37, wherein the long acting CNP derivative is CH₃(CH₂)₁₆C(=O)KKKKGGGGLSKGCFGLKLDRIGSMSGLGC [SEQ ID NO. 6].
A40. The method of any one of Paragraphs A35 to A37, wherein the long acting CNP derivative is CH₃(CH₂)₁₈C(=O)KKKKGGGGLSKGCFGLKLDRIGSMSGLGC [SEQ ID NO. 7].
A41. The method of any one of Paragraphs A35 to A37, wherein the long acting CNP derivative is CH₃(CH₂)₂₀C(=O)KKKKGGGGLSKGCFGLKLDRIGSMSGLGC [SEQ ID NO. 8].
A42. The method of any one of Paragraphs A35 to A37, wherein the long acting CNP derivative is CH₃(CH₂)₂₂C(=O)KKKKGGGGLSKGCFGLKLDRIGSMSGLGC [SEQ ID NO. 9].
A43. The method of any one of Paragraphs A35 to A37, wherein the long acting CNP derivative is HOC(=O)(CH₂)₁₆C(-O)-γE-Aeea-Aeea-GCFGLKLDRIGShomoQSGLGC comprising a disulfide bond between the cysteine residues [SEQ ID NO. 20].
A44. The method of any one of Paragraphs A35 to A37, wherein the long acting CNP derivative is HOC(=O)(CH₂)₁₆C(=O)-Aeea-Aeea-GCFGLKLDRIGShomoQSGLGC comprising a disulfide bond between the cysteine residues [SEQ ID NO. 21].
A45. The method of any one of Paragraphs A35 to A44, wherein the composition comprises a very long acting CNP derivative composition comprising a long acting CNP derivative and a polymer excipient, the polymer excipient comprising a poly(amino acid) grafted with polyethylene glycol, fatty acid, anionic moieties, or any combination thereof; wherein the polymer excipient is adapted to sequester or non-covalently bind to the long acting CNP derivative.
A46. The method of any one of Paragraphs A1 to A45, wherein administering to the subject the therapeutically effective bolus dose of the composition decreases a total number of cells and total proteins in a BALF sample from the subject.
A47. The method of any one of Paragraphs A1 to A46, wherein administering to the subject the therapeutically effective bolus dose of the composition decreases MPO in a lung tissue from the subject.
A48. The method of any one of Paragraphs A1 to A47, wherein administering to the subject the therapeutically effective bolus dose of the composition attenuates inflammatory cytokine expression (*e.g*., IL-6, IL-1b, TNFa, MCP-1, and/or IFNg expression) in the subject.
A49. The method of any one of Paragraphs A1 to A48, wherein administering to the subject the therapeutically effective bolus dose of the composition decreases a fibrotic area in a lung in a subject having idiopathic pulmonary fibrosis.
A50. The method of any one of Paragraphs A1 to A49, wherein administering to the subject the therapeutically effective bolus dose of the composition decreases cell numbers and protein levels, and decreases the expression of any one of IL-6, IL-1b, TNFα, MCP-1, IFNg or any combination thereof in a subject having idiopathic pulmonary fibrosis.
A51. The method of any one of Paragraphs A1 to A49, wherein administering to the subject the therapeutically effective bolus dose of the composition decreases the expression of any one of IL-6, IL-1b, TNFα, MCP-1, IFNg, or any combination thereof, and decreases lethality in a subject having sepsis.
A52. The method of any one of Claims A1 to A51, wherein administering to the subject the therapeutically effective bolus dose of the composition decreases the expression of AST, ALT, α-SMA, IL-6, IL-1b, TNFα, MCP-1, IFNg, iNOS, Elf-1, Tollip, IRAK-1, P-P38, P-P65, β-act, STAT1, P-STAT1, STAT2, STAT3, STAT6, a fibrotic area, serum creatinine, an albumin/creatinine ratio in urine, hydroxyproline in a lung, or any combination thereof, of the subject.
A53. A composition comprising a long acting CNP derivative of comprising a formula U-CFGLKLDRIGSxSGLGC [SEQ ID NO. 30], wherein
   x is a natural or unnatural amino acid residue, provided that x is not a methionine residue; and
   U has is a moiety of Formula (I):

      (aliphatic)ₐ-(X)-; (I)
   wherein a is 0 or 1 (preferably a is 1);
   aliphatic is an optionally substituted C₄₋₂₄ chain (*e.g.*, an optionally substituted C₁₀₋₂₄ chain, an optionally substituted C₁₂₋₁₈ chain), covalently bound to X via a chemical linkage, such as a carbonyl (*e.g.*, as part of an amide or an ester linkage), a thioether, an ether, a thioether, a carbamate moiety, a bond, or the like with X; preferably via a carbonyl as part of an amide or an ester linkage; or more preferably via a carbonyl as part of an amide linkage with X;
   X is a linker (γE)ₘ-(B)ₙ wherein B is 1-8 amino acid residue or peptide sequence wherein each amino acid residue is independently selected from 2-[2-(2-aminoethoxy)ethoxy]acetic acid residue, Gly, Ala, Leu, Ser, Arg, and Lys; m is 0, 1, 2, or 3; n is 0, 1, 2, or 3; and the sum of m and n is at least 1.
A54. The composition of Paragraph A53, wherein x is homoglutamine, aliphatic is a branched or straight optionally substituted C₁₈ chain covalently bound to X via a carbonyl (e.g., CH₃(CH₂)₁₆C(=O)) or a C₁₈ chain covalently bound to X via a chemical linkage, such a thioether, an ether, a thioether, a carbamate moiety, a bond, or the like with X; preferably aliphatic is a branched or straight optionally substituted C₁₈ chain covalently bound to X via a carbonyl (*e.g.*, CH₃(CH₂)₁₆C(=O)) as part of an amide or ester linkage with X; or more preferably aliphatic is a branched or straight optionally substituted C₁₈ chain covalently bound to X via a carbonyl (*e.g.*, CH₃(CH₂)₁₆C(=O)) as part of an amide linkage with X, or aliphatic is HOC(=O)(CH₂)₁₆C(=O)); a is 1; B is Gly; m is 0, 1, or 2; and n is 1.
A55. The composition of Paragraph A53, wherein x is homoglutamine, aliphatic is a branched or straight optionally substituted C₁₈ chain covalently bound to X via a carbonyl (*e.g.*, CH₃(CH₂)₁₆C(=O)) or a C₁₈ chain covalently bound to X via a chemical linkage, such a thioether, an ether, a thioether, a carbamate moiety, a bond, or the like with X; preferably aliphatic is a branched or straight optionally substituted C₁₈ chain covalently bound to X via a carbonyl (*e.g.*, CH₃(CH₂)₁₆C(=O)) as part of an amide or ester linkage with X; or more preferably aliphatic is a branched or straight optionally substituted C₁₈ chain covalently bound to X via a carbonyl (*e.g.*, CH₃(CH₂)₁₆C(=O)) as part of an amide linkage with X, or aliphatic is HOC(=O)(CH₂)₁₆C(=O)); a is 1; B is Gly; m is 1; and n is 1.
A56. The composition of Paragraph A53, wherein x is homoglutamine, aliphatic is a branched or straight optionally substituted C₁₈ chain covalently bound to X via a carbonyl (*e.g.*, CH₃(CH₂)₁₆C(=O)) or a C₁₈ chain covalently bound to X via a chemical linkage, such a thioether, an ether, a thioether, a carbamate moiety, a bond, or the like with X; preferably aliphatic is a branched or straight optionally substituted C₁₈ chain covalently bound to X via a carbonyl (*e.g.*, CH₃(CH₂)₁₆C(=O)) as part of an amide or ester linkage with X; or more preferably aliphatic is a branched or straight optionally substituted C₁₈ chain covalently bound to X via a carbonyl (*e.g.,* CH₃(CH₂)₁₆C(=O)) as part of an amide linkage with X, or aliphatic is HOC(=O)(CH₂)₁₆C(=O)); a is 1; m is 1; and n is 0.
A57. The composition of Paragraph A53, wherein x is homoglutamine (homoQ) [SEQ ID NO. 16], U is (aliphatic)ₐ-(X)-; wherein a is 0 or 1 (preferably a is 1); aliphatic is a branched or straight optionally substituted C₁₈ chain covalently bound to X via a carbonyl (*e.g.,* CH₃(CH₂)₁₆C(=O)) or a C₁₈ chain covalently bound to X via a chemical linkage, such a thioether, an ether, a thioether, a carbamate moiety, a bond, or the like with X; preferably aliphatic is a branched or straight optionally substituted C₁₈ chain covalently bound to X via a carbonyl (*e.g.,* CH₃(CH₂)₁₆C(=O)) as part of an amide or ester linkage with X; or more preferably aliphatic is a branched or straight optionally substituted C₁₈ chain covalently bound to X via a carbonyl (*e.g.,* CH₃(CH₂)₁₆C(=O)) as part of an amide linkage with X, or aliphatic is HOC(=O)(CH₂)₁₆C(=O)); X is a linker (γE)ₘ-(B)ₙ; B is 2-[2-(2-aminoethoxy)ethoxy]acetic acid residue, m is 0, and n is 2.
A58. The composition of Paragraph A53, wherein x is homoglutamine (homoQ) [SEQ ID NO. 17], U is (aliphatic)ₐ-(X)-; wherein a is 0 or 1 (preferably a is 1); aliphatic is a branched or straight optionally substituted C₁₈ chain covalently bound to X via a carbonyl (*e.g.*, CH₃(CH₂)₁₆C(=O)) or a C₁₈ chain covalently bound to X via a chemical linkage, such a thioether, an ether, a thioether, a carbamate moiety, a bond, or the like with X; preferably aliphatic is a branched or straight optionally substituted C₁₈ chain covalently bound to X via a carbonyl (e.g., CH₃(CH₂)₁₆C(=O)) as part of an amide or ester linkage with X; or more preferably aliphatic is a branched or straight optionally substituted C₁₈ chain covalently bound to X via a carbonyl (*e.g.,* CH₃(CH₂)₁₆C(=O)) as part of an amide linkage with X, or aliphatic is HOC(=O)(CH₂)₁₆C(=O)); X is a linker (γE)ₘ-(B)ₙ; B is 2-[2-(2-aminoethoxy)ethoxy]acetic acid residue, m is 1, and n is 2.
A59. The composition of Paragraph A53, wherein x is homoglutamine, aliphatic is CH₃(CH₂)₁₆C(=O) or HOC(=O)(CH₂)₁₆C(=O); B is a 2-[2-(2-aminoethoxy)ethoxy]acetic acid residue, m is 0, and n is 2.
A60. The composition of Paragraph A53, wherein x is homoglutamine, aliphatic is CH₃(CH₂)₁₆C(=O) or HOC(=O)(CH₂)₁₆C(=O); B is a 2-[2-(2-aminoethoxy)ethoxy]acetic acid residue, m is 1, and n is 2.
A61. The composition of Paragraph A53, wherein x is homoglutamine, aliphatic is CH₃(CH₂)₁₆C(=O) or HOC(=O)(CH₂)₁₆C(=O); B is (2-[2-(2-aminoethoxy)ethoxy]acetic acid)-(2-[2-(2-aminoethoxy)ethoxy]acetic acid)-(Gly), m is 1, and n is 1.
A62. The composition of Paragraph A53, wherein the long acting CNP derivative is HOC(=O)(CH₂)₁₆C(=O)-γE-Aeea-Aeea-GCFGLKLDRIGShomoQSGLGC with a disulfide bond between the cysteine residues SEQ ID NO. 20].
A63. The composition of Paragraph A53, wherein the long acting CNP derivative is HOC(=O)(CH₂)₁₆C(=O)-Aeea-Aeea-GCFGLKLDRIGShomoQSGLGC with a disulfide bond between the cysteine residues SEQ ID NO. 21].
A64. The method of any one of Paragraphs A2 to A52, wherein a is 1.
A65. The composition of any one of Paragraphs A53 to A63, wherein a is 1.

While illustrative embodiments have been illustrated and described, it will be appreciated that various changes can be made therein without departing from the spirit and scope of the disclosure.

### EMBODIMENTS OF THE INVENTION

Also provided are the following embodiments:
1. A method of treating a subject having a lung, liver, and/or kidney injury, or a symptom associated with a lung, liver, and/or kidney injury, comprising:
   administering to the subject a therapeutically effective bolus dose of a composition comprising a long acting CNP, a long acting CNP derivative, a long acting NPRB agonist, a very long acting CNP, a very long acting CNP derivative, a very long acting NPRB agonist, a long acting CNP agonist, a very long acting CNP agonist, or any combination thereof,
   wherein the composition does not decrease blood pressure by more than 20% of a baseline blood pressure measurement taken prior to administration of the therapeutically effective bolus dose of the composition,
   wherein the composition increases plasma cyclic-GMP level at from 1 hour to 12 hours after administration to above 1.5x of a baseline plasma cyclic-GMP level, the baseline plasma cyclic-GMP level is an average plasma cyclic-GMP level prior to administration of the composition or the average plasma cyclic-GMP level of a healthy subject, and
   wherein the lung, liver, and/or kidney injury, or the symptom associated with lung, liver, and/or kidney injury is selected from
      i) acute lung injury (ALI),
      ii) acute respiratory distress syndrome (ARDS),
      iii) pulmonary edema,
      iv) elevated level of inflammatory cells in the lung,
      v) increased level or expression of inflammatory cytokines in the lung as compared to healthy lung,
      vi) increased protein level in lung alveolar space as compared to healthy lung,
      vii) low arterial blood oxygenation, wherein low arterial blood oxygenation is a blood PaO2 of below 60 mm Hg and/or a blood hemoglobin oxygen saturation (SpO2) of below 90%,
      viii) pneumonia,
      ix) fibrosis,
      x) kidney injury,
   and any combination thereof.
2. The method of embodiment [1], wherein the long acting CNP derivative or the very long acting CNP derivative comprises U-GLSKGCFGLKLDRIGSMSGLGC [SEQ ID NO. 2], U-GLSKGCFGLK(U)LDRIGSMSGLGC [SEQ ID NO. 3], GLSKGCFGLK(U)LDRIGSMSGLGC [SEQ ID NO. 4], U-CFGLKLDRIGSxSGLGC, where x is a natural or unnatural amino acid residue [SEQ ID NO. 11], or any combination thereof,
   wherein:
   U is a moiety of Formula (I) or (II), where Formula (I) is

      (aliphatic)ₐ-(X)-; (I)
   wherein
      a is 1;
      aliphatic is an optionally substituted C₄₋₂₄ chain (*e.g.,* an optionally substituted C₁₀₋₂₄ chain, an optionally substituted C₁₂₋₁₈ chain), covalently bound to X via a chemical linkage, such as a carbonyl (*e.g.,* as part of an amide or an ester linkage), a thioether, an ether, a thioether, a carbamate moiety, a bond, or the like with X; preferably via a carbonyl as part of an amide or an ester linkage; or more preferably via a carbonyl as part of an amide linkage with X;
      X is a 1-10 amino acid residue or peptide sequence, wherein each amino acid residue is independently selected from lysine (K), arginine (R), glycine (G), alanine (A), glutamic acid (E), and aspartic acid (D); or
      X is a linker (γE)ₘ-(B)ₙ,
      wherein B is a 1-8 amino acid residue sequence wherein each amino acid residue is independently selected from 2-[2-(2-aminoethoxy)ethoxy]acetic acid residue, Gly, Ala, Leu, Ser, Arg, and Lys;
      m is 0, 1, 2, or 3;
      n is 0, 1, 2, or 3; and
      the sum of m and n is at least 1,
   and Formula (II) is

      (polymer)ₐ-(Y)-; (II)
   wherein
      a is 1;
      polymer is cellulose, poly(ethylene glycol) (PEG), methoxy poly(ethylene glycol) (MPEG), poly(lactic-co-glycolic acid), poly(N-vinyl pyrrolidone), or a derivative thereof;
      Y is:
         a 1-10 amino acid residue or peptide sequence, wherein each amino acid residue is independently selected from lysine (K), arginine (R), glycine (G), alanine (A), glutamic acid (E), and aspartic acid (D);
         a non-amino acid linker comprising an ester, an amide, a thioether, an ether, a thioether, a carbamate moiety, or a combination thereof;
         an amino acid residue-containing linker, wherein the amino acid residue is covalently attached to (polymer)ₐ; or
         a peptide linker different from the 1-10 amino acid residue or peptide sequence.
3. The method of embodiment [2], wherein Y is a linker (γE)ₘ-(B)ₙ, wherein B is a 1-8 amino acid residue or peptide sequence wherein each amino acid residue is independently selected from a 2-[2-(2-aminoethoxy)ethoxy]acetic acid residue, Gly, Ala, Leu, Ser, Arg, and Lys; m is 0, 1, 2, or 3; n is 0, 1, 2, or 3; and the sum of m and n is at least 1.
4. The method of any one of embodiments [1] to [3], wherein the long acting CNP derivative or the very long acting CNP derivative comprises U-GLSKGCFGLKLDRIGSMSGLGC [SEQ ID NO. 2], U-GLSKGCFGLK(U)LDRIGSMSGLGC [SEQ ID NO. 3], GLSKGCFGLK(U)LDRIGSMSGLGC [SEQ ID NO. 4], U-CFGLKLDRIGSxSGLGC, where x is a natural or unnatural amino acid residue [SEQ ID NO. 12], or any combination thereof; and;
   wherein:
   U is a moiety of Formula (I), where Formula (I) is

      (aliphatic)ₐ-(X)-; (I)
   wherein
      a is 1;
      aliphatic is an optionally substituted C₁₀₋₂₄ chain (*e.g.,* an optionally substituted C₁₂₋₁₈ chain), covalently bound to X via a chemical linkage, such as a carbonyl (*e.g.,* as part of an amide or an ester linkage), a thioether, an ether, a thioether, a carbamate moiety, a bond, or the like with X; preferably via a carbonyl as part of an amide or an ester linkage; or more preferably via a carbonyl as part of an amide linkage with X;
      X is a 1-10 amino acid residue or peptide sequence, wherein each amino acid residue is independently selected from lysine (K), arginine (R), glycine (G), alanine (A), glutamic acid (E), and aspartic acid (D); or
      X is a linker (γE)ₘ-(B)ₙ,
      wherein B is a 1-8 amino acid residue or peptide sequence wherein each amino acid residue is independently selected from 2-[2-(2-aminoethoxy)ethoxy]acetic acid residue, Gly, Ala, Leu, Ser, Arg, and Lys;
      m is 0, 1, 2, or 3;
      n is 0, 1, 2, or 3; and
      the sum of m and n is at least 1.
5. The method of embodiment [2] or embodiment [3], wherein X is a 4-7 amino acid sequence, wherein each amino acid residue is independently selected from lysine (K), arginine (R), and glycine (G), or
   X is a linker (γE)ₘ-(B)ₙ wherein B is a 1-8 amino acid residue sequence wherein each amino acid residue is independently selected from 2-[2-(2-aminoethoxy)ethoxy]acetic acid residue, Gly, Ala, Leu, Ser, Arg, and Lys; m is 0, 1, 2, or 3; n is 0, 1, 2, or 3; and the sum of m and n is at least 1.
6. The method of any one of embodiments [1] to [3], wherein the long acting CNP derivative or the very long acting CNP derivative comprises U-GLSKGCFGLKLDRIGSMSGLGC [SEQ ID NO. 2],
   wherein:
   U is (aliphatic)ₐ-(X)-;
   wherein
      a is 1;
      aliphatic is an optionally substituted C₄₋₂₄ chain (*e.g.,* an optionally substituted C₁₀₋₂₄ chain, an optionally substituted C₁₂₋₁₈ chain), covalently bound to X via a chemical linkage, such as a carbonyl (*e.g.,* as part of an amide or an ester linkage), a thioether, an ether, a thioether, a carbamate moiety, a bond, or the like with X; preferably via a carbonyl as part of an amide or an ester linkage; or more preferably via a carbonyl as part of an amide linkage with X;
      X is a 1-10 amino acid residue or peptide sequence, wherein each amino acid residue is independently selected from lysine (K), arginine (R), glycine (G), alanine (A), glutamic acid (E), and aspartic acid (D); or
      X is a linker (γE)ₘ-(B)ₙ wherein B is a 1-8 amino acid residue sequence wherein each amino acid residue is independently selected from 2-[2-(2-aminoethoxy)ethoxy]acetic acid residue, Gly, Ala, Leu, Ser, Arg, and Lys; m is 0, 1, 2, or 3; n is 0, 1, 2, or 3; and the sum of m and n is at least 1.
7. The method of embodiment [2] or embodiment [3], wherein aliphatic does not comprise a straight or branched optionally substituted C₄₋₉ chain (*e.g.,* an optionally substituted C₃₋₈ alkyl-C(=O)- moiety, and/or an optionally substituted C₄₋₉ chain that is covalently bound to the peptide via a linkage such as a thioether, an ether, a thioether, a carbamate moiety, a bond, or the like).
8. The method of any one of embodiments [1] to [3], wherein the long acting CNP derivative is selected from
   CH₃(CH₂)₁₄C(=O)KKKKGGGGLSKGCFGLKLDRIGSMSGLGC [SEQ ID NO. 5];
   CH₃(CH₂)₁₆C(=O)KKKKGGGGLSKGCFGLKLDRIGSMSGLGC [SEQ ID NO. 6];
   CH₃(CH₂)₁₈C(=O)KKKKGGGGLSKGCFGLKLDRIGSMSGLGC [SEQ ID NO. 7];
   CH₃(CH₂)₂₀C(=O)KKKKGGGGLSKGCFGLKLDRIGSMSGLGC [SEQ ID NO. 8];
   CH₃(CH₂)₂₂C(=O)KKKKGGGGLSKGCFGLKLDRIGSMSGLGC [SEQ ID NO. 9];
   HOC(=O)(CH₂)₁₆C(=O)-γE-Aeea-Aeea-GCFGLKLDRIGShomoQSGLGC comprising a disulfide bond between the cysteine residues [SEQ ID NO. 20]; and
   HOC(=O)(CH₂)₁₆C(=O)-Aeea-Aeea-GCFGLKLDRIGShomoQSGLGC comprising a disulfide bond between the cysteine residues [SEQ ID NO. 21].
9. The method of any one of embodiments [1] to [3], wherein the long acting CNP derivative is CH₃(CH₂)₁₄C(=O)KKKKGGGGLSKGCFGLKLDRIGSMSGLGC [SEQ ID NO. 5].
10. The method of any one of embodiments [1] to [3], wherein the long acting CNP derivative is CH₃(CH₂)₁₆C(=O)KKKKGGGGLSKGCFGLKLDRIGSMSGLGC [SEQ ID NO. 6].
11. The method of any one of embodiments [1] to [3], wherein the long acting CNP derivative is CH₃(CH₂)₁₈C(=O)KKKKGGGGLSKGCFGLKLDRIGSMSGLGC [SEQ ID NO. 7].
12. The method of any one of embodiments [1] to [3], wherein the long acting CNP derivative is CH₃(CH₂)₂₀C(=O)KKKKGGGGLSKGCFGLKLDRIGSMSGLGC [SEQ ID NO. 8].
13. The method of any one of embodiments [1] to [3], wherein the long acting CNP derivative is CH₃(CH₂)₂₂C(=O)KKKKGGGGLSKGCFGLKLDRIGSMSGLGC [SEQ ID NO. 9].
14. The method of any one of embodiments [1] to [3], wherein the long acting CNP derivative is HOC(=O)(CH₂)₁₆C(=O)-γE-Aeea-Aeea-GCFGLKLDRIGShomoQSGLGC comprising a disulfide bond between the cysteine residues [SEQ ID NO. 20].
15. The method of any one of embodiments [1] to [3], wherein the long acting CNP derivative is HOC(=O)(CH₂)₁₆C(=O)-Aeea-Aeea-GCFGLKLDRIGShomoQSGLGC comprising a disulfide bond between the cysteine residues [SEQ ID NO. 21].
16. The method of any one of embodiments [1] to [3], wherein the long acting CNP derivative or the very long acting CNP derivative comprises U-GLSKGCFGLKLDRIGSMSGLGC [SEQ ID NO. 2], U-GLSKGCFGLK(U)LDRIGSMSGLGC [SEQ ID NO. 3], GLSKGCFGLK(U)LDRIGSMSGLGC [SEQ ID NO. 4], U-CFGLKLDRIGSxSGLGC, where x is a natural or unnatural amino acid residue [SEQ ID NO. 27], or any combination thereof;
   wherein:
   U is a moiety of Formula (II), where Formula (II) is

      (polymer)ₐ-(Y)-; (II)
   wherein
      a is 1;
      polymer is cellulose, poly(ethylene glycol) (PEG), methoxy poly(ethylene glycol) (MPEG), poly(lactic-co-glycolic acid), or poly(N-vinyl pyrrolidone);
      Y is:
         a 4-10 amino acid residue or peptide sequence, wherein each amino acid residue is independently selected from lysine (K), arginine (R), and glycine (G);
         a non-amino acid linker comprising an ester, an amide, a thioether, an ether, a thioether, a carbamate moiety, or a combination thereof; or
         a linker (γE)ₘ-(B)ₙ, wherein B is 1-8 amino acid residue sequence wherein each amino acid residue is independently selected from 2-[2-(2-aminoethoxy)ethoxy]acetic acid residue, Gly, Ala, Leu, Ser, Arg, and Lys; m is 0, 1, 2, or 3; n is 0, 1, 2, or 3; and the sum of m and n is at least 1.
17. The method of any one of embodiments [1] to [3], wherein the long acting CNP derivative or the very long acting CNP derivative comprises U-GLSKGCFGLKLDRIGSMSGLGC [SEQ ID NO. 2], U-GLSKGCFGLK(U)LDRIGSMSGLGC [SEQ ID NO. 3], or any combination thereof;
   wherein:
   U is a moiety of Formula (II), where Formula (II) is

      (polymer)ₐ-(Y)-; (II)
   wherein
      a is 1;
      polymer is cellulose, poly(ethylene glycol) (PEG), methoxy poly(ethylene glycol) (MPEG), poly(lactic-co-glycolic acid), poly(N-vinyl pyrrolidone), or a derivative thereof;
      Y is:
         a 1-10 amino acid residue or peptide sequence, wherein each amino acid residue is independently selected from lysine (K), arginine (R), glycine (G), alanine (A), glutamic acid (E), and aspartic acid (D);
         a non-amino acid linker comprising an ester, an amide, a thioether, an ether, a thioether, a carbamate moiety, or a combination thereof;
         an amino acid residue-containing linker, wherein the amino acid residue is covalently attached to (polymer)ₐ;
         a peptide linker different from the 1-10 amino acid residue or peptide sequence; or
         a linker (γE)ₘ-(B)ₙ, wherein B is 1-8 amino acid residue sequence wherein each amino acid residue is independently selected from a 2-[2-(2-aminoethoxy)ethoxy]acetic acid residue, Gly, Ala, Leu, Ser, Arg, and Lys; m is 0, 1, 2, or 3; n is 0, 1, 2, or 3; and the sum of m and n is at least 1.
18. The method of any one of embodiments [1] to [3], wherein the polymer does not include poly(ethylene glycol), MPEG, or both poly(ethylene glycol) and MPEG.
19. The method of any one of embodiments [1] to [3], wherein Y is:
   a 4-10 amino acid residue or peptide sequence, wherein each amino acid residue is independently selected from lysine (K), arginine (R), and glycine (G); or
   a linker (γE)ₘ-(B)ₙ, wherein B is 1-8 amino acid residue or peptide sequence wherein each amino acid residue is independently selected from 2-[2-(2-aminoethoxy)ethoxy]acetic acid residue, Gly, Ala, Leu, Ser, Arg, and Lys; m is 0, 1, 2, or 3; n is 0, 1, 2, or 3; and the sum of m and n is at least 1.
20. The method of any one of embodiments [1] to [3], wherein the bolus dose administration occurs at most twice a day and the route of administration comprises subcutaneous, intravenous, intramuscular, nasal, by inhalation, enteral, or any combination thereof, or
   wherein the route of administration is subcutaneous; or
   wherein the route of administration is intravenous; or
   wherein the route of administration is intramuscular; or
   wherein the route of administration is by inhalation; or
   wherein the route of administration is nasal; or
   wherein the enteral route of administration is oral.
21. The method of any one of embodiments [1] to [3], wherein the subject has ALI or ARDS associated with pulmonary edema; low arterial blood oxygenation; elevated level of inflammatory cells in the lung; increase level or expression of inflammatory cytokine in the lung; sepsis; bacteremia; pneumonia, pulmonary fibrosis, or any combination thereof.
22. The method of any one of embodiments [1] to [3], wherein inflammatory cytokine comprises IL-6, IL-1b, TNFa , MCP-1, IFNg, or any combination thereof.
23. The method of any one of embodiments [1] to [3], wherein the pneumonia comprises bacterial pneumonia, viral pneumonia, aseptic pneumonia,
24. The method of any one of embodiments [1] to [3], wherein the ALI or ARDS is caused by:
   (i) a systemic insult selected from trauma, sepsis, bacteremia, pancreatitis, shock, multiple transfusions, disseminated intravascular coagulation, burns, drug overdose or toxicity, opioids, aspirin, phenothiazines, tricyclic antidepressant, amiodarone, chemotherapeutic agents, nitrofurantoin, protamine, thrombotic thrombocytopenia purpura, head injury, paraquat, and any combination thereof; or
   (ii) a pulmonary insult selected from aspiration of gastric content, lung intubation, embolism, tuberculosis, viral pneumonia, bacterial pneumonia, cytogenic organizing pneumonitis, airway obstruction, smoking free-base cocaine, near-drowning, toxic gas inhalation, oxygen toxicity, lung contusion, radiation exposure, high-altitude exposure, lung re-expansion, reperfusion, and any combination thereof.
25. The method of embodiment [24], wherein the embolism is caused by a thrombus, fat, air, or amniotic fluid.
26. The method of embodiment [23], wherein the viral pneumonia is SARS caused by a coronavirus or an influenza virus.
27. The method of any one of embodiments [1] to [3], wherein:
   ALI or ARDS caused by an infectious disease, or
   ALI or ARDS caused by PF, or
   ALI or ARDS caused by sepsis; or
   ALI or ARDS caused by bacteremia; or
   ALI or ARDS caused by intubation; or
   ALI or ARDS caused by a toxic gas selected from group consisting of chlorine gas, smoke, phosgene, concentrated oxygen, and any combination thereof.
28. The method of embodiment [27], wherein the infectious disease is caused by a coronavirus or an influenza virus.
29. The method of any one of embodiments [1] to [3], wherein the fibrosis comprises lung or pulmonary fibrosis, cirrhosis, and kidney glomerular sclerosis.
30. The method of any one of embodiments [1] to [3], wherein the composition comprises a long acting CNP composition or a very long acting CNP composition, comprising a CNP, a CNP derivative, or a long acting CNP derivative and a polymer excipient, the polymer excipient comprising a poly(amino acid) grafted with polyethylene glycol, fatty acid, and/or anionic moieties; wherein the polymer excipient is adapted to sequester or non-covalently bind to any of the CNP or CNP derivatives.
31. The method of any one of embodiments [1] to [3], wherein the composition comprises a very long acting CNP derivative composition comprising a long acting CNP derivative and a polymer excipient, the polymer excipient comprising a poly(amino acid) grafted with polyethylene glycol, fatty acid, anionic moieties, or any combination thereof; and wherein the polymer excipient is adapted to sequester or non-covalently bind to the long acting CNP derivative.
32. The method of any one of embodiments [1] to [3], wherein the long acting NPRB agonist or the very long acting NPRB agonist comprises a polypeptide.
33. The method of embodiment [32], wherein the polypeptide comprises an antibody.
34. The method of any one of embodiments [1] to [3], wherein the long acting NPRB agonist or the very long acting NPRB agonist comprises a molecule of a molecular weight of less than 2kDa.
35. A method of treating a subject having, or at risk of developing ALI or ARDS, comprising
   administering to the subject a therapeutically effective bolus dose of a composition comprising a long acting CNP derivative or a very long acting CNP derivative comprising U-GLSKGCFGLKLDRIGSMSGLGC [SEQ ID NO. 2], U-GLSKGCFGLK(U)LDRIGSMSGLGC [SEQ ID NO. 3], GLSKGCFGLK(U)LDRIGSMSGLGC [SEQ ID NO. 4], U-CFGLKLDRIGSxSGLGC, where x is a natural or unnatural amino acid residue [SEQ ID NO. 11], or any combination thereof,
   wherein:
      U is a moiety of Formula (I) or (II), where Formula (I) is

         (aliphatic)ₐ-(X)-; (I)
      wherein
         a is 1;
         aliphatic is an optionally substituted C₄₋₂₄ chain (*e.g.,* an optionally substituted C₁₀₋₂₄ chain, an optionally substituted C₁₂₋₁₈ chain), covalently bound to X via a chemical linkage, such as a carbonyl (*e.g.,* as part of an amide or an ester linkage), a thioether, an ether, a thioether, a carbamate moiety, a bond, or the like with X; preferably via a carbonyl as part of an amide or an ester linkage; or more preferably via a carbonyl as part of an amide linkage with X;
         X is a 1-10 amino acid residue or peptide sequence, wherein each amino acid residue is independently selected from lysine (K), arginine (R), glycine (G), alanine (A), glutamic acid (E), and aspartic acid (D); or
         X is a linker (γE)ₘ-(B)ₙ wherein B is 1-8 amino acid residue or peptide sequence wherein each amino acid residue is independently selected from 2-[2-(2-aminoethoxy)ethoxy]acetic acid residue, Gly, Ala, Leu, Ser, Arg, and Lys; m is 0, 1, 2, or 3; n is 0, 1, 2, or 3; and the sum of m and n is at least 1;
      and Formula (II) is

         (polymer)ₐ-(Y)-; (II)
      wherein
         a is 1;
         polymer is cellulose, poly(ethylene glycol) (PEG), methoxy poly(ethylene glycol) (MPEG), poly(lactic-co-glycolic acid), poly(N-vinyl pyrrolidone), or a derivative thereof;
         Y is:
            a 1-10 amino acid residue or peptide sequence, wherein each amino acid residue is independently selected from lysine (K), arginine (R), glycine (G), alanine (A), glutamic acid (E), and aspartic acid (D);
            a non-amino acid linker comprising an ester, an amide, a thioether, an ether, a thioether, a carbamate moiety, or a combination thereof;
            an amino acid residue-containing linker, wherein the amino acid residue is covalently attached to (polymer)ₐ; or
            a peptide linker different from the 1-10 amino acid residue or peptide sequence;
   wherein the composition does not decrease blood pressure by more than 15% of a baseline blood pressure measurement; and
   wherein the composition increases plasma cyclic-GMP level at from 1 hour to 12 hours of a baseline plasma cyclic-GMP level, and the baseline plasma cyclic-GMP level is an average plasma cyclic-GMP level prior to administration of the composition or the average plasma cyclic-GMP level of a healthy subject.
36. The method of embodiment [35], wherein Y is a linker (γE)ₘ-(B)ₙ, wherein B is 1-8 amino acid residue or peptide sequence wherein each amino acid residue is independently selected from 2-[2-(2-aminoethoxy)ethoxy]acetic acid residue, Gly, Ala, Leu, Ser, Arg, and Lys; m is 0, 1, 2, or 3; n is 0, 1, 2, or 3; and the sum of m and n is at least 1.
37. The method of embodiment [35] or embodiment [36], wherein the long acting CNP derivative is selected from CH₃(CH₂)₁₄C(=O)KKKKGGGGLSKGCFGLKLDRIGSMSGLGC [SEQ ID NO. 5];
   CH₃(CH₂)₁₆C(=O)KKKKGGGGLSKGCFGLKLDRIGSMSGLGC [SEQ ID NO. 6];
   CH₃(CH₂)₁₈C(=O)KKKKGGGGLSKGCFGLKLDRIGSMSGLGC [SEQ ID NO. 7];
   CH₃(CH₂)₂₀C(=O)KKKKGGGGLSKGCFGLKLDRIGSMSGLGC [SEQ ID NO. 8];
   CH₃(CH₂)₂₂C(=O)KKKKGGGGLSKGCFGLKLDRIGSMSGLGC [SEQ ID NO. 9];
   HOC(=O)(CH₂)₁₆C(=O)-γE-Aeea-Aeea-GCFGLKLDRIGShomoQSGLGC comprising a disulfide bond between the cysteine residues [SEQ ID NO. 20]; and
   HOC(=O)(CH₂)₁₆C(=O)-Aeea-Aeea-GCFGLKLDRIGShomoQSGLGC comprising a disulfide bond between the cysteine residues [SEQ ID NO. 21].
38. The method of any one of embodiments [35] to [37], wherein the long acting CNP derivative is CH₃(CH₂)₁₄C(=O)KKKKGGGGLSKGCFGLKLDRIGSMSGLGC [SEQ ID NO. 5].
39. The method of any one of embodiments [35] to [37], wherein the long acting CNP derivative is CH₃(CH₂)₁₆C(=O)KKKKGGGGLSKGCFGLKLDRIGSMSGLGC [SEQ ID NO. 6].
40. The method of any one of embodiments [35] to [37], wherein the long acting CNP derivative is CH₃(CH₂)₁₈C(=O)KKKKGGGGLSKGCFGLKLDRIGSMSGLGC [SEQ ID NO. 7].
41. The method of any one of embodiments [35] to [37], wherein the long acting CNP derivative is CH₃(CH₂)₂₀C(=O)KKKKGGGGLSKGCFGLKLDRIGSMSGLGC [SEQ ID NO. 8].
42. The method of any one of embodiments [35] to [37], wherein the long acting CNP derivative is CH₃(CH₂)₂₂C(=O)KKKKGGGGLSKGCFGLKLDRIGSMSGLGC [SEQ ID NO. 9].
43. The method of any one of embodiments [35] to [37], wherein the long acting CNP derivative is HOC(=O)(CH₂)₁₆C(=O)-γE-Aeea-Aeea-GCFGLKLDRIGShomoQSGLGC comprising a disulfide bond between the cysteine residues [SEQ ID NO. 20].
44. The method of any one of embodiments [35] to [37], wherein the long acting CNP derivative is HOC(=O)(CH₂)₁₆C(=O)-Aeea-Aeea-GCFGLKLDRIGShomoQSGLGC comprising a disulfide bond between the cysteine residues [SEQ ID NO. 21].
45. The method of any one of embodiments [35] to [44], wherein the composition comprises a very long acting CNP derivative composition comprising a long acting CNP derivative and a polymer excipient, the polymer excipient comprising a poly(amino acid) grafted with polyethylene glycol, fatty acid, anionic moieties, or any combination thereof; wherein the polymer excipient is adapted to sequester or non-covalently bind to the long acting CNP derivative.
46. The method of any one of embodiments [1] to [45], wherein administering to the subject the therapeutically effective bolus dose of the composition decreases a total number of cells and total proteins in a BALF sample from the subject.
47. The method of any one of embodiments [1] to [46], wherein administering to the subject the therapeutically effective bolus dose of the composition decreases MPO in a lung tissue from the subject.
48. The method of any one of embodiments [1] to [47], wherein administering to the subject the therapeutically effective bolus dose of the composition decreases inflammatory cytokine expression in the subject.
49. The method of any one of embodiments [1] to [48], wherein administering to the subject the therapeutically effective bolus dose of the composition decreases a fibrotic area in a lung in a subject having idiopathic pulmonary fibrosis.
50. The method of any one of embodiments [1] to [49], wherein administering to the subject the therapeutically effective bolus dose of the composition decreases cell numbers and protein levels, and decreases the expression of any one of IL-6, IL-1b, TNFα, MCP-1, IFNg, or any combination thereof in a subject having idiopathic pulmonary fibrosis.
51. The method of any one of embodiments [1] to [49], wherein administering to the subject the therapeutically effective bolus dose of the composition decreases the expression of any one of IL-6, IL-1b, TNFα, MCP-1, IFNg, or any combination thereof, and decreases lethality in a subject having sepsis.
52. The method of any one of embodiments [1] to [51], wherein administering to the subject the therapeutically effective bolus dose of the composition decreases the expression of any one of AST, ALT, α-SMA, IL-6, IL-1b, TNFα, MCP-1, IFNg, iNOS, Elf-1, Tollip, IRAK-1, P-P38, P-P65, β-act, STAT1, P-STAT1, STAT2, STAT3, STAT6, a fibrotic area, serum creatinine, an albumin/creatinine ratio in urine, hydroxyproline in a lung, or any combination thereof, of the subject.
53. A composition comprising a long acting CNP derivative of comprising a formula U-CFGLKLDRIGSxSGLGC [SEQ ID NO. 30], wherein
   x is a natural or unnatural amino acid residue, provided that x is not a methionine residue; and
   U has is a moiety of Formula (I):

      (aliphatic)ₐ-(X)-; (I)
   wherein a is 1;
   aliphatic is an optionally substituted C₄₋₂₄ chain (*e.g.,* an optionally substituted C₁₀₋₂₄ chain, an optionally substituted C₁₂₋₁₈ chain), covalently bound to X via a chemical linkage, such as a carbonyl (*e.g.,* as part of an amide or an ester linkage), a thioether, an ether, a thioether, a carbamate moiety, a bond, or the like with X; preferably via a carbonyl as part of an amide or an ester linkage; or more preferably via a carbonyl as part of an amide linkage with X;
   X is a linker (γE)ₘ-(B)ₙ wherein B is 1-8 amino acid residue or peptide sequence wherein each amino acid residue is independently selected from 2-[2-(2-aminoethoxy)ethoxy]acetic acid residue, Gly, Ala, Leu, Ser, Arg, and Lys; m is 0, 1, 2, or 3; n is 0, 1, 2, or 3; and the sum of m and n is at least 1.
54. The composition of embodiment [53], wherein x is homoglutamine, aliphatic is a branched or straight optionally substituted C₁₈ chain covalently bound to X via a carbonyl (*e.g*., CH₃(CH₂)₁₆C(=O)) or a C₁₈ chain covalently bound to X via a chemical linkage, such a thioether, an ether, a thioether, a carbamate moiety, a bond, or the like with X; preferably aliphatic is a branched or straight optionally substituted C₁₈ chain covalently bound to X via a carbonyl (*e.g*., CH₃(CH₂)₁₆C(=O)) as part of an amide or ester linkage with X; or more preferably aliphatic is a branched or straight optionally substituted C₁₈ chain covalently bound to X via a carbonyl (*e.g.*, CH₃(CH₂)₁₆C(=O)) as part of an amide linkage with X, or aliphatic is HOC(=O)(CH₂)₁₆C(=O)); a is 1; B is Gly; m is 0, 1, or 2; and n is 1.
55. The composition of embodiment [53], wherein x is homoglutamine, aliphatic is a branched or straight optionally substituted C₁₈ chain covalently bound to X via a carbonyl (*e.g.,* CH₃(CH₂)₁₆C(=O)) or a C₁₈ chain covalently bound to X via a chemical linkage, such a thioether, an ether, a thioether, a carbamate moiety, a bond, or the like with X; preferably aliphatic is a branched or straight optionally substituted C₁₈ chain covalently bound to X via a carbonyl (*e.g.*, CH₃(CH₂)₁₆C(=O)) as part of an amide or ester linkage with X; or more preferably aliphatic is a branched or straight optionally substituted C₁₈ chain covalently bound to X via a carbonyl (*e.g.*, CH₃(CH₂)₁₆C(=O)) as part of an amide linkage with X, or aliphatic is HOC(=O)(CH₂)₁₆C(=O)); a is 1; B is Gly; m is 1; and n is 1.
56. The composition of embodiment [53], x is homoglutamine, aliphatic is a branched or straight optionally substituted C₁₈ chain covalently bound to X via a carbonyl (*e.g.,* CH₃(CH₂)₁₆C(=O)) or a C₁₈ chain covalently bound to X via a chemical linkage, such a thioether, an ether, a thioether, a carbamate moiety, a bond, or the like with X; preferably aliphatic is a branched or straight optionally substituted C₁₈ chain covalently bound to X via a carbonyl (*e.g.,* CH₃(CH₂)₁₆C(=O)) as part of an amide or ester linkage with X; or more preferably aliphatic is a branched or straight optionally substituted C₁₈ chain covalently bound to X via a carbonyl (*e.g*., CH₃(CH₂)₁₆C(=O)) as part of an amide linkage with X, or aliphatic is HOC(=O)(CH₂)₁₆C(=O)); a is 1; m is 1; and n is 0.
57. The composition of embodiment [53], wherein x is homoglutamine (homoQ) [SEQ ID NO. 16], U is (aliphatic)ₐ-(X)-; wherein a is 0 or 1 (preferably a is 1); aliphatic is a branched or straight optionally substituted C₁₈ chain covalently bound to X via a carbonyl (*e.g.*, CH₃(CH₂)₁₆C(=O)) or a C₁₈ chain covalently bound to X via a chemical linkage, such a thioether, an ether, a thioether, a carbamate moiety, a bond, or the like with X; preferably aliphatic is a branched or straight optionally substituted C₁₈ chain covalently bound to X via a carbonyl (*e.g.,* CH₃(CH₂)₁₆C(=O)) as part of an amide or ester linkage with X; or more preferably aliphatic is a branched or straight optionally substituted C₁₈ chain covalently bound to X via a carbonyl (*e.g.,* CH₃(CH₂)₁₆C(=O)) as part of an amide linkage with X, or aliphatic is HOC(=O)(CH₂)₁₆C(=O)); X is a linker (γE)ₘ-(B)ₙ; B is 2-[2-(2-aminoethoxy)ethoxy]acetic acid residue, m is 0, and n is 2.
58. The composition of embodiment [53], wherein x is homoglutamine (homoQ) [SEQ ID NO. 17], U is (aliphatic)ₐ-(X)-; wherein a is 0 or 1 (preferably a is 1); aliphatic is a branched or straight optionally substituted C₁₈ chain covalently bound to X via a carbonyl (*e.g.*, CH₃(CH₂)₁₆C(=O)) or a C₁₈ chain covalently bound to X via a chemical linkage, such a thioether, an ether, a thioether, a carbamate moiety, a bond, or the like with X; preferably aliphatic is a branched or straight optionally substituted C₁₈ chain covalently bound to X via a carbonyl (*e.g.,* CH₃(CH₂)₁₆C(=O)) as part of an amide or ester linkage with X; or more preferably aliphatic is a branched or straight optionally substituted C₁₈ chain covalently bound to X via a carbonyl (*e.g.,* CH₃(CH₂)₁₆C(=O)) as part of an amide linkage with X, or aliphatic is HOC(=O)(CH₂)₁₆C(=O)); X is a linker (γE)ₘ-(B)ₙ; B is 2-[2-(2-aminoethoxy)ethoxy]acetic acid residue, m is 1, and n is 2
59. The composition of embodiment [53], wherein x is homoglutamine, aliphatic is a CH₃(CH₂)₁₆C(=O) or HOC(=O)(CH₂)₁₆C(=O); B is a 2-[2-(2-aminoethoxy)ethoxy]acetic acid residue, m is 0, and n is 2.
60. The composition of embodiment [53], wherein x is homoglutamine, aliphatic is a CH₃(CH₂)₁₆C(=O) or HOC(=O)(CH₂)₁₆C(=O); B is a 2-[2-(2-aminoethoxy)ethoxy]acetic acid residue, m is 1, and n is 2.
61. The composition of embodiment [53], wherein x is homoglutamine, aliphatic is a CH₃(CH₂)₁₆C(=O) or HOC(=O)(CH₂)₁₆C(=O); B is (2-[2-(2-aminoethoxy)ethoxy]acetic acid)-(2-[2-(2-aminoethoxy)ethoxy]acetic acid)-(Gly), m is 1, and n is 1.
62. The composition of embodiment [53], wherein the long acting CNP derivative is HOC(=O)(CH₂)₁₆C(=O)-γE-Aeea-Aeea-GCFGLKLDRIGShomoQSGLGC comprising a disulfide bond between the cysteine residues [SEQ ID NO. 20].
63. The composition of embodiment [53], wherein the long acting CNP derivative is HOC(=O)(CH₂)₁₆C(=O)-Aeea-Aeea-GCFGLKLDRIGShomoQSGLGC comprising a disulfide bond between the cysteine residues [SEQ ID NO. 21].

## Claims

1. A composition for use in a method of treating a lung, liver, and/or kidney injury, or a symptom associated with a lung, liver, and/or kidney injury, in a subject, the method comprising administering to the subject a therapeutically effective bolus dose of the composition, the composition comprising CH3(CH2)16C(=O)KKKKGGGGLSKGCFGLKLDRIGSMSGLGC [SEQ ID NO. 6],
wherein the composition does not decrease blood pressure by more than 20% of a baseline blood pressure measurement taken prior to administration of the therapeutically effective bolus dose of the composition,
wherein the composition increases plasma cyclic-GMP level at from 1 hour to 12 hours after administration to above 1.5x of a baseline plasma cyclic-GMP level, the baseline plasma cyclic-GMP level is an average plasma cyclic-GMP level prior to administration of the composition or the average plasma cyclic-GMP level of a healthy subject, and
wherein the lung, liver, and/or kidney injury, or the symptom associated with lung, liver, and/or kidney injury is selected from
i) acute lung injury (ALI),
ii) fibrosis,
iii) kidney injury,
iv) acute respiratory distress syndrome (ARDS),
v) pulmonary edema,
vi) elevated level of inflammatory cells in the lung,
vii) increased level or expression of inflammatory cytokines in the lung as compared to healthy lung,
viii) increased protein level in lung alveolar space as compared to healthy lung,
ix) low arterial blood oxygenation, wherein low arterial blood oxygenation is a blood PaO2 of below 60 mm Hg and/or a blood hemoglobin oxygen saturation (SpO2) of below 90%,
x) pneumonia,
and any combination thereof.

2. The composition for use according to claim 1, wherein the lung, liver, and/or kidney injury, or the symptom associated with lung, liver, and/or kidney injury is selected from
i) acute lung injury (ALI),
ii) fibrosis,
iii) kidney injury,
and any combination thereof.

3. The composition for use according to claim 1, wherein the lung, liver, and/or kidney injury, or the symptom associated with lung, liver, and/or kidney injury is acute lung injury (ALI).

4. The composition for use according to claim 1, wherein the lung, liver, and/or kidney injury, or the symptom associated with lung, liver, and/or kidney injury is fibrosis.

5. The composition for use according to claim 4, wherein the fibrosis is liver fibrosis.

6. The composition for use according to claim 4, wherein the fibrosis is lung fibrosis.

7. The composition for use according to claim 6, wherein the lung fibrosis is idiopathic pulmonary fibrosis.

8. The composition for use according to claim 1, wherein the lung, liver, and/or kidney injury, or the symptom associated with lung, liver, and/or kidney injury is kidney injury.

9. The composition for use according to claim 8, wherein the kidney injury is acute kidney injury.

10. The composition for use according to any of the preceding claims, wherein the composition further comprises one or more excipient or carrier such as a polymer, protein, sugar, detergent, and/or buffer.

11. The composition for use according to any of the preceding claims, wherein:
the bolus dose administration occurs at most twice a day and the route of administration comprises subcutaneous, intravenous, intramuscular, nasal, by inhalation, enteral, or any combination thereof, optionally wherein
the route of administration is subcutaneous, intravenous, intramuscular, by inhalation, nasal or oral.

12. The composition for use of any one of the preceding claims, wherein the composition further comprises a polymer excipient, the polymer excipient comprising a poly(amino acid) grafted with polyethylene glycol, fatty acid, anionic moieties, or any combination thereof; wherein the polymer excipient is adapted to sequester or non-covalently bind to the CH3(CH2)16C(=O)KKKKGGGGLSKGCFGLKLDRIGSMSGLGC [SEQ ID NO. 6].

13. The composition for use according to claim 12, wherein the polymer excipient comprises polylysine grafted with polyethylene glycol.
